# EUROPEAN PATENT SPECIFICATION

(11) **EP 4 562 004 B1**
(45) Date of publication and mention of the grant of the patent: **16.09.2026**
(21) Application number: 23748061.1
(22) Date of filing: 26.07.2023
(51) Int. Cl.: C07D 403/14, A61P 25/00, A61K 31/437

(54) **NOVEL 7-SUBSTITUTED INDOLE SULFONAMIDE DERIVATIVES**
NEUARTIGE 7-SUBSTITUIERTE INDOLSULFONAMIDDERIVATE
NOUVEAUX DÉRIVÉS DU SULFONAMIDE INDOLE SUBSTITUÉ À 7

(30) Priority: 28.07.2022 EP 22187365
(43) Date of publication of application: 04.06.2025
(73) Proprietor: F. Hoffmann-La Roche AG, 4070 Basel (CH)
(72) Inventor: GALLEY, Guido, 4070 Basel (CH); GOBBI, Luca Claudio, 4070 Basel (CH); GUBA, Wolfgang, 4070 Basel (CH); MAZUNIN, Dmitry, 4070 Basel (CH); PINARD, Emmanuel, 4070 Basel (CH)
(74) Representative: Jochnowitz, Evan
(86) International application number: PCT/EP2023/070658
(87) International publication number: WO 2024/023129

(56) References cited:
- WO-A1-2019/243398
- WO-A1-2022/180136

## Description

The present invention relates to organic compounds useful for therapy and/or prophylaxis in a mammal, and in particular to compounds that modulate GPR17 activity.

The present invention provides novel compounds of formula I 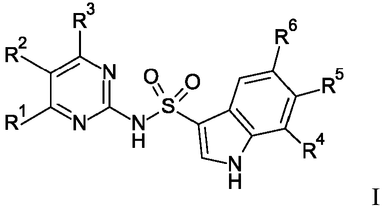 wherein,
R¹ is alkoxy or haloalkoxy;
R² is halo, alkyl, alkoxy, haloalkyl, haloalkoxy, cyanoalkyl, cyanoalkoxy, or cyclopropyl optionally substituted with up to two substituents independently selected from cyano and halo;
R³ is H, alkoxy, or haloalkoxy;
R⁵ is H, halo, alkyl, or haloalkyl;
R⁶ is H or halo;
R⁴ is an N-linked heteroaryl or substituted N-linked heteroaryl, represented by 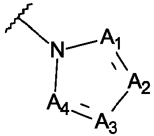
A₁ is CR^{y1} or N;
A₂ is CR^{y2} or N;
A₃ is CR^{y3} or N;
A₄ is CR^{y4} or N;
wherein R^{y1}, R^{y2}, R^{y3} and R^{y4} are independently selected from H, alkyl, halo and haloalkyl;
and pharmaceutically acceptable salts.

Furthermore, the invention includes all racemic mixtures, all their corresponding enantiomers and/or optical isomers.

Furthermore, the invention includes all substituents in their corresponding deuterated form, wherever applicable, of the compound of formula (I).

### Background of the Invention

Myelination is a process that occurs robustly during development and despite the abundant presence of oligodendrocyte precursor cells (OPCs) throughout the adult CNS, the transition to myelinating oligodendrocytes and the production of restorative myelin sheaths around denuded axons is impaired in chronic demyelinating diseases. During development, myelination proceeds in a very orderly manner, with OPCs, characterized by expression of markers such as neural/glial antigen 2 (NG2) and platelet-derived growth factor alpha (PDGFRα), differentiating into oligodendrocytes which lose NG2 and PDGFRα expression and gain the expression of markers such as myelin basic protein (MBP) and myelin oligodendrocyte glycoprotein (MOG). The production of myelin by oligodendrocytes is a very tightly regulated process and in the CNS, this can be controlled by interactions with axons, well-understood in the peripheral but not in the central nervous system (Macklin, W.B. (2010). Sci. Signal. 3, pe32-pe32, "The myelin brake: When Enough Is Enough"). Myelination can also be controlled by internal brakes within oligodendrocytes themselves, through the transcription factor EB (TFEB)-PUMA axis or through GPR17 antagonism (Chen, Y., et al. (2009). Nat Neurosci 12, 1398-1406, "The oligodendrocyte-specific G protein-coupled receptor GPR17 is a cell-intrinsic timer of myelination") (Sun, L.O., et al. (2018). Cell 175, 1811-1826.e21, "Spatiotemporal Control of CNS Myelination by Oligodendrocyte Programmed Cell Death through the TFEB-PUMA Axis"). Myelin serves not only to protect axons and facilitate neuronal transmission, but oligodendrocytes have also been shown to play an important role in metabolism of axons as well as in maintaining the electrolyte balance around axons (Schirmer, L., et al. (2014). Ann Neurol 75, 810-828, "Differential loss of KIR4.1 immunoreactivity in multiple sclerosis lesions") (Simons, M., and Nave, K.-A. (2015). Cold Spring Harb Perspect Biol. 22, "Oligodendrocytes: Myelination and Axonal Support").

GPR17 is a Class A orphan G protein-coupled receptor (GPCR). GPCRs are 7 domain transmembrane proteins that couple extracellular ligands with intracellular signaling via their intracellular association with small, heterotrimeric G-protein complexes consisting of G_{α}, G_{β}, G_{Υ} subunits. It is the coupling of the GPCR to the G_{α} subunit that confers results in downstream intracellular signaling pathways. GPR17 is known to be coupled directly to G_{α i/o}, which leads to inhibition of adenylate cyclase activity, resulting in a reduction in cyclic AMP production (cAMP). GPR17 has also been shown to couple to G_{q/11}, that targets phospholipase C. Activation of phospholipase C leads to the cleavage of phosphatidylinositol 4,5-bisphosphate which produces inositol triphosphate (IP₃) and diacylglycerol (DAG). IP₃ consequently binds to the IP₃ receptor on the endoplasmic reticulum and causes an increase in intracellular calcium levels (Hanlon, C.D., and Andrew, D.J. (2015). J Cell Sci. 128, 3533-3542, "Outside-in signaling-a brief review of GPCR signaling with a focus on the Drosophila GPCR family") (Inoue, A., et al. (2019), Cell 177, 1933-1947.e25, "Illuminating G-Protein-Coupling Selectivity of GPCRs").

The role of GPR17 in myelination was first identified in a screen of the optic nerves of *Olig1* knockout mice to identify genes regulating myelination. GPR17 expression was found to be expressed only in the myelinating cells of the CNS and absent from the Schwann cells, the peripheral nervous system's myelinating cells. The expression of GPR17 was found to be exclusively expressed in the oligodendrocyte lineage cells and was downregulated in myelinating oligodendrocyte (Chen, Y., et al. (2009)). Specifically, GPR17 expression is found to be present at low levels early on in the OPC and increases in the pre-myelinating oligodendrocyte before the expression is downregulated in the mature, myelinating oligodendrocyte (Boda, E., et al. (2011), Glia 59, 1958-1973, "The GPR17 receptor in NG2 expressing cells: Focus on in vivocell maturation and participation in acute trauma and chronic damage") (Dziedzic, A., et al. (2020). Int. J. Mol. Sci. 21, 1852, "The gpr17 receptor-a promising goal for therapy and a potential marker of the neurodegenerative process in multiple sclerosis") (Fumagalli, M. et al. (2011), J Biol Chem 286, 10593-10604, "Phenotypic changes, signaling pathway, and functional correlates of GPR17-expressing neural precursor cells during oligodendrocyte differentiation"). GPR17 knockout animals were shown to exhibit precocious myelination throughout the CNS and conversely, transgenic mice overexpressing *GPR17* in oligodendrocytes with the CNP-Cre (2', 3' - cyclic-nucleotide 3'-phosphodiesterase) promoter exhibited myelinogenesis defects, in line with what is to be expected of a cell-intrinsic brake on the myelination process (Chen, Y., et al. (2009)). Furthermore, loss of GPR17 enhances remyelination following demyelination with lysophosphatidylcholine-induced demyelination (Lu, C., Dong, et al. (2018), Sci. Rep. 8, 4502, "G-Protein-Coupled Receptor Gpr17 Regulates Oligodendrocyte Differentiation in Response to Lysolecithin-Induced Demyelination"). As such, antagonism of GPR17 that promotes the differentiation of oligodendrocyte lineage cells into mature, myelinating oligodendrocytes would lead to increase in myelination following demyelination.

Multiple sclerosis (MS) is a chronic neurodegenerative disease that is characterized by the loss of myelin, the protective fatty lipid layer surrounding axons, in the central nervous system (CNS). Prevention of myelin loss or remyelination of denuded axons is thought to prevent axonal degeneration and thus prevent progression of the disease (Franklin, R.J. (2002), Nat Rev Neurosci 3, 705-714, "Why does remyelination fail in multiple sclerosis?"). Due to the restorative impact that myelin repair has on the central nervous system, such a treatment will benefit all types of MS namely relapse-remitting, secondary progressive, primary progressive and progressive relapsing MS. Reparation of lost myelin will alleviate neurological symptoms associated with MS due to the neuroprotective effect of preserving axons.

Due to the essential role that myelination plays in functioning of the nervous system, facilitating OPC to oligodendrocyte differentiation has the potential to impact multiple diseases where white matter defects/irregularities due to either loss of myelinating oligodendrocytes or hampered differentiation of OPCs to oligodendrocytes have been observed, due to the disease itself or inflammation. This is in addition to the diseases where GPR17 expression itself is altered.

The diseases that GPR17 antagonism can be thus used to yield a positive disease outcome include, but are not limited to:
Direct damage to myelin sheaths:
   - Metabolic conditions that lead to destruction of central myelin such as central pontine myelinolysis, extra-pontine myelinolysis due to overly-rapid correction of hyponatremia in conditions for instance, but not limited to, alcoholism, liver disease, immunosuppression after transplantation
   - Carbon monoxide poisoning where oligodendrocyte dysfunction and failure to regenerate has been reported in the deep white matter layers of the brain
   - Nutritional deficiency that results in myelin loss or failure to properly generate myelin during development
   - Virus-induced demyelination
Primary demyelinating disorders
   - Multiple Sclerosis (relapse-remitting, secondary progressive, primary progressive and progressive relapsing MS)
   - Acute and multiphasic disseminated encephalomyelitis
   - Neuromyelitis optica spectrum disorders including optic neuritis
   - Transverse myelitis
   - Leukodystrophies such as adrenoleukodystrophy, adrenomyeloneuropathy and other inherited leukodystrophies that result in myelin loss
CNS disorders with associated myelin loss:
   - Alzheimer's Disease
   - Schizophrenia
   - Parkinson's Disease
   - Huntington's disease
   - Amyotrophic lateral
   - Ischemia due to stroke
Other diseases:
   - Inflammation in the CNS for instance following encephalitis, primary angiitis, meningitis The compounds of formula I bind to and modulates GPR17 activity.

The compounds of formula I are therefore particularly useful in the treatment of diseases related to GPR17 antagonism.

The compounds of formula I are particularly useful in the treatment or prophylaxis of multiple sclerosis (MS), conditions related to direct damage to myelin sheaths such as carbon monoxide poisoning or virus induced demyelination, primary demyelinating disorders such as acute and multiphasic disseminated encephalomyelitis, and other CNS disorders associated with myelin loss such as Alzheimer's disease, schizophrenia, Parkinson's disease and Huntington's disease.

WO2019/243398 A1 discloses pyridin-2-yl-1H-indole-3-sulfonamide type GPR17 antagonists.

### Summary of the Invention

The present invention provides novel compounds of formula I 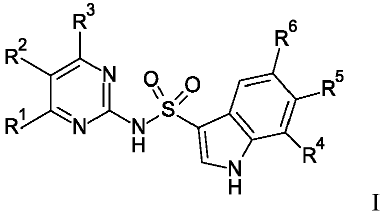 wherein,
R¹ is alkoxy or haloalkoxy;
R² is halo, alkyl, alkoxy, haloalkyl, haloalkoxy, cyanoalkyl, cyanoalkoxy, or cyclopropyl optionally substituted with up to two substituents independently selected from cyano and halo;
R³ is H, alkoxy, or haloalkoxy;
R⁵ is H, halo, alkyl, or haloalkyl;
R⁶ is H or halo;
R⁴ is an N-linked heteroaryl or substituted N-linked heteroaryl, represented by 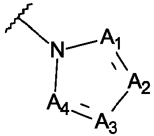
A₁ is CR^{y1} or N;
A₂ is CR^{y2} or N;
A₃ is CR^{y3} or N;
A₄ is CR^{y4} or N;
wherein R^{y1}, R^{y2}, R^{y3} and R^{y4} are independently selected from H, alkyl, halo and haloalkyl;
and pharmaceutically acceptable salts.

The term "alkyl" denotes a monovalent linear or branched saturated hydrocarbon group of 1 to 6 carbon atoms. In some embodiments, if not otherwise described, alkyl comprises 1 to 6 carbon atoms (C₁₋₆-alkyl), or 1 to 4 carbon atoms (C₁₋₄-alkyl). Examples of C₁₋₆-alkyl include methyl, ethyl, propyl, isopropyl, n-butyl, iso-butyl, sec-butyl, tert-butyl and pentyl. Particular alkyl group is methyl. When an alkyl residue having a specific number of carbons is named, all geometric isomers having that number of carbons may be encompassed. Thus, for example, "butyl" can include n-butyl, sec-butyl, isobutyl and t-butyl, and "propyl" can include n-propyl and isopropyl. Particular example of alkyl is methyl.

The term "alkoxy" denotes a group of the formula -O-R', wherein R' is a C₁₋₆-alkyl group. Examples of C₁₋₆-alkoxy groups include methoxy, ethoxy, n-propoxy, isopropoxy, n-butoxy, isobutoxy and tert-butoxy. Particular example of alkoxy is methoxy.

The term "halogen", "halide" and "halo" are used interchangeably herein and denote fluoro, chloro, bromo or iodo. Particular halogens are fluoro, chloro and bromo.

The term "haloalkyl" denotes a C₁₋₆-alkyl group wherein at least one of the hydrogen atoms of the C₁₋₆-alkyl group has been replaced by the same or different halogen atoms. Particular examples of haloalkyl are difluoromethyl, difluoroethyl and diflouropropyl.

The term "haloalkoxy" denotes a C₁₋₆-alkoxy group wherein at least one of the hydrogen atoms of the C₁₋₆-alkoxy group has been replaced by the same or different halogen atoms. Particular examples of haloalkoxy are fluoroethoxy, difluoroethoxy, and difluoromethoxy. Other particular examples of haloalkoxy are trifluoroethoxy such as 1,1,2-trifluoroethoxy, and 2-fluoro-1,1-dimethyl-ethoxy.

The term "cyano" denotes a -C≡N group.

"Cyanoalkyl" means a moiety of the formula -R'-R", where R' is alkyl as defined herein and R" is cyano or nitrile. An example of cyanoalkyl is cyanoethyl. Particular example of cyanoalkyl is cyanoethyl.

"Cyanoalkoxy" means a moiety of the formula -R'-R", where R' is alkoxy as defined herein and R" is cyano or nitrile. Particular example of cyanoalkoxy is cyanomethoxy.

The term "heteroaryl" denotes a monovalent aromatic mono- or bicyclic ring system of 4 to 9 ring atoms, comprising 1, 2, 3, or 4 ring heteroatoms selected from N and O, the remaining ring atoms being carbon. Bicyclic means consisting of two cycles having one or two ring atoms in common. Example for heteroaryl are pyrazolyl, imidazolyl and triazolyl.

The term "N-linked heteroaryl" means denotes a heteroaryl system up to 9 ring atoms, wherein the heteroaryl system is attached to the rest of the molecule through an N-heteroatom.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition these salts may be prepared from addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The compound of formula I can also be present in the form of zwitterions. Particularly preferred pharmaceutically acceptable salts of compounds of formula I are the salts formed with formic acid and the salts formed with hydrochloric acid yielding a hydrochloride, dihydrochloride or trihydrochloride salt.

The abbreviation uM means microMolar and is equivalent to the symbol µM.

The abbreviation uL means microliter and is equivalent to the symbol µL.

The abbreviation ug means microgram and is equivalent to the symbol µg.

The compounds of formula I can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Furthermore, structures depicted herein are also meant to include compounds that differ only in the presence of one or more isotopically enriched atoms. For example wherein one or more hydrogen atoms are replaced by deuterium (²H), or one or more carbon atoms are replaced by a ¹³C- or ¹⁴C-enriched carbon are within the scope of this invention.

Also an embodiment of the present invention provides compounds according to formula I as described herein and pharmaceutically acceptable salts or esters thereof, in particular compounds according to formula I as described herein and pharmaceutically acceptable salts thereof, more particularly compounds according to formula I as described herein.

An embodiment of the present invention provides compounds according to formula I as described herein, wherein R¹ is alkoxy.

An embodiment of the present invention provides compounds according to formula I as described herein, wherein R² is halo, alkyl, haloalkyl, haloalkoxy, cyanoalkyl, cyanoalkoxy, or cyclopropyl optionally substituted with cyano.

An embodiment of the present invention provides compounds according to formula I as described herein, wherein R² is haloalkyl, haloalkoxy, or cyclopropyl substituted with cyano.

An embodiment of the present invention provides compounds according to formula I as described herein, wherein R² is haloalkoxy.

An embodiment of the present invention provides compounds according to formula I as described herein, wherein R³ is H or alkoxy.

An embodiment of the present invention provides compounds according to formula I as described herein, wherein R³ is alkoxy.

An embodiment of the present invention provides compounds according to formula I as described herein, wherein R⁵ is halo.

An embodiment of the present invention provides compounds according to formula I as described herein, wherein R⁶ is H.

An embodiment of the present invention provides compounds according to formula I as described herein, wherein at least one, but no more than two, of A₁, A₂, A₃ and A₄ are N, and R^{y1}, R^{y2}, R^{y3} and R^{y4} are independently selected from H, halo and alkyl.

An embodiment of the present invention provides compounds according to formula I as described herein, wherein at least one, but no more than two, of A₁, A₂, A₃ and A₄ are N, and R^{y1}, R^{y2}, R^{y3} and R^{y4} are independently selected from H, and alkyl.

An embodiment of the present invention provides compounds according to formula I as described herein, wherein R⁴ is selected from

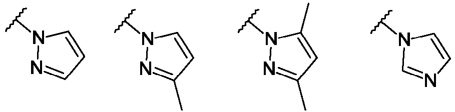

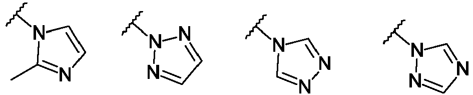

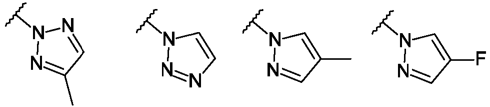

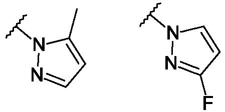

An embodiment of the present invention provides compounds according to formula I as described herein, wherein R⁴ is selected from

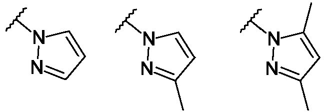

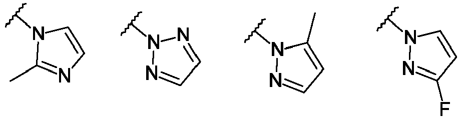

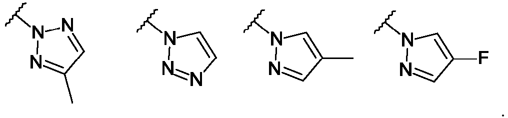

An embodiment of the present invention provides compounds according to formula I as described herein, wherein R⁴ is selected from

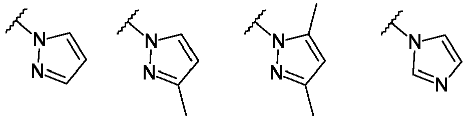

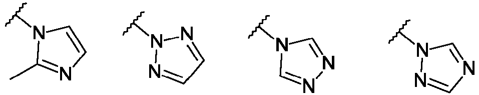

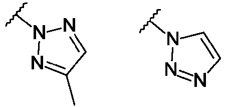

An embodiment of the present invention provides compounds according to formula I as described herein, wherein R⁴ is selected from

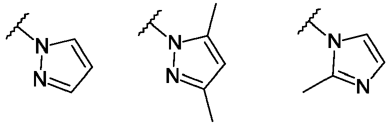

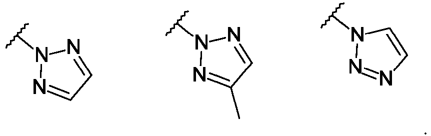

An embodiment of the present invention provides compounds according to formula I as described herein, wherein R⁴ is triazolyl.

An embodiment of the present invention provides compounds according to formula I as described herein, wherein R⁴ is

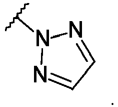

An embodiment of the present invention provides compounds according to formula I as described herein, wherein,
R¹ is alkoxy;
R² is halo, alkyl, haloalkyl, haloalkoxy, cyanoalkyl, cyanoalkoxy, or cyclopropyl substituted with cyano;
R³ is H or alkoxy;
R⁵ is H, halo, alkyl, or haloalkyl;
R⁶ is H or halo;
R⁴ is an N-linked heteroaryl or substituted N-linked heteroaryl, represented by 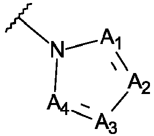
A₁ is CR^{y1} or N;
A₂ is CR^{y2} or N;
A₃ is CR^{y3} or N;
A₄ is CR^{y4} or N;
wherein R^{y1}, R^{y2}, R^{y3} and R^{y4} are independently selected from H, alkyl, and halo;
and pharmaceutically acceptable salts.

An embodiment of the present invention provides compounds according to formula I as described herein, wherein,
R¹ is alkoxy;
R² is halo, alkyl, haloalkyl, haloalkoxy, cyanoalkyl, cyanoalkoxy, or cyclopropyl substituted with cyano;
R³ is H or alkoxy;
R⁵ is H, halo, alkyl, or haloalkyl;
R⁶ is H or halo;
R⁴ is is selected from 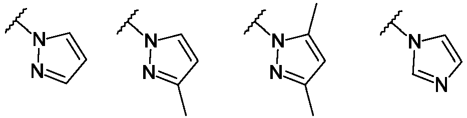 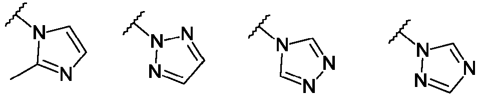 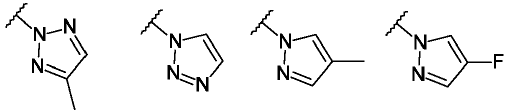 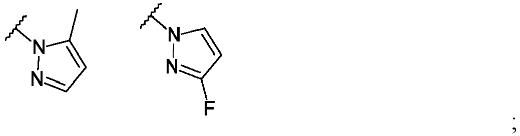
and pharmaceutically acceptable salts.

An embodiment of the present invention provides compounds according to formula I as described herein, wherein,
R¹ is alkoxy;
R² is halo, alkyl, haloalkyl, haloalkoxy, cyanoalkyl, cyanoalkoxy, or cyclopropyl substituted with cyano;
R³ is H or alkoxy;
R⁵ is H, halo, alkyl, or haloalkyl;
R⁶ is H;
R⁴ is selected from 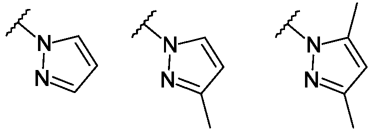 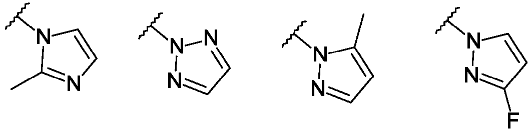 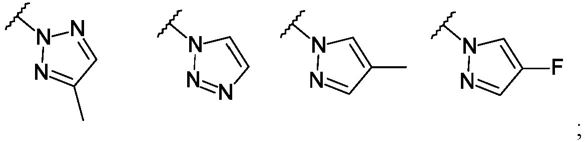
and pharmaceutically acceptable salts.

An embodiment of the present invention provides compounds according to formula I as described herein, wherein,
R¹ is alkoxy;
R² is haloalkoxy;
R³ is alkoxy;
R⁵ is halo;
R⁶ is H;
R⁴ is 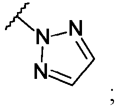
and pharmaceutically acceptable salts.

An embodiment of the present invention provides compounds according to formula I as described herein, wherein,
R¹ is alkoxy or haloalkoxy;
R² is halo, alkyl, alkoxy, haloalkyl, haloalkoxy, cyanoalkyl, cyanoalkoxy, or cyclopropyl optionally substituted with up to two substituents independently selected from cyano and halo;
R³ is H, alkoxy, or haloalkoxy;
R⁵ is H, halo, alkyl, or haloalkyl;
R⁶ is H or halo;
R⁴ is an N-linked heteroaryl or substituted N-linked heteroaryl, represented by 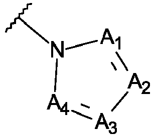
A₁ is CR^{y1} or N;
A₂ is CR^{y2} or N;
A₃ is CR^{y3} or N;
A₄ is CR^{y4} or N;
wherein R^{y1}, R^{y2}, R^{y3} and R^{y4} are independently selected from H, alkyl, or haloalkyl;
and pharmaceutically acceptable salts.

An embodiment of the present invention provides compounds according to formula I as described herein, wherein
R¹ is alkoxy;
R² is haloalkyl, haloalkoxy, or cyclopropyl optionally substituted with cyano;
R³ is H or alkoxy;
R⁵ is H, halo, alkyl, or haloalkyl;
R⁶ is H;
R⁴ is selected from 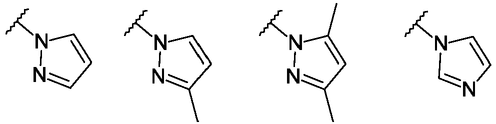 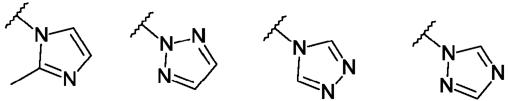 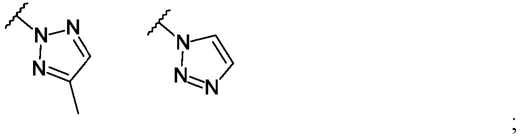
and pharmaceutical salts thereof.

An embodiment of the present invention provides compounds according to formula I as described herein, wherein
R¹ is alkoxy;
R² is haloalkyl, haloalkoxy, or cyclopropyl optionally substituted with cyano;
R³ is alkoxy;
R⁵ is H, halo, alkyl, or haloalkyl;
R⁶ is H;
R⁴ is selected from 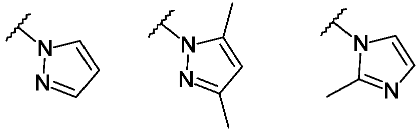 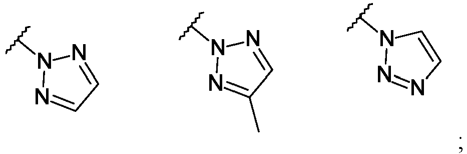
and pharmaceutical salts thereof.

Particular examples of compounds of formula I as described herein are selected from
N-[5-(2,2-difluoroethyl)-4,6-dimethoxy-pyrimidin-2-yl]-7-(3-methylpyrazol-1-yl)-1H-indole-3-sulfonamide;
N-[5-(2,2-difluoroethyl)-4,6-dimethoxy-pyrimidin-2-yl]-7-(3,5-dimethylpyrazol-1-yl)-1H-indole-3-sulfonamide;
N-[5-(2,2-difluoroethyl)-4,6-dimethoxy-pyrimidin-2-yl]-7-imidazol-1-yl-1H-indole-3-sulfonamide;
6-chloro-N-[5-(2,2-difluoroethyl)-4,6-dimethoxy-pyrimidin-2-yl]-7-pyrazol-1-yl-1H-indole-3-sulfonamide;
N-[5-(2,2-difluoroethyl)-4,6-dimethoxy-pyrimidin-2-yl]-7-(2-methylimidazol-1-yl)-1H-indole-3-sulfonamide;
6-chloro-N-[5-(2,2-difluoroethoxy)-4,6-dimethoxy-pyrimidin-2-yl]-7-imidazol-1-yl-1H-indole-3-sulfonamide;
6-chloro-N-[5-(2,2-difluoroethyl)-4,6-dimethoxy-pyrimidin-2-yl]-7-(triazol-2-yl)-1H-indole-3-sulfonamide;
6-chloro-N-[5-(2,2-difluoroethyl)-4-methoxy-pyrimidin-2-yl]-7-imidazol-1-yl-1H-indole-3-sulfonamide;
6-chloro-N-[5-(2,2-difluoroethoxy)-4,6-dimethoxy-pyrimidin-2-yl]-7-(triazol-2-yl)-1H-indole-3-sulfonamide;
6-chloro-N-[5-(2,2-difluoroethyl)-4,6-dimethoxy-pyrimidin-2-yl]-7-(1,2,4-triazol-4-yl)-1H-indole-3-sulfonamide;
N-[5-(2,2-difluoroethoxy)-4,6-dimethoxy-pyrimidin-2-yl]-6-(difluoromethyl)-7-(triazol-2-yl)-1H-indole-3-sulfonami e;
6-chloro-N-[5-(2-fluoroethoxy)-4-methoxy-pyrimidin-2-yl]-7-(triazol-2-yl)-1H-indole-3-sulfonamide;
N-[5-(2,2-difluoroethyl)-4,6-dimethoxy-pyrimidin-2-yl]-7-(triazol-2-yl)-1H-indole-3-sulfonamide;
6-chloro-N-[5-(2-fluoroethoxy)-4,6-dimethoxy-pyrimidin-2-yl]-7-(triazol-2-yl)-1H-indole-3-sulfonamide;
6-chloro-N-[5-(difluoromethoxy)-4,6-dimethoxy-pyrimidin-2-yl]-7-(triazol-2-yl)-1H-indole-3-sulfonamide;
6-chloro-N-[5-(2,3-difluoropropyl)-4,6-dimethoxy-pyrimidin-2-yl]-7-(triazol-2-yl)-1H-indole-3-sulfonamide;
6-chloro-N-[5-(3,3-difluoropropyl)-4,6-dimethoxy-pyrimidin-2-yl]-7-(triazol-2-yl)-1H-indole-3-sulfonamide;
6-chloro-N-[5-(2-cyanocyclopropyl)-4,6-dimethoxy-pyrimidin-2-yl]-7-(triazol-2-yl)-1H-indole-3-sulfonamide;
N-[5-(2-fluoroethoxy)-4,6-dimethoxy-pyrimidin-2-yl]-7-(triazol-2-yl)-1H-indole-3-sulfonamide;
N-[5-(2,2-difluoroethoxy)-4,6-dimethoxy-pyrimidin-2-yl]-7-(triazol-2-yl)-1H-indole-3-sulfonamide;
N-[5-(2-cyanocyclopropyl)-4,6-dimethoxy-pyrimidin-2-yl]-7-(triazol-2-yl)-1H-indole-3-sulfonamide;
N-[5-(2,2-difluoroethyl)-4,6-dimethoxy-pyrimidin-2-yl]-6-methyl-7-(triazol-2-yl)-1H-indole-3-sulfonamide;
N-[5-(2-fluoroethoxy)-4,6-dimethoxy-pyrimidin-2-yl]-6-methyl-7-(triazol-2-yl)-1H-indole-3-sulfonamide;
N-[5-(difluoromethoxy)-4,6-dimethoxy-pyrimidin-2-yl]-6-methyl-7-(triazol-2-yl)-1H-indole-3-sulfonamide;
N-[5-(2,2-difluoroethoxy)-4,6-dimethoxy-pyrimidin-2-yl]-6-methyl-7-(triazol-2-yl)-1H-indole-3-sulfonamide;
N-[5-(2-cyanocyclopropyl)-4,6-dimethoxy-pyrimidin-2-yl]-6-methyl-7-(triazol-2-yl)-1H-indole-3-sulfonamide;
6-bromo-N-[5-(2,2-difluoroethyl)-4,6-dimethoxy-pyrimidin-2-yl]-7-(triazol-2-yl)-1H-indole-3-sulfonamide;
6-bromo-N-[5-(2-fluoroethoxy)-4,6-dimethoxy-pyrimidin-2-yl]-7-(triazol-2-yl)-1H-indole-3-sulfonamide;
6-bromo-N-[5-(difluoromethoxy)-4,6-dimethoxy-pyrimidin-2-yl]-7-(triazol-2-yl)-1H-indole-3-sulfonamide;
6-bromo-N-[5-(2,2-difluoroethoxy)-4,6-dimethoxy-pyrimidin-2-yl]-7-(triazol-2-yl)-1H-indole-3-sulfonamide;
6-bromo-N-[5-(2-cyanocyclopropyl)-4,6-dimethoxy-pyrimidin-2-yl]-7-(triazol-2-yl)-1H-indole-3-sulfonamide;
N-[5-(2,2-difluoroethyl)-4,6-dimethoxy-pyrimidin-2-yl]-6-fluoro-7-(triazol-2-yl)-1H-indole-3-sulfonamide;
6-fluoro-N-[5-(2-fluoroethoxy)-4,6-dimethoxy-pyrimidin-2-yl]-7-(triazol-2-yl)-1H-indole-3-sulfonamide;
N-[5-(difluoromethoxy)-4,6-dimethoxy-pyrimidin-2-yl]-6-fluoro-7-(triazol-2-yl)-1H-indole-3-sulfonamide;
N-[5-(2,2-difluoroethoxy)-4,6-dimethoxy-pyrimidin-2-yl]-6-fluoro-7-(triazol-2-yl)-1H-indole-3-sulfonamide;
N-[5-(2-cyanocyclopropyl)-4,6-dimethoxy-pyrimidin-2-yl]-6-fluoro-7-(triazol-2-yl)-1H-indole-3-sulfonamide;
6-chloro-N-[5-(2,2-difluoroethyl)-4,6-dimethoxy-pyrimidin-2-yl]-7-(1,2,4-triazol-1-yl)-1H-indole-3-sulfonamide;
6-chloro-N-[5-(2,2-difluoroethoxy)-4,6-dimethoxy-pyrimidin-2-yl]-7-(4-methyltriazol-2-yl)-1H-indole-3-sulfonamide;
6-chloro-N-[5-(2-fluoroethoxy)-4,6-dimethoxy-pyrimidin-2-yl]-7-(4-methyltriazol-2-yl)-1H-indole-3-sulfonamide;
6-chloro-N-[5-(2,2-difluoroethyl)-4,6-dimethoxy-pyrimidin-2-yl]-7-(triazol-1-yl)-1H-indole-3-sulfonamide;
6-(difluoromethyl)-N-[5-(2-fluoroethoxy)-4,6-dimethoxy-pyrimidin-2-yl]-7-(triazol-2-yl)-1H-indole-3-sulfonami e;
N-[5-(2,2-difluoroethyl)-4,6-dimethoxy-pyrimidin-2-yl]-6-(difluoromethyl)-7-(triazol-2-yl)-1H-indole-3-sulfonamide; and
pharmaceutically acceptable salts thereof.

Other particular examples of compounds of formula I as described herein are selected from
6-chloro-N-[5-(2,2-difluoroethoxy)-4,6-dimethoxy-pyrimidin-2-yl]-7-(4-methylpyrazol-1-yl)-1H-indole-3-sulfonamide;
6-chloro-N-[5-(2,2-difluoroethoxy)-4,6-dimethoxy-pyrimidin-2-yl]-7-(3-methylpyrazol-1-yl)-1H-indole-3-sulfonamide;
6-chloro-N-[5-(2,2-difluoroethoxy)-4,6-dimethoxy-pyrimidin-2-yl]-7-(4-fluoropyrazol-1-yl)-1H-indole-3-sulfonamide;
6-chloro-N-[5-(2,2-difluoroethoxy)-4,6-dimethoxy-pyrimidin-2-yl]-7-(5-methylpyrazol-1-yl)-1H-indole-3-sulfonamide;
6-chloro-N-[5-(2-fluoroethoxy)-4,6-dimethoxy-pyrimidin-2-yl]-7-(4-fluoropyrazol-1-yl)-1H-indole-3-sulfonamide;
6-chloro-N-[5-(difluoromethoxy)-4,6-dimethoxy-pyrimidin-2-yl]-7-(4-fluoropyrazol-1-yl)-1H-indole-3-sulfonamide;
N-[5-(difluoromethoxy)-4,6-dimethoxy-pyrimidin-2-yl]-6-(difluoromethyl)-7-pyrazol-1-yl-1H-indole-3-sulfonamide;
N-[5-(2,2-difluoroethoxy)-4,6-dimethoxy-pyrimidin-2-yl]-6-(difluoromethyl)-7-pyrazol-1-yl-1H-indole-3-sulfonamide;
6-(difluoromethyl)-N-[5-(2-fluoroethoxy)-4,6-dimethoxy-pyrimidin-2-yl]-7-pyrazol-1-yl-1H-indole-3-sulfonamide;
N-[5-(2,2-difluoroethyl)-4,6-dimethoxy-pyrimidin-2-yl]-6-(difluoromethyl)-7-pyrazol-1-yl-1H-indole-3-sulfonamide;
6-chloro-N-[5-(2,2-difluoroethoxy)-4,6-dimethoxy-pyrimidin-2-yl]-7-(3-fluoropyrazol-1-yl)-1H-indole-3-sulfonamide;
6-chloro-N-[5-(difluoromethoxy)-4,6-dimethoxy-pyrimidin-2-yl]-7-(3-fluoropyrazol-1-yl)-1H-indole-3-sulfonamide;
6-chloro-N-[5-(2,2-difluoroethyl)-4,6-dimethoxy-pyrimidin-2-yl]-7-(3-fluoropyrazol-1-yl)-1H-indole-3-sulfonamide;
6-chloro-N-[5-(2-fluoroethoxy)-4,6-dimethoxy-pyrimidin-2-yl]-7-(3-fluoropyrazol-1-yl)-1H-indole-3-sulfonamide;
6-chloro-N-[4,6-dimethoxy-5-(1,1,2-trifluoroethoxy)pyrimidin-2-yl]-7-(triazol-2-yl)-1H-indole-3-sulfonamide;
6-chloro-N-[5-(2-fluoro-1,1-dimethyl-ethoxy)-4,6-dimethoxy-pyrimidin-2-yl]-7-(triazol-2-yl)-1H-indole-3-sulfonami e;
6-chloro-N-[5-(1,1-dideuterio-2,2-difluoro-ethoxy)-4,6-dimethoxy-pyrimidin-2-yl]-7-(triazol-2-yl)-1H-indole-3-sulfonamide;
6-chloro-N-[5-(cyanomethoxy)-4,6-dimethoxy-pyrimidin-2-yl]-7-(triazol-2-yl)-1H-indole-3-sulfonamide;
6-chloro-N-[5-(2-cyanoethyl)-4,6-dimethoxy-pyrimidin-2-yl]-7-(triazol-2-yl)-1H-indole-3-sulfonamide;
6-chloro-N-(4,6-dimethoxy-5-methyl-pyrimidin-2-yl)-7-(triazol-2-yl)-1H-indole-3-sulfonamide;
N-(5-bromo-4,6-dimethoxy-pyrimidin-2-yl)-6-chloro-7-(triazol-2-yl)-1H-indole-3-sulfonamide;
6-chloro-N-[5-(2,2-difluoroethoxy)-4,6-dimethoxy-pyrimidin-2-yl]-5-fluoro-7-(triazol-2-yl)-1H-indole-3-sulfonami e;
6-chloro-N-[5-(2,2-difluoroethoxy)-4,6-dimethoxy-pyrimidin-2-yl]-7-pyrazol-1-yl-1H-indole-3-sulfonamide;
6-chloro-N-[5-(difluoromethoxy)-4,6-dimethoxy-pyrimidin-2-yl]-7-pyrazol-1-yl-1H-indole-3-sulfonamide;
6-chloro-N-[5-(2-fluoroethoxy)-4,6-dimethoxy-pyrimidin-2-yl]-7-pyrazol-1-yl-1H-indole-3-sulfonamide;
6-chloro-N-[4,6-dimethoxy-5-(1,1,2,2-tetradeuteri o-2-fluoro-ethoxy)pyrimidin-2-yl]-7-pyrazol-1-yl-1H-indole-3-sulfonamide;
6-chloro-N-[5-(cyanomethoxy)-4,6-dimethoxy-pyrimidin-2-yl]-7-pyrazol-1-yl-1H-indole-3-sulfonamide;
6-chloro-N-[5-(2-cyanoethyl)-4,6-dimethoxy-pyrimidin-2-yl]-7-pyrazol-1-yl-1H-indole-3-sulfonamide;
6-chloro-N-[5-(2,2-difluoroethyl)-4-methoxy-pyrimidin-2-yl]-7-pyrazol-1-yl-1H-indole-3-sulfonamide;
6-chloro-N-[5-(1,1-dideuterio-2,2-difluoro-ethyl)-4-methoxy-pyrimidin-2-yl]-7-pyrazol-1-yl-1H-indole-3-sulfonamide;
6-chloro-N-[5-(2-cyanocyclopropyl)-4,6-dimethoxy-pyrimidin-2-yl]-7-pyrazol-1-yl-1H-indole-3-sulfonamide;
N-[5-(2,2-difluoroethoxy)-4,6-dimethoxy-pyrimidin-2-yl]-6-methyl-7-pyrazol-1-yl-1H-indole-3-sulfonamide;
6-bromo-N-[5-(2,2-difluoroethoxy)-4,6-dimethoxy-pyrimidin-2-yl]-7-pyrazol-1-yl-1H-indole-3-sulfonamide;
N-[5-(difluoromethoxy)-4,6-dimethoxy-pyrimidin-2-yl]-6-methyl-7-pyrazol-1-yl-1H-indole-3-sulfonamide;
6-bromo-N-[5-(difluoromethoxy)-4,6-dimethoxy-pyrimidin-2-yl]-7-pyrazol-1-yl-1H-indole-3-sulfonamide;
6-chloro-N-[5-(2,2-difluoroethyl)-4-methoxy-pyrimidin-2-yl]-7-(triazol-2-yl)-1H-indole-3-sulfonamide;
6-chloro-N-[5-(1,1-dideuterio-2,2-difluoro-ethyl)-4-methoxy-pyrimidin-2-yl]-7-(triazol-2-yl)-1H-indole-3-sulfonami e;
N-[5-(2,2-difluoroethyl)-4,6-dimethoxy-pyrimidin-2-yl]-6-methyl-7-pyrazol-1-yl-1H-indole-3-sulfonamide;
6-bromo-N-[5-(2,2-difluoroethyl)-4,6-dimethoxy-pyrimidin-2-yl]-7-pyrazol-1-yl-1H-indole-3-sulfonamide;
N-[5-(2-fluoroethoxy)-4,6-dimethoxy-pyrimidin-2-yl]-6-methyl-7-pyrazol-1-yl-1H-indole-3-sulfonamide;
6-bromo-N-[5-(2-fluoroethoxy)-4,6-dimethoxy-pyrimidin-2-yl]-7-pyrazol-1-yl-1H-indole-3-sulfonamide;
6-bromo-N-[4,6-dimethoxy-5-(1,1,2,2-tetradeuterio-2-fluoro-ethoxy)pyrimidin-2-yl]-7-pyrazol-1-yl-1H-indole-3-sulfonamide;
6-chloro-N-[4,6-dimethoxy-5-(1,1,2,2-tetradeuteri o-2-fluoro-ethoxy)pyrimidin-2-yl]-7-(triazol-2-yl)-1H-indole-3-sulfonamide;
6-chloro-N-[5-(1,1-dideuterio-2,2-difluoro-ethyl)-4,6-dimethoxy-pyrimidin-2-yl]-7-pyrazol-1-yl-1H-indole-3-sulfonamide;
and pharmaceutically acceptable salts thereof.

Preferred examples of compounds of formula I as described herein are selected from
N-[5-(2,2-difluoroethyl)-4,6-dimethoxy-pyrimidin-2-yl]-7-(3,5-dimethylpyrazol-1-yl)-1H-indole-3-sulfonamide;
6-chloro-N-[5-(2,2-difluoroethyl)-4,6-dimethoxy-pyrimidin-2-yl]-7-pyrazol-1-yl-1H-indole-3-sulfonamide;
N-[5-(2,2-difluoroethyl)-4,6-dimethoxy-pyrimidin-2-yl]-7-(2-methylimidazol-1-yl)-1H-indole-3-sulfonamide;
6-chloro-N-[5-(2,2-difluoroethyl)-4,6-dimethoxy-pyrimidin-2-yl]-7-(triazol-2-yl)-1H-indole-3-sulfonamide;
6-chloro-N-[5-(2,2-difluoroethoxy)-4,6-dimethoxy-pyrimidin-2-yl]-7-(triazol-2-yl)-1H-indole-3-sulfonamide;
6-chloro-N-[5-(2-fluoroethoxy)-4,6-dimethoxy-pyrimidin-2-yl]-7-(triazol-2-yl)-1H-indole-3-sulfonamide;
6-chloro-N-[5-(difluoromethoxy)-4,6-dimethoxy-pyrimidin-2-yl]-7-(triazol-2-yl)-1H-indole-3-sulfonamide;
6-chloro-N-[5-(2,3-difluoropropyl)-4,6-dimethoxy-pyrimidin-2-yl]-7-(triazol-2-yl)-1H-indole-3-sulfonamide;
6-chloro-N-[5-(3,3-difluoropropyl)-4,6-dimethoxy-pyrimidin-2-yl]-7-(triazol-2-yl)-1H-indole-3-sulfonamide;
6-chloro-N-[5-(2-cyanocyclopropyl)-4,6-dimethoxy-pyrimidin-2-yl]-7-(triazol-2-yl)-1H-indole-3-sulfonamide;
N-[5-(2,2-difluoroethyl)-4,6-dimethoxy-pyrimidin-2-yl]-6-methyl-7-(triazol-2-yl)-1H-indole-3-sulfonamide;
N-[5-(2-fluoroethoxy)-4,6-dimethoxy-pyrimidin-2-yl]-6-methyl-7-(triazol-2-yl)-1H-indole-3-sulfonamide;
N-[5-(difluoromethoxy)-4,6-dimethoxy-pyrimidin-2-yl]-6-methyl-7-(triazol-2-yl)-1H-indole-3-sulfonamide;
N-[5-(2,2-difluoroethoxy)-4,6-dimethoxy-pyrimidin-2-yl]-6-methyl-7-(triazol-2-yl)-1H-indole-3-sulfonamide;
N-[5-(2-cyanocyclopropyl)-4,6-dimethoxy-pyrimidin-2-yl]-6-methyl-7-(triazol-2-yl)-1H-indole-3-sulfonamide;
6-bromo-N-[5-(2,2-difluoroethyl)-4,6-dimethoxy-pyrimidin-2-yl]-7-(triazol-2-yl)-1H-indole-3-sulfonamide;
6-bromo-N-[5-(2-fluoroethoxy)-4,6-dimethoxy-pyrimidin-2-yl]-7-(triazol-2-yl)-1H-indole-3-sulfonamide;
6-bromo-N-[5-(difluoromethoxy)-4,6-dimethoxy-pyrimidin-2-yl]-7-(triazol-2-yl)-1H-indole-3-sulfonamide;
6-bromo-N-[5-(2,2-difluoroethoxy)-4,6-dimethoxy-pyrimidin-2-yl]-7-(triazol-2-yl)-1H-indole-3-sulfonamide;
6-bromo-N-[5-(2-cyanocyclopropyl)-4,6-dimethoxy-pyrimidin-2-yl]-7-(triazol-2-yl)-1H-indole-3-sulfonamide;
6-chloro-N-[5-(2,2-difluoroethoxy)-4,6-dimethoxy-pyrimidin-2-yl]-7-(4-methyltriazol-2-yl)-1H-indole-3-sulfonamide;
6-chloro-N-[5-(2-fluoroethoxy)-4,6-dimethoxy-pyrimidin-2-yl]-7-(4-methyltriazol-2-yl)-1H-indole-3-sulfonamide;
6-chloro-N-[5-(2,2-difluoroethyl)-4,6-dimethoxy-pyrimidin-2-yl]-7-(triazol-1-yl)-1H-indole-3-sulfonamide;
N-[5-(2,2-difluoroethyl)-4,6-dimethoxy-pyrimidin-2-yl]-6-(difluoromethyl)-7-(triazol-2-yl)-1H-indole-3-sulfonamide; and
pharmaceutically acceptable salts thereof.

Other preferred examples of compounds of formula I as described herein are selected from
6-chloro-N-[5-(2,2-difluoroethoxy)-4,6-dimethoxy-pyrimidin-2-yl]-7-(4-methylpyrazol-1-yl)-1H-indole-3-sulfonamide;
6-chloro-N-[5-(2,2-difluoroethoxy)-4,6-dimethoxy-pyrimidin-2-yl]-7-(3-methylpyrazol-1-yl)-1H-indole-3-sulfonamide;
6-chloro-N-[5-(2,2-difluoroethoxy)-4,6-dimethoxy-pyrimidin-2-yl]-7-(4-fluoropyrazol-1-yl)-1H-indole-3-sulfonamide;
6-chloro-N-[5-(2,2-difluoroethoxy)-4,6-dimethoxy-pyrimidin-2-yl]-7-(5-methylpyrazol-1-yl)-1H-indole-3-sulfonamide;
6-chloro-N-[5-(2-fluoroethoxy)-4,6-dimethoxy-pyrimidin-2-yl]-7-(4-fluoropyrazol-1-yl)-1H-indole-3-sulfonamide;
6-chloro-N-[5-(2,2-difluoroethoxy)-4,6-dimethoxy-pyrimidin-2-yl]-7-(3-fluoropyrazol-1-yl)-1H-indole-3-sulfonamide;
6-chloro-N-[5-(difluoromethoxy)-4,6-dimethoxy-pyrimidin-2-yl]-7-(3-fluoropyrazol-1-yl)-1H-indole-3-sulfonamide;
6-chloro-N-[5-(2,2-difluoroethyl)-4,6-dimethoxy-pyrimidin-2-yl]-7-(3-fluoropyrazol-1-yl)-1H-indole-3-sulfonamide;
6-chloro-N-[5-(2-fluoroethoxy)-4,6-dimethoxy-pyrimidin-2-yl]-7-(3-fluoropyrazol-1-yl)-1H-indole-3-sulfonamide;
6-chloro-N-[5-(cyanomethoxy)-4,6-dimethoxy-pyrimidin-2-yl]-7-(triazol-2-yl)-1H-indole-3-sulfonamide;
6-chloro-N-[5-(2-cyanoethyl)-4,6-dimethoxy-pyrimidin-2-yl]-7-(triazol-2-yl)-1H-indole-3-sulfonamide;
6-chloro-N-(4,6-dimethoxy-5-methyl-pyrimidin-2-yl)-7-(triazol-2-yl)-1H-indole-3-sulfonamide;
N-(5-bromo-4,6-dimethoxy-pyrimidin-2-yl)-6-chloro-7-(triazol-2-yl)-1H-indole-3-sulfonamide;
6-chloro-N-[5-(2,2-difluoroethoxy)-4,6-dimethoxy-pyrimidin-2-yl]-7-pyrazol-1-yl-1H-indole-3-sulfonamide;
6-chloro-N-[5-(difluoromethoxy)-4,6-dimethoxy-pyrimidin-2-yl]-7-pyrazol-1-yl-1H-indole-3-sulfonamide;
6-chloro-N-[5-(2-fluoroethoxy)-4,6-dimethoxy-pyrimidin-2-yl]-7-pyrazol-1-yl-1H-indole-3-sulfonamide;
6-chloro-N-[4,6-dimethoxy-5-(1,1,2,2-tetradeuteri o-2-fluoro-ethoxy)pyrimidin-2-yl]-7-pyrazol-1-yl-1H-indole-3-sulfonamide;
6-chloro-N-[5-(2-cyanoethyl)-4,6-dimethoxy-pyrimidin-2-yl]-7-pyrazol-1-yl-1H-indole-3-sulfonamide;
6-chloro-N-[5-(1,1-dideuterio-2,2-difluoro-ethyl)-4-methoxy-pyrimidin-2-yl]-7-pyrazol-1-yl-1H-indole-3-sulfonamide;
6-chloro-N-[5-(2-cyanocyclopropyl)-4,6-dimethoxy-pyrimidin-2-yl]-7-pyrazol-1-yl-1H-indole-3-sulfonamide;
6-bromo-N-[5-(2,2-difluoroethoxy)-4,6-dimethoxy-pyrimidin-2-yl]-7-pyrazol-1-yl-1H-indole-3-sulfonamide;
6-bromo-N-[5-(difluoromethoxy)-4,6-dimethoxy-pyrimidin-2-yl]-7-pyrazol-1-yl-1H-indole-3-sulfonamide;
6-chloro-N-[5-(1,1-dideuterio-2,2-difluoro-ethyl)-4-methoxy-pyrimidin-2-yl]-7-(triazol-2-yl)-1H-indole-3-sulfonami e;
N-[5-(2,2-difluoroethyl)-4,6-dimethoxy-pyrimidin-2-yl]-6-methyl-7-pyrazol-1-yl-1H-indole-3-sulfonamide;
6-bromo-N-[5-(2,2-difluoroethyl)-4,6-dimethoxy-pyrimidin-2-yl]-7-pyrazol-1-yl-1H-indole-3-sulfonamide;
6-bromo-N-[5-(2-fluoroethoxy)-4,6-dimethoxy-pyrimidin-2-yl]-7-pyrazol-1-yl-1H-indole-3-sulfonamide;
6-bromo-N-[4,6-dimethoxy-5-(1,1,2,2-tetradeuterio-2-fluoro-ethoxy)pyrimidin-2-yl]-7-pyrazol-1-yl-1H-indole-3-sulfonamide;
6-chloro-N-[4,6-dimethoxy-5-(1,1,2,2-tetradeuteri o-2-fluoro-ethoxy)pyrimidin-2-yl]-7-(triazol-2-yl)-1H-indole-3-sulfonamide;
6-chloro-N-[5-(1,1-dideuterio-2,2-difluoro-ethyl)-4,6-dimethoxy-pyrimidin-2-yl]-7-pyrazol-1-yl-1H-indole-3-sulfonamide;
and pharmaceutically acceptable salts thereof.

Most preferred examples of compounds of formula I as described herein are selected from
6-chloro-N-[5-(2,2-difluoroethoxy)-4,6-dimethoxy-pyrimidin-2-yl]-7-(triazol-2-yl)-1H-indole-3-sulfonamide;
6-chloro-N-[5-(difluoromethoxy)-4,6-dimethoxy-pyrimidin-2-yl]-7-(triazol-2-yl)-1H-indole-3-sulfonamide;
and pharmaceutically acceptable salts thereof.

Processes for the manufacture of compounds of formula I as described herein are an object of the invention.

The present compounds of formula I and their pharmaceutically acceptable salts can be prepared by methods known in the art, for example, by processes described below, which process comprises reacting a compound of formula III with a compound of formula II in the presence of a base selected from N-ethyldiisopropylamine, pyridine, potassium phosphate or sodium hydride, to provide a compound of formula I, 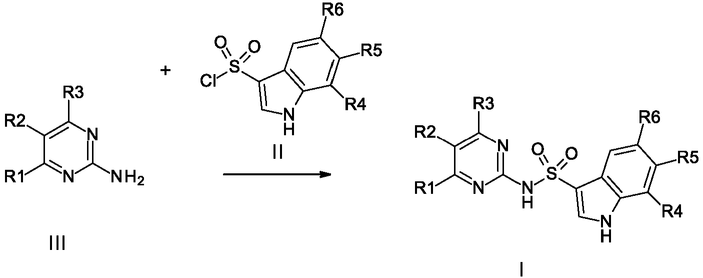 wherein R¹, R², R³, R³, R⁴, R⁵ and R⁶ are as described above.

### General Synthetic Schemes

The compounds of formula I may be prepared in accordance with the process variant described above and with the following scheme 1. The starting materials are commercially available or may be prepared in accordance with known methods.

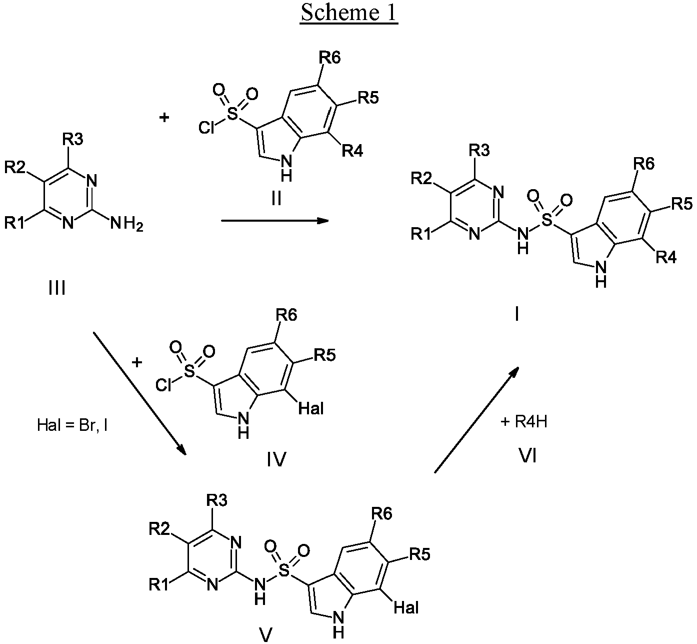

Compounds of general formula I can be prepared by reacting sulfonylchloride II with amines III in the presence of a base like N-ethyldiisopropylamine, pyridine, potassium phosphate or sodium hydride. Furthermore, compounds of general formula I can be obtained by a reaction of amine III with sulfonylchloride IV and a base as described before, followed by reaction of the resulting compound V with the heterocycle VI using a suitable palladium or a copper catalyst and a base.

The starting materials are commercially available or may be prepared in accordance with known methods or methods described in the following schemes.

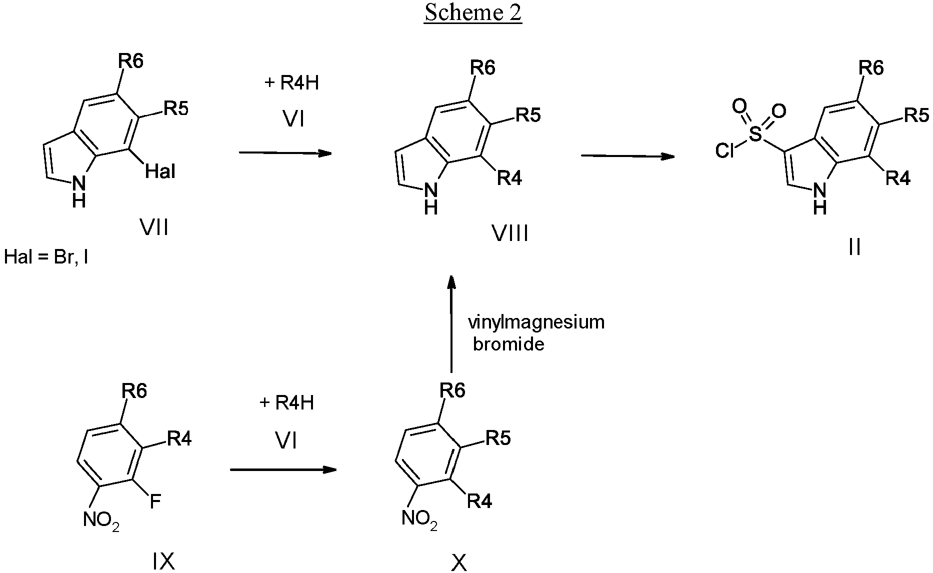

Intermediate of formula II can be prepared by reaction of compound VII with the heterocycle VI using a suitable palladium or a copper catalyst and a base, followed by reaction of the resulting compound VIII with a chlorosulfonylating agent like chlorosulfonic acid, or a sulfonylating agent like sulfuric acid or sulfur trioxide N,N-dimethylformamide complex, followed by chlorination of the intermediate sulfonic acid with a chlorinating agent like thionylchloride. Furthermore, a 2-fluoronitro derivative IX can be reacted with heterocycle VI- and a base to form intermediate X, which can undergo a Bartoli reaction in the presence of vinylmagnesium bromide to produce compound VIII in an alternative route.

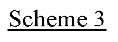

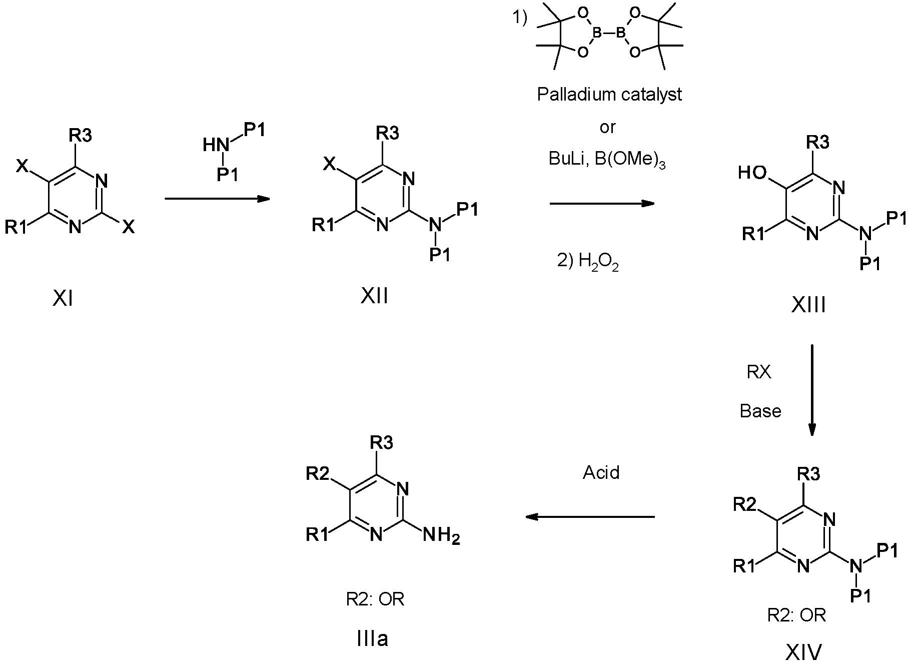

2-Amino-pyrimidine of formula IIIa wherein R² is an alkoxy group can be prepared by deprotection of intermediate XIV in the presence of an acid like trifluoro acetic acid wherein P1 is a protective group like p-methoxy-benzyl, 3-4-dimethoxybenzyl or a Boc group. XIV can be obtained by alkylation of alcohol XIII in the presence of a base like cesium- or potassium-carbonate or sodium- or potassium-hydroxide and an alkylating agent RX. Alcohol XIII can be prepared from dihalogenated starting material XI, by reacting XI with a protected amine to provide intermediate XII which is first transformed into a boronic ester that is then oxidized in the presence of an oxidant like hydrogen peroxide.

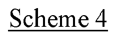

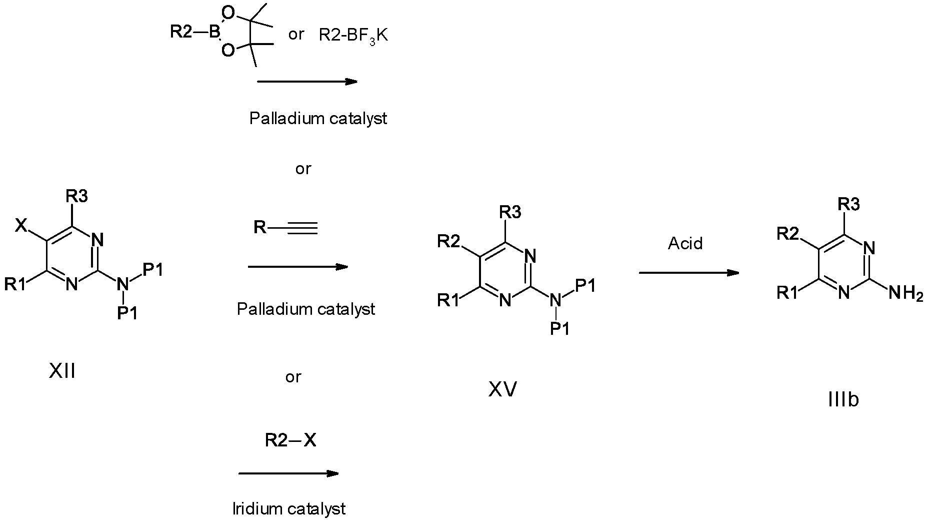

2-Amino-pyrimidine of formula IIIb wherein R² is an alkyl, alkenylalkyl, alkynyl, cyanoalkyl, cycloalkyl, heterocycloalkyl can be prepared by deprotection of intermediate XV in the presence of an acid like trifluoro acetic acid wherein P1 is a protective group like p-methoxy-benzyl or a Boc group. Compound XV can be obtained from intermediate XII under well known metal-catalyzed cross coupling reactions conditions.

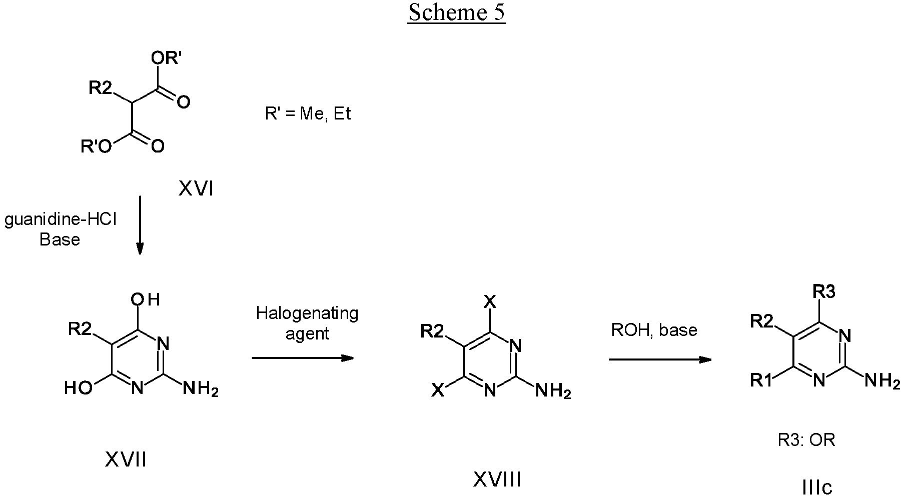

2-Amino-pyrimidine of formula IIIc wherein R1 and R3 is an alkoxy group can be prepared by reaction of halogenated starting material XVIII in the presence of an alcohol and a base like sodium hydride. Compound XVII can be prepared from malonester XVI by its reaction with guanidine-hydrochloride salt in the presence of a base like sodium methoxide to provide intermediate XVII which is then reacted with a halogenating agent like phosphorus oxychloride to give halogenated starting material XVIII.

Another embodiment of the invention provides a pharmaceutical composition or medicament containing a compound of the invention and a therapeutically inert carrier, diluent or excipient, as well as a method of using the compounds of the invention to prepare such composition and medicament. In one example, the compound of formula I may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula I is formulated in an acetate buffer, at pH 5. In another embodiment, the compound of formula I is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The compounds of formula I and their pharmaceutically acceptable salts can be processed with pharmaceutically inert, inorganic or organic adjuvants for the production of tablets, coated tablets, dragées,hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

The invention also relates in particular to:
A compound of formula I for use as therapeutically active substance;

A compound of formula I for use in the treatment of a disease modulated by GPR17;

Likewise an object of the present invention is a pharmaceutical composition comprising a compound according to formula I as described herein and a therapeutically inert carrier.

The use of a compound of formula I for the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity.

An embodiment of the present invention is the use of a compound of formula I for the treatment or prophylaxis of multiple sclerosis, Alzheimer's disease, Parkinson's disease, or Huntington's disease.

A particular embodiment of the invention is the use of a compound of formula I for the treatment or prophylaxis of multiple sclerosis.

The use of a compound of formula I for the preparation of a medicament for the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity.

An embodiment of the present invention is the use of a compound of formula I for the preparation of a medicament for the treatment or prophylaxis of multiple sclerosis, Alzheimer's disease, Parkinson's disease, or Huntington's disease.

A particular embodiment of the invention is the use of a compound of formula I for the preparation of a medicament for the treatment or prophylaxis of multiple sclerosis.

A compound according to formula I for use in the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity.

An embodiment of the present invention is a compound of formula I for use in the treatment or prophylaxis of multiple sclerosis, Alzheimer's disease, Parkinson's disease, or Huntington's disease.

A particular embodiment of the invention is a compound according to formula I for use in the treatment or prophylaxis of multiple sclerosis.

A method for the treatment or propylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity, which method comprises administering an effective amount of a compound of formula I to a patient in need thereof.

An embodiment of the present invention is a method for the treatment or prophylaxis of multiple sclerosis, Alzheimer's disease, Parkinson's disease, or Huntington's disease, which method comprises administering an effective amount of a compound of formula I to a patient in need thereof.

A particular embodiment of the invention is a method for the treatment or prophylaxis of multiple sclerosis, which method comprises administering an effective amount of a compound of formula I to a patient in need thereof.

Also an embodiment of the present invention provides compounds of formula I as described herein, when manufactured according to any one of the described processes.

### Assay Procedures

### GPR17 cAMP Assay Protocol:

CHO-K1 cells stably expressing vector containing untagged human GPR17 short isoform (Roche) were cultured at 37°C / 5% CO2 in DMEM (Dulbecco's Modified Eagle Medium):F-12 (1:1) supplemented with 10% foetal bovine serum and 400 µg/ml Geneticin.

Changes in intracellular cyclic adenosine monophosphate (cAMP) levels were quantified using the Nano-TRF Detection Assay kit (Roche Diagnostics, Cat. No. 05214386001). This assay allows for direct cAMP quantification in a homogeneous solution. cAMP is detected based on time-resolved fluorescence energy transfer (TR-FRET) and competitive binding of ruthenylated cAMP and endogenous cAMP to an anti-cAMP monoclonal antibody labeled with AlexaFluor-700. The Ruthenium complex serves as the FRET donor and transfers energy to AlexaFluor-700. The FRET signal is inversely proportional to the cAMP concentration.

CHO-GPR17S cells were detached with Accutase and resuspended in assay buffer consisting of Hank's Balanced Salt Solution (HBSS), 10mM HEPES (4-(2-hydroxyethyl) piperazine-1-ethanesulfonic acid solution) and 0.1% bovine serum albumin (pH 7.4). The cells were seeded in black 384-well plates (Corning) at a density of 10'000 cells / 20µl assay buffer until the addition of compounds.

Test antagonist compounds were serially diluted in dimethyl sulfoxide (DMSO) and spotted in 384-well plates. The compounds were then diluted in HBSS buffer supplemented with an EC80 concentration of MDL29,951 (3-(2-Carboxy-4,6-dichloroindol-3-yl)propionic acid) (GPR17 agonist) plus 3-Isobutyl-1-methylxanthine (IBMX) (0.5mM final concentration) and added to the cells at room temperature. Forskolin (15µM final concentration) was added 5 minutes after the test compounds and the cells were incubated at room temperature for 30 minutes. The assay was stopped by adding cAMP detection mix (containing detergents for cell lysis) for 90 minutes at room temperature.

Cellular cAMP was measured using a Paradigm reader (Molecular Devices). The raw data was used to calculate the FRET signal based on the assay's P-factor as per cAMP kit instructions. The data was normalized to the maximal activity of a reference antagonist and dose response curves were fitted to the percent activity of the test compounds using a sigmoidal dose response model (Genedata Screener).

Results in the hGPR17 cAMP assay are provided for compounds of formula I in Table 1

**Table 1:**

| **Example number** | **hGPR17 cAMP IC50 [µM]** |
|---|---|
| 1 | 0.068 |
| 2 | 0.032 |
| 3 | 0.300 |
| 4 | 0.022 |
| 5 | 0.048 |
| 6 | 0.050 |
| 7 | 0.021 |
| 8 | 0.248 |
| 9 | 0.032 |
| 10 | 0.100 |
| 11 | 0.074 |
| 12 | 0.052 |
| 13 | 0.203 |
| 14 | 0.028 |
| 15 | 0.025 |
| 16 | 0.024 |
| 17 | 0.024 |
| 18 | 0.025 |
| 19 | 0.316 |
| 20 | 0.447 |
| 21 | 0.195 |
| 22 | 0.021 |
| 23 | 0.035 |
| 24 | 0.038 |
| 25 | 0.034 |
| 26 | 0.025 |
| 27 | 0.019 |
| 28 | 0.027 |
| 29 | 0.022 |
| 30 | 0.023 |
| 31 | 0.030 |
| 32 | 0.069 |
| 33 | 0.141 |
| 34 | 0.226 |
| 35 | 0.126 |
| 36 | 0.082 |
| 37 | 0.060 |
| 38 | 0.034 |
| 39 | 0.055 |
| 40 | 0.040 |
| 41 | 0.064 |
| 42 | 0.050 |
| 43 | 0.029 |
| 44 | 0.023 |
| 45 | 0.025 |
| 46 | 0.045 |
| 47 | 0.026 |
| 48 | 0.069 |
| 49 | 0.108 |
| 50 | 0.065 |
| 51 | 0.094 |
| 52 | 0.068 |
| 53 | 0.025 |
| 54 | 0.027 |
| 55 | 0.025 |
| 56 | 0.049 |
| 57 | 0.085 |
| 58 | 1.266 |
| 59 | 0.074 |
| 60 | 0.049 |
| 61 | 0.029 |
| 62 | 0.019 |
| 63 | 0.037 |
| 64 | 0.075 |
| 65 | 0.022 |
| 66 | 0.021 |
| 67 | 0.031 |
| 68 | 0.036 |
| 69 | 0.052 |
| 70 | 0.037 |
| 71 | 0.063 |
| 72 | 0.041 |
| 73 | 0.041 |
| 74 | 0.059 |
| 75 | 0.042 |
| 76 | 0.075 |
| 77 | 0.027 |
| 78 | 0.055 |
| 79 | 0.036 |
| 80 | 0.029 |
| 81 | 0.018 |
| 82 | 0.068 |
| 83 | 0.036 |
| 84 | 0.026 |
| 85 | 0.042 |
| 86 | 0.044 |

The invention will now be illustrated by the following examples which have no limiting character.

In case the preparative examples are obtained as a mixture of enantiomers, the pure enantiomers can be obtained by methods described herein or by methods known to those skilled in the art, such as e.g. chiral chromatography or crystallization.

### Examples

All examples and intermediates were prepared under nitrogen atmosphere if not specified otherwise.

### Intermediates A

### Intermediate A1: 7-(2-methylimidazol-1-yl)-1H-indole-3-sulfonyl chloride

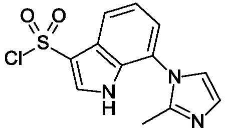

### Step 1: 7-(2-methylimidazol-1-yl)-1H-indole

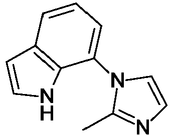

In a glass tube, 7-bromo-1H-indole (1 g, 5.1 mmol), tert-butanol (20 ml), 2-methylimidazole (628 mg, 7.65 mmol), 8-hydroxyquinoline (111 mg, 0.765 mmol). copper(I) iodide (97 mg, 0.51 mmol) and cesium carbonate (4.16 g, 12.7 mmol) were added under argon and the reaction mixture was stirred at 110°C overnight. The mixture was filtered over dicalite and washed with ethyl acetate. The filtrate was concentrated in vacuo. The crude material was purified by flash chromatography (Silicycle SiliaSep Amine, 0 % to 100 % ethyl acetate in heptane) followed by a second chromatography (silica gel, 0 % to 10 % methanol in ethyl acetate) to provide 7-(2-methylimidazol-1-yl)-1H-indole (170 mg, 16% yield) as off-white solid. MS (ESI) m/z= 198.1 [M+H]⁺

### Step 2: 7-(2-methylimidazol-1-yl)-1H-indole-3-sulfonyl chloride

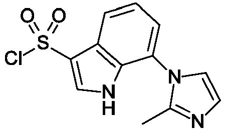

A suspension of 7-(2-methylimidazol-1-yl)-1H-indole (267 mg, 1.33 mmol) in acetonitrile (8 ml) was cooled to 0°C and chlorosulfonic acid (464 mg, 267 ul, 3.98 mmol) was added. The ice-bath was removed and the reaction mixture was stirred 3 h at room temperature. The reaction mixture was poured into ice and water, then the water was concentrated in vacuo. The residue was suspended in acetonitrile, filtered and dried. This material was suspended in acetonitrile (8 ml), phosphorus oxychloride (814 mg, 495 ul, 5.31 mmol) was added and the reaction mixture was stirred at 70°C overnight. The mixture was poured into ice and water and extracted with ethyl acetate twice. The combined organic layers was washed with water and brine, dried over sodium sulfate, filtered and concentrated in vacuo. The residue was triturated in dichloromethane, filtered and washed with dichloromethane to afford the title compound (374 mg, 95% yield) as off-white solid. MS (ESI) m/z= 296.1 [M+H]⁺

### Intermediate A2: 6-chloro-7-(triazol-2-yl)-1H-indole-3-sulfonyl chloride

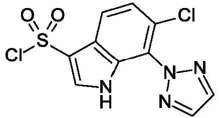

### Step 1: 2-(2-chloro-6-nitro-phenyl)triazole and 1-(2-chloro-6-nitro-phenyl)triazole

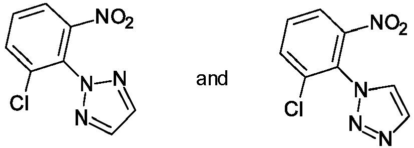

A mixture of 1-chloro-2-fluoro-3-nitro-benzene (1.49 g, 1 ml, 8.49 mmol), 1,2,3-triazole (704 mg, 592 ul, 10.2 mmol) and potassium carbonate (3.52 g, 25.5 mmol) in 1,4-dioxane (20 ml) was stirred at 80°C. After cooling to room temperature, the solid was filtered off and washed with ethyl acetate. The filtrate was concentrated in vacuo. The crude material was purified by flash chromatography (silica gel, 0 % to 50 % ethyl acetate in heptane) to afford 1-(2-chloro-6-nitrophenyl)triazole (1.7 g, 85% yield) as light yellow solid and 2-(2-chloro-6-nitro-phenyl)triazole (459 mg, 23% yield) as light yellow solid. MS (ESI) m/z= 225.1 [M+H]⁺ and MS (ESI) m/z= 225.0 [M+H]⁺

### Step 2: 6-chloro-7-(triazol-2-yl)-1H-indole

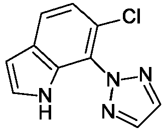

In a 500 ml three-necked flask, 2-(2-chloro-6-nitro-phenyl)triazole (2.52 g, 11.22 mmol) was dissolved in tetrahydrofuran (extra dry, 130 ml) and the solution was cooled to -60°C. Vinylmagnesium bromide solution (1M in THF, 45 ml, 45 mmol) was added and the reaction mixture was further stirred at -60°C for 1 h. The reaction mixture was quenched with saturated ammonium chloride solution (100 ml), poured into water and extracted with ethyl acetate twice. The combined organic layers were washed with brine, dried over sodium sulfate, filtered and concentrated in vacuo. The crude material was purified by flash chromatography (silica gel, 0 % to 50 % ethyl acetate in heptane) to afford 6-chloro-7-(triazol-2-yl)-1H-indole (1.59 g, 64% yield) as white solid. MS (ESI) m/z= 219.1 [M+H]⁺

### Step 3: 6-chloro-7-(triazol-2-yl)-1H-indole-3-sulfonyl chloride

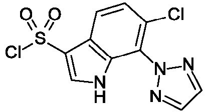

A solution of 6-chloro-7-(triazol-2-yl)-1H-indole (1.59 g, 7.27 mmol) in acetonitrile (110 ml) was cooled to 0°C and chlorosulfonic acid (2.54 g, 1.46 ml, 21.82 mmol) was added. The ice-bath was removed and the reaction mixture was stirred 2 h at room temperature. Phosphorus oxychloride (4.46 g, 2.71 ml, 29.1 mmol) was added and the reaction mixture was stirred at 60°C overnight. The mixture was poured into ice and water and extracted with ethyl acetate twice. The combined organic layers were washed with ice and brine, dried over sodium sulfate and concentrated in vacuo. The crude material was suspended in dichloromethane, filtered and washed with small amount of dichloromethane to afford the title compound (1.9 g, 78% yield) as light grey solid. MS (ESI) m/z= 317.0 [M+H]⁺

### Intermediate A3: 6-chloro-7-imidazol-1-yl-1H-indole-3-sulfonyl chloride

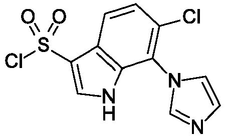

The title compound was prepared in analogy to Intermediate A1 from 7-bromo-6-chloro-1H-indole instead of 7-bromo-1H-indole and imidazole instead of 2-methylimidazole in Step 1) as a white solid.

### Intermediate A4: 6-chloro-7-(triazol-1-yl)-1H-indole-3-sulfonyl chloride

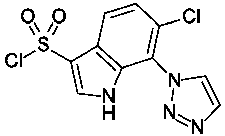

The title compound was prepared in analogy to Intermediate A2 from 1-(2-chloro-6-nitrophenyl)triazole instead of 2-(2-chloro-6-nitro-phenyl)triazole in Step 2) as a white solid. MS (ESI) m/z= 317.0 [M+H]⁺

### Intermediate A5: 6-(difluoromethyl)-7-(triazol-2-yl)-1H-indole-3-sulfonyl chloride

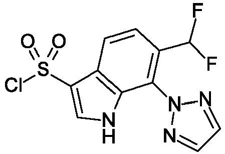

### Step 1: 1-(difluoromethyl)-2-fluoro-3-nitro-benzene

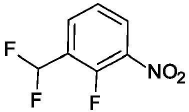

To a solution of 2-fluoro-3-nitro-benzaldehyde (1.2 g, 7.1 mmol) in dichloromethane (18 ml) was added at 5 °C dropwise over a period of 15 min diethylaminosulfur trifluoride (1M in dichloromethane, 28.5 ml, 28.5 mmol) and the mixture was stirred at 5 °C for 2.5 h. The mixture was quenched carefully dropwise with saturated sodium bicarbonate solution (80 ml). The mixture was stirred for 10 min at room temperature, then extracted with dichloromethane twice. The organic layers were dried over sodium sulfate, filtered and concentrated in vacuo. The crude material was purified by flash chromatography (silica gel, 0 % to 50 % ethyl acetate in heptane) to provide 1-(difluoromethyl)-2-fluoro-3-nitro-benzene (1.14 g, 84% yield) as a brown liquid.

### Step 2: 2-[2-(difluoromethyl)-6-nitro-phenyl]triazole

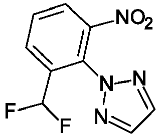

A mixture of 1-(difluoromethyl)-2-fluoro-3-nitro-benzene (1.2 g, 6.28 mmol), 1,2,3-triazole (520 mg, 7.53 mmol) and potassium carbonate (2.6 g, 18.8 mmol) in 1,4-dioxane (15 ml) was stirred at 80°C for 5 h. After cooling to room temperature, the solid was filtered off and washed with ethyl acetate. The filtrate was concentrated in vacuo. The crude material was purified by flash chromatography (silica gel, 0 % to 50 % ethyl acetate in heptane) to afford 2-[2-(difluoromethyl)-6-nitro-phenyl]triazole (320 mg, 21% yield) as brown semisolid. MS (ESI) m/z= 241.1 [M+H]⁺

### Step 3: 6-(difluoromethyl)-7-(triazol-2-yl)-1H-indole

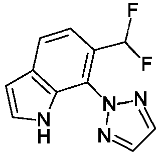

In a 50 ml three-necked flask, 2-[2-(difluoromethyl)-6-nitro-phenyl]triazole (322 mg, 1.34 mmol) was dissolved in tetrahydrofuran (extra dry, 15 mL) and the solution was cooled to -60°C. Vinylmagnesium bromide solution (1M in THF, 5.4 mL, 5.4 mmol) was added and the reaction mixture was further stirred at -60°C for 1 h. The reaction was quenched with saturated ammonium chloride solution, poured into water and extracted with ethyl acetate twice. The combined organic layers were washed with brine, dried over sodium sulfate, filtered and concentrated in vacuo. The crude material was purified by flash chromatography (silica gel, 0 % to 50 % ethyl acetate in heptane) to afford 6-(difluoromethyl)-7-(triazol-2-yl)-1H-indole (180 mg, 57%) as off-white solid. MS (ESI) m/z= 233.2 [M-H]⁻

### Step 4: 6-(difluoromethyl)-7-(triazol-2-yl)-1H-indole-3-sulfonyl chloride

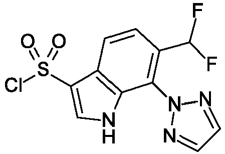

A solution of 6-(difluoromethyl)-7-(triazol-2-yl)-1H-indole (180 mg, 769 umol) in acetonitrile (12 ml) was cooled to 0°C and chlorosulfonic acid (269 mg, 154 ul, 2.31 mmol) was added. The ice-bath was removed and the reaction mixture was stirred at room temperature for 2 h. Phosphorus oxychloride (471 mg, 286 ul, 3.07 mmol) was added and the reaction mixture was further stirred at room temperature overnight. The reaction mixture was poured into ice and water and extracted with ethyl acetate twice. The combined organic layers were washed with ice and brine, dried over sodium sulfate and concentrated in vacuo to afford the title compound (202 mg, 79% yield) as brown semisolid. MS (ESI) m/z= 331.1 [M-H]⁻

### Intermediate A6: 7-(triazol-2-yl)-1H-indole-3-sulfonyl chloride

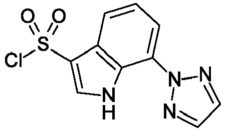

The title compound was prepared in analogy to Intermediate A2 from 2-fluoro-3-nitro-benzene instead of 1-chloro-2-fluoro-3-nitro-benzene in Step 1) as a purple solid. MS (ESI) m/z= 281.0 [M-H]⁻

### Intermediate A7: 6-methyl-7-(triazol-2-yl)-1H-indole-3-sulfonyl chloride

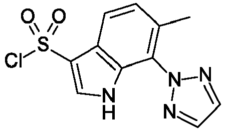

The title compound was prepared in analogy to Intermediate A2 from 2-fluoro-1-methyl-3-nitrobenzene instead of 1-chloro-2-fluoro-3-nitro-benzene in Step 1) as a purple solid. MS (ESI) m/z= 295.0 [M-H]⁻

### Intermediate A8: 6-bromo-7-(triazol-2-yl)-1H-indole-3-sulfonyl chloride

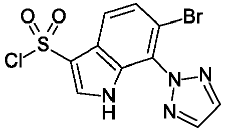

The title compound was prepared in analogy to Intermediate A2 from 1-bromo-2-fluoro-3-nitrobenzene instead of 1-chloro-2-fluoro-3-nitro-benzene in Step 1) as a grey solid. MS (ESI) m/z= 361.0 [M-H]⁻

### Intermediate A9: 6-fluoro-7-(triazol-2-yl)-1H-indole-3-sulfonyl chloride

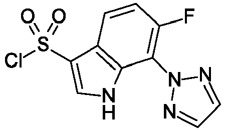

The title compound was prepared in analogy to Intermediate A2 from 1,2-difluoro-3-nitrobenzene instead of 1-chloro-2-fluoro-3-nitro-benzene in Step 1) as a pink solid. MS (ESI) m/z= 301.0 [M+H]⁺

### Intermediate A10: 6-chloro-7-(1,2,4-triazol-1-yl)-1H-indole-3-sulfonyl chloride

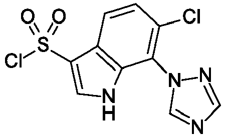

### Step 1: 4-(2-chloro-6-nitro-phenyl)-1,2,4-triazole and 4-(2-chloro-6-nitro-phenyl)-1,2,4-triazole

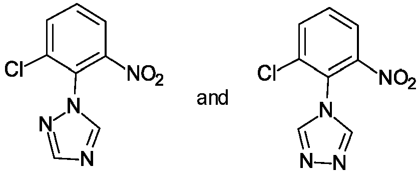

A mixture of 1-chloro-2-fluoro-3-nitro-benzene (1.49 g, 1 ml, 8.49 mmol), 1,2,4-triazole (704 mg, 10.2 mmol) and potassium carbonate (3.52 g, 25.5 mmol) in 1,4-dioxane (20 ml) was stirred at 80°C for 1 h, then at 60°C overnight. After cooling to room temperature, the solid was filtered off and washed with ethyl acetate. The filtrate was concentrated in vacuo. The residue was purified by flash chromatography (silica gel, 0 % to 100 % ethyl acetate in heptane) to afford 1-(2-chloro-6-nitro-phenyl)-1,2,4-triazole (1.60 g, 84% yield) as off-white solid and 4-(2-chloro-6-nitro-phenyl)-1,2,4-triazole (280 mg, 15% yield) as light yellow solid. MS (ESI) m/z= 225.0 [M+H]⁺ and MS (ESI) m/z= 225.0 [M+H]⁺

### Step 2: 6-chloro-7-(1,2,4-triazol-1-yl)-1H-indole

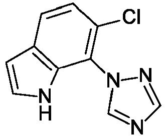

In a 50 ml three-necked flask, 1-(2-chloro-6-nitro-phenyl)-1,2,4-triazole (400 mg, 1.78 mmol) was dissolved in tetrahydrofuran (20 ml) and the solution was cooled to -60°C. Vinylmagnesium bromide solution in THF (1M, 7.1 ml, 7.12 mmol) was added. The reaction mixture was further stirred at -60°C for 1h. The reaction mixture was quenched with saturated ammonium chloride solution, poured into water and extracted with ethyl acetate twice. The combined organic layers were washed with brine, dried over sodium sulfate, filtered and concentrated in vacuo. The residue was purified by flash chromatography (silica gel, 0 % to 100 % ethyl acetate in heptane) to afford 6-chloro-7-(1,2,4-triazol-1-yl)-1H-indole (195 mg, 50% yield) as light yellow solid. MS (ESI) m/z= 219.1 [M+H]⁺

### Step 3: 6-chloro-7-(1,2,4-triazol-1-yl)-1H-indole-3-sulfonyl chloride

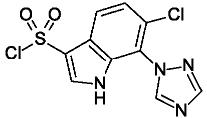

A suspension of 6-chloro-7-(1,2,4-triazol-1-yl)-1H-indole (200 mg, 0.915 mmol) in acetonitrile (15 ml) was cooled to 0°C. Chlorosulfonic acid (320 mg, 184 ul, 2.74 mmol) was added. The ice-bath was removed and the reaction mixture was stirred for 2 h at room temperature. Phosphorus oxychloride (561 mg, 341 uL, 3.66 mmol) was added and the reaction mixture was stirred at 70°C overnight. The mixture was poured into ice and water and extracted with ethyl acetate twice. The combined organic layers were washed with ice and brine, dried over sodium sulfate and concentrated in vacuo. The crude material was suspended in dichloromethane, filtered and washed with small amount of dichloromethane to afford 6-chloro-7-(1,2,4-triazol-1-yl)-1H-indole-3-sulfonyl chloride (243 mg, 84% yield) as off-white solid. MS (ESI) m/z= 317.0 [M+H]⁺

### Intermediate A11: 6-chloro-7-(1,2,4-triazol-4-yl)-1H-indole-3-sulfonyl chloride

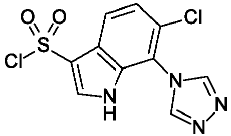

The title compound was prepared in analogy to Intermediate A10 from 4-(2-chloro-6-nitrophenyl)-1,2,4-triazole instead of 1-(2-chloro-6-nitro-phenyl)-1,2,4-triazole in Step 2) as a white solid. MS (ESI) m/z= 317.0 [M+H]⁺

### Intermediate A12: 6-chloro-7-(4-methyltriazol-2-yl)-1H-indole-3-sulfonyl chloride

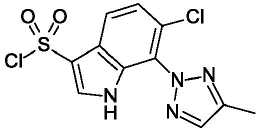

### Step 1: 2-(2-chloro-6-nitro-phenyl)-4-methyl-triazole

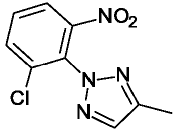

A solution of 1-chloro-2-fluoro-3-nitro-benzene (447 mg, 0.300 ml, 2.55 mmol) and 4-methyl-1H-triazole (254 mg, 3.06 mmol) in acetonitrile (6 ml) was cooled to 0°C. Cesium carbonate (1.66 g, 5.09 mmol) was added, the ice-bath was removed and the reaction mixture was stirred at room temperature for 1 h. The mixture was diluted with ethyl acetate, the solid was filtered off and washed with ethyl acetate. The filtrate was concentrated in vacuo and the residue was purified by flash chromatography (silica gel, 0 % to 50 % ethyl acetate in heptane) to afford 2-(2-chloro-6-nitro-phenyl)-4-methyl-triazole (258 mg, 42%) as off-white solid MS (ESI) m/z= 239.0 [M+H]⁺

### Step 2: 6-chloro-7-(4-methyltriazol-2-yl)-1H-indole-3-sulfonyl chloride

The title compound was prepared in analogy to Intermediate A10 from 2-(2-chloro-6-nitrophenyl)-4-methyl-triazole instead of 1-(2-chloro-6-nitro-phenyl)-1,2,4-triazole in Step 2) as an off-white solid. MS (ESI) m/z= 329.0 [M-H]⁻

### Intermediate A13: 6-chloro-7-pyrazol-1-yl-1H-indole-3-sulfonyl chloride

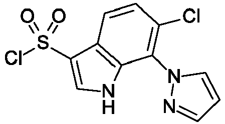

### Step 1: 1-(2-chloro-6-nitro-phenyl)pyrazole

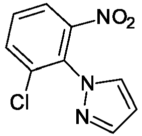

To a solution of 1-chloro-2-fluoro-3-nitro-benzene (3 g, 2.01 ml, 17 mmol) in acetonitrile (40 ml) were added at 0°C 1H-pyrazole (1.42 g, 20.5 mmol) and potassium carbonate (7.16 g, 51.3 mmol). After the addition, the ice bath was removed and the reaction mixture was stirred at room temperature overnight. The reaction mixture was poured into water and extracted twice with ethyl acetate. The organic layers were dried over Na2SO4, filtered and concentrated in vacuo. The residue was purified by flash chromatography (silica gel, 0% to 50% ethyl acetate in heptane) to provide 1-(2-chloro-6-nitro-phenyl)pyrazole (4.0 g, 100% yield) as off-white solid, MS (ESI) m/z: 224.0 [M+H]⁺.

### Step 2: 6-chloro-7-pyrazol-1-yl-1H-indole

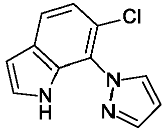

In a 1-liter four-necked flask, 1-(2-chloro-6-nitro-phenyl)pyrazole (4.0 g, 18 mmol) was dissolved in tetrahydrofuran (180 ml) and the solution was cooled to -60°C. Vinylmagnesium bromide solution (1M in THF, 73 ml, 73 mmol) was added and the reaction mixture was further stirred at -60°C for 1 h. The reaction mixture was quenched with saturated ammonium chloride solution, poured into water and extracted with ethyl acetate twice. The combined organic layers were washed with brine, dried over Na2SO4, filtered and concentrated in vacuo. The residue was purified by flash chromatography (silica gel, 0 % to 50 % ethyl acetate in heptane) to afford 6-chloro-7-pyrazol-1-yl-1H-indole (2.13 g, 54% yield) as white solid, MS (ESI) m/z: 218.1 [M+H]⁺.

### Step 3: 6-chloro-7-pyrazol-1-yl-1H-indole-3-sulfonyl chloride

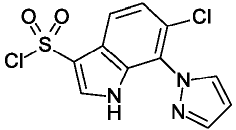

A solution of 6-chloro-7-pyrazol-1-yl-1H-indole (2.54 g, 11.5 mmol) in acetonitrile (60 ml) was cooled to 0°C. Chlorosulfonic acid (1.75 g, 1.01 ml, 15.02 mmol) was added. The ice-bath was removed and the reaction mixture was stirred 2 h at room temperature. Phosphorus oxychloride (7.09 g, 4.31 ml, 46.2 mmol) was added and the reaction mixture was stirred at 60°C overnight. The reaction mixture was poured into ice and water and extracted with ethyl acetate. The organic layers was washed with ice and brine, dried over Na2SO4 and concentrated in vacuo to afford the title compound (1.35 g, 28% yield) as brown solid, MS (ESI) m/z: 315.9 [M+H]⁺.

### Intermediate A14: 6-bromo-7-pyrazol-1-yl-1H-indole-3-sulfonyl chloride

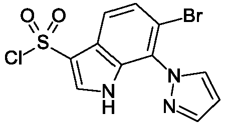

The title compound was prepared in analogy to Intermediate A13 from 1-bromo-2-fluoro-3-nitro-benzene instead of 1-chloro-2-fluoro-3-nitro-benzene in Step 1) as light brown solid. MS (ESI) m/z: 361.9 [M+H]⁺.

### Intermediate A15: 6-methyl-7-pyrazol-1-yl-1H-indole-3-sulfonyl chloride

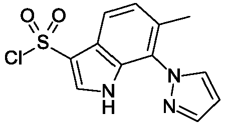

The title compound was prepared in analogy to Intermediate A13 from 2-fluoro-1-methyl-3-nitro-benzene instead of 1-chloro-2-fluoro-3-nitro-benzene in Step 1) as light yellow solid. MS (ESI) m/z: 296.1 [M+H]⁺.

### Intermediate A16: 6-chloro-5-fluoro-7-(triazol-2-yl)-1H-indole-3-sulfonyl chloride

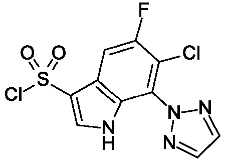

The title compound was prepared in analogy to Intermediate A13 from 2-chloro-1,3-difluoro-4-nitro-benzene instead of 1-chloro-2-fluoro-3-nitro-benzene and 1,2,3-triazole instead of 1H-pyrazole in Step 1) as light brown foam.

### Intermediate A17: 6-chloro-7-(5-methylpyrazol-1-yl)-1H-indole-3-sulfonyl chloride

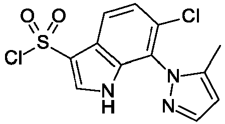

The title compound was prepared in analogy to Intermediate A13 from 3-methyl-1H-pyrazole instead of 1H-pyrazole in Step 1) and continuing the reaction sequence with 1-(2-chloro-6-nitrophenyl)-5-methyl-pyrazole, as off-white solid, MS (ESI) m/z: 328.1 [M-H]⁻.

### Intermediate A18: 6-chloro-7-(3-methylpyrazol-1-yl)-1H-indole-3-sulfonyl chloride

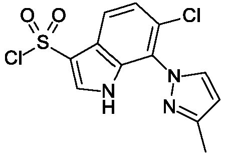

The title compound was prepared in analogy to Intermediate A13 from 3-methyl-1H-pyrazole instead of 1H-pyrazole in Step 1) and continuing the reaction sequence with 1-(2-chloro-6-nitrophenyl)-3-methyl-pyrazole, as white solid, MS (ESI) m/z: 328.1 [M-H]⁻.

### Intermediate A19: 6-chloro-7-(4-fluoropyrazol-1-yl)-1H-indole-3-sulfonyl chloride

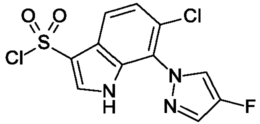

The title compound was prepared in analogy to Intermediate A13 from 4-fluoro-1H-pyrazole instead of 1H-pyrazole in Step 1) as off-white solid, MS (ESI) m/z: 334.0 [M+H]⁺.

### Intermediate A20: 6-chloro-7-(4-methylpyrazol-1-yl)-1H-indole-3-sulfonyl chloride

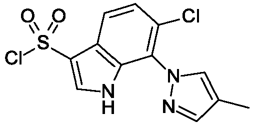

The title compound was prepared in analogy to Intermediate A13 from 4-methyl-1H-pyrazole instead of 1H-pyrazole in Step 1) as off-white solid, MS (ESI) m/z: 328.0 [M-H]⁻.

### Intermediate A21: 6-chloro-7-(3-fluoropyrazol-1-yl)-1H-indole-3-sulfonyl chloride

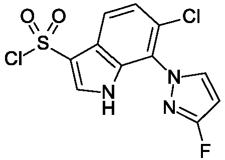

The title compound was prepared in analogy to Intermediate A13 from 3-fluoro-1H-pyrazole instead of 1H-pyrazole in Step 1) as light brown foam, MS (ESI) m/z: 334.0 [M+H]⁺.

### Intermediate A22: 6-(difluoromethyl)-7-pyrazol-1-yl-1H-indole-3-sulfonyl chloride

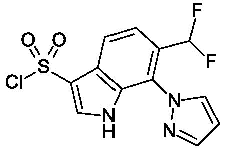

The title compound was prepared in analogy to Intermediate A13 from 1-(difluoromethyl)-2-fluoro-3-nitro-benzene (see Intermediate A5) instead of 1-chloro-2-fluoro-3-nitro-benzene in Step 1) as light brown foam, MS (ESI) m/z: 332.0 [M+H]⁺.

### Intermediates B

### Intermediate B1: 5-(2,2-difluoroethyl)-4,6-dimethoxy-pyrimidin-2-amine

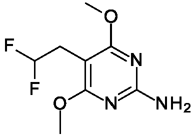

### Step 1: diethyl 2-(2,2-difluoroethyl)propanedioate

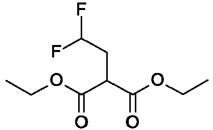

Diethyl malonate (75.8 ml, 500 mmol) was combined with tetrahydrofuran (450 ml). Sodium ethoxide (prepared from ethanol (150 mL) and sodium (11.48 g, 500 mmol)) was added at room temperature and the reaction mixture was stirred 15 min at room temperature. A solution of 2,2-difluoroethyl trifluoromethanesulfonate (76 ml, 500 mmol) in tetrahydrofuran (10 ml) was added slowly. The reaction mixture was stirred for 18 hours at 20 °C, then cooled to 0 °C, quenched with a saturated ammonium chloride solution and extracted twice with ethyl acetate. The organic layers were washed with brine, dried over sodium sulfate, filtered and concentrated in vacuo to provide the title compound (100.5 g, 90 % yield). MS (ESI) m/z= 225.0 [M+H]⁺

### Step 2: 2-amino-5-(2,2-difluoroethyl)pyrimidine-4,6-diol

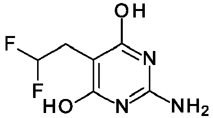

To a stirred solution of diethyl 2-(2,2-difluoroethyl)propanedioate (46.8 g, 209 mmol) in ethanol (5 mL) was added guanidine hydrochloride (19.9 g, 208 mmol), followed by sodium ethoxide (prepared from ethanol and sodium (14.38 g, 625 mmol)). The resulting orange suspension was heated to 80 °C and stirred for 4 hours. The reaction mixture was concentrated by half, 50 ml of water was added, followed by acetic acid (42.57 g, 709 mmol). The mixture was heated to 80 °C and stirred for 10 min, then cooled to room temperature. The solid product was filtered off, washed successively with water, ethanol and methyl tert-butyl ether to provide the title compound (22.3 g, 50 % yield). MS (ESI) m/z= 192.0 [M+H]⁺

### Step 3: 4,6-dichloro-5-(2,2-difluoroethyl)pyrimidin-2-amine

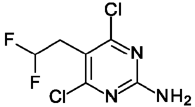

2-amino-5-(2,2-difluoroethyl)pyrimidine-4,6-diol (13.2 g, 69.1 mmol) was suspended in phosphorus oxychloride (80.5 ml, 863 mmol). The reaction mixture was heated to 100 °C and stirred for 18 hours and concentrated in vacuo. The residue was diluted with ethyl acetate and carefully poured into ice / saturated sodium bicarbonate solution. The resulting biphasic mixture was stirred at room temperature for 5 min and extracted twice with ethyl acetate. The organic layers were dried over sodium sulfate, filtered and concentrated in vacuo. The residue was purified by flash chromatography over silica gel to provide the title compound (7.35 g, 47 % yield). MS (ESI) m/z= 227.8 [M+H]⁺

### Step 4: 5-(2,2-difluoroethyl)-4,6-dimethoxy-pyrimidin-2-amine

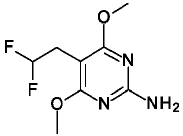

In a sealed tube, a mixture of 4,6-dichloro-5-(2,2-difluoroethyl)pyrimidin-2-amine (7.6 g, 33.33 mmol) and sodium methylate (prepared from sodium (7.66 g, 333.29 mmol) in methanol (50 ml)) was heated to 75 °C and stirred for 18 hours. The reaction mixture was quenched with water and extracted twice with ethyl acetate. The organic layers were dried over sodium sulfate, filtered and concentrated in vacuo to provide the title compound as a light yellow solid (6.6 g, 86 % yield). MS (ESI) m/z= 220.0 [M+H]⁺

### Intermediate B2: 5-(2-fluoroethoxy)-4-methoxy-pyrimidin-2-amine

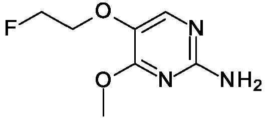

### Step 1: [4-methoxy-5-(4,4,5,5-tetramethyl-1,3,2-dioxaborolan-2-yl)pyrimidin-2-yl]-bis(p-anisyl)amine

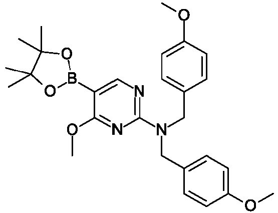

A suspension of (5-bromo-4-methoxy-pyrimidin-2-yl)-bis(p-anisyl)amine (500 mg, 1.13 mmol, see Intermediate B3 step 1), bis(pinacolato)diboron (354 mg, 1.35 mmol) and potassium acetate (335 mg, 3.38 mmol) in 1,4-dioxane (10 mL) was purged with argon for 5 min. dichloro[1,1'-bis(diphenylphosphino)ferrocene]palladium(ii) dichloromethane adduct (91.9 mg, 0.113 mmol) was added. The reaction mixture was heated to 90 °C and stirred for 16 hours. The resulting dark suspension was poured into ethylacetate and washed once with sat. NaCl. The organic layer was dried over sodium sulfate, filtered and concentrated in vacuo. The residue was purified by column chromatography on silica gel using a gradient ethylacetate/heptane 0-30% to provide the title compound as a colorless viscous oil (157 mg, 29 % yield). MS (ESI): m/z= 492.4 [M+H]⁺

### Step 2: 2-[bis(p-anisyl)amino]-4-methoxy-pyrimidin-5-ol

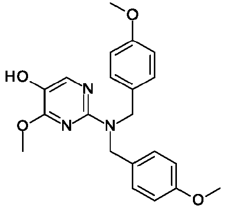

A solution of [4-methoxy-5-(4,4,5,5-tetramethyl-1,3,2-dioxaborolan-2-yl)pyrimidin-2-yl]-bis(p-anisyl)amine (130 mg, 0.265 mmol) in tetrahydrofuran (2.5 mL) was cooled to 0°C. Hydrogen peroxide 35% (500 uL, 5.71 mmol) was added. The reaction mixture was stirred at 0 °C for 15 min, allowed to warm to rt and stirred for 3 hours. The reaction mixture was poured into cold 0.1 N sodiumsulfite solution and extracted twice with EtOAc. The organic layers were washed twice with brine, dried over sodiumsulfate, filtered and concentrated in vacuo to provide the title compound as a light yellow viscous oil (103 mg, 100 % yield). MS (ESI): m/z= 382.3 [M+H]⁺

### Step 3: [5-(2-fluoroethoxy)-4-methoxy-pyrimidin-2-yl]-bis(p-anisyl)amine

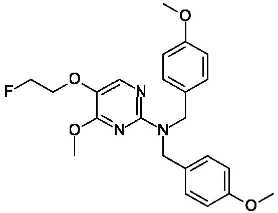

A suspension of 2-[bis(p-anisyl)amino]-4-methoxy-pyrimidin-5-ol (100 mg, 0.236 mmol), potassium carbonate (98.82 mg, 0.708 mmol) and 1-bromo-2-fluoroethane (61.14 mg, 35.75 uL, 0.472 mmol) in acetonitrile (2.5 mL) was stirred at room temperature for 15 min and at 80°C for 6 hours. The reaction mixture was poured into water and extracted twice with EtOAc. The organic layers were dried over sodium sulfate, filtered and concentrated in vacuo. The residue was purified by flash chromatography on silica gel using a gradient ethylacetate/heptane 0-30% to provide the title compound as a colorless viscous oil (22 mg, 22 % yield). MS (ESI): m/z= 428.3 [M+H]⁺

### Step 4: [5-(2-fluoroethoxy)-4-methoxy-pyrimidin-2-yl]amine

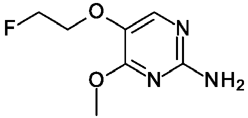

A solution of [5-(2-fluoroethoxy)-4-methoxy-pyrimidin-2-yl]-bis(p-anisyl)amine (87 mg, 0.204 mmol) in dichloromethane (500 uL) was cooled to 0 °C. Trifluoroacetic acid (1.41 g, 944.56 uL, 12.21 mmol) was added. The reaction mixture was allowed to warm to room temperature and stirred for 16 hours and at 55°C for two additional hours. The resulting purple solution was poured into a sat. aqueous sodium bicarbonate solution and extracted twice with EtOAc. The organic layers were dried over sodium sulfate, filtered and concentrated in vacuo. The residue was purified by flash chromatography on silica gel using a gradient ethylacetate/heptane 0-100% to provide the title compound as an off-white solid (27 mg, 71 % yield). MS (ESI): m/z= 188.1 [M+H]⁺

### Intermediate B3: 5-(2,2-difluoroethyl)-4-methoxy-pyrimidin-2-amine

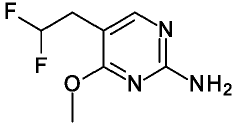

### Step 1: (5-bromo-4-methoxy-pyrimidin-2-yl)-bis(p-anisyl)amine

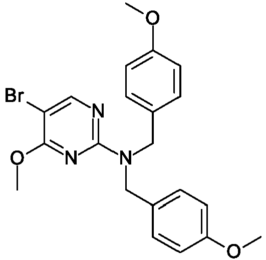

A suspension of 5-bromo-2-chloro-4-methoxy-pyrimidine (1.02 g, 4.48 mmol, CAS: 57054-92-9), bis(p-anisyl)amine (1.29 g, 4.92 mmol) and n-ethyldiisopropylamine (858 uL, 4.92 mmol) in acetonitrile (20 mL) was heated at 70 °C for 2 days. The resulting solution was poured into a saturated aqueous sodium bicarbonate solution and extracted twice with ethylacetate. The organic layers were dried over sodium sulfate, filtered and dried in vacuo. The residue was purified by flash chromatography over silica gel using a gradient ethylacetate/heptane 0-20% to provide the title compound as a colorless viscous oil (998 mg, 50 % yield). MS (ESI): m/z= 446.2 [M+H]⁺

### Step 2: 5-[(E)-2-ethoxyvinyl]-4-methoxy-N,N-bis[(4-methoxyphenyl)methyl]pyrimidin-2-amine

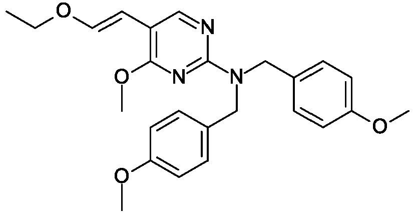

In a 250 mL round-bottomed flask were added one after the other, (5-bromo-4-methoxy-pyrimidin-2-yl)-bis(p-anisyl)amine (11.1 g, 23.73 mmol), 2-[(E)-2-ethoxyvinyl]-4,4,5,5-tetramethyl-1,3,2-dioxaborolane (6.11 g, 30.85 mmol), 1,4-dioxane (97 mL) / water (16 mL), dichloro[1,1'-bis(diphenylphosphino)ferrocene]palladium(II) dichloromethane adduct (1.94 g, 2.37 mmol) and cesium carbonate (23.2 g, 71.2 mmol) (vacuum/argon after each addition). The reaction mixture was stirred at 110 °C for 15 hours, then poured into water and extracted three times with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as a light yellow oil (4.2 g, 39 % yield). MS (ESI) m/z= 436.3 [M+H]⁺

### Step 3: 2-[2-[bis[(4-methoxyphenyl)methyl]amino]-4-methoxy-pyrimidin-5-yl]acetaldehyde

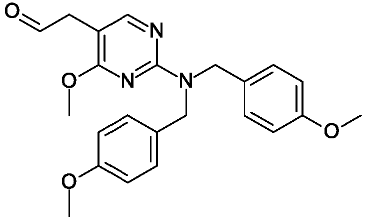

5-[(E)-2-ethoxyvinyl]-4-methoxy-N,N-bis[(4-methoxyphenyl)methyl]pyrimidin-2-amine (1 g, 2.3 mmol) in formic acid (3.17 g, 2.64 mL, 68.88 mmol) was stirred at 60 °C for 30 min, then poured into sat. NaHCO3 under ice cooling and extracted twice with ethyl acetate. The organic layers were washed with water and brine, dried over sodium sulfate, filtered and concentrated in vacuo to provide the title compound as a light yellow oil (1.01g, 97 % yield). MS (ESI) m/z= 408.3 [M+H]⁺

### Step 4: 5-(2,2-difluoroethyl)-4-methoxy-N,N-bis[(4-methoxyphenyl)methyl]pyrimidin-2-amine

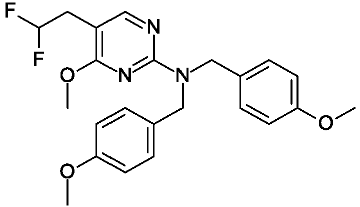

A solution of 2-[2-[bis[(4-methoxyphenyl)methyl]amino]-4-methoxy-pyrimidin-5-yl]acetaldehyde (1.01 g, 2.23 mmol) in dichloromethane, extra dry (10 mL) was cooled to 0 °C. Deoxo-fluor ^{®}, 2.7M (50 wt.%) solution in toluene (1.98 g, 1.65 mL, 4.46 mmol) was added dropwise. The reaction mixture was stirred at 0 °C for 30 min, then poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a colorless oil (763 mg, 72 % yield). MS (ESI) m/z= 430.3 [M+H]⁺

### Step 5: 5-(2,2-difluoroethyl)-4-methoxy-pyrimidin-2-amine

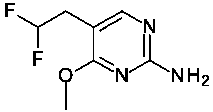

To a stirred solution of 5-(2,2-difluoroethyl)-4-methoxy-N,N-bis[(4-methoxyphenyl)methyl]pyrimidin-2-amine (763 mg, 1.6 mmol) in dichloromethane (8 mL) was added trifluoroacetic acid (10.94 g, 7.39 mL, 95.94 mmol). The reaction mixture was stirred at 50 °C for 2 days, then concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a light yellow solid (220 mg, 73 % yield). MS (ESI) m/z= 190.1 [M+H]⁺

### Intermediate B4: 5-(2,2-difluoroethoxy)-4,6-dimethoxy-pyrimidin-2-amine

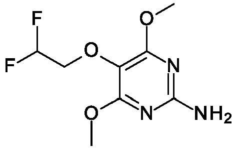

### Step 1: 5-bromo-4,6-dimethoxy-pyrimidin-2-amine

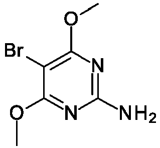

To a stirred solution of (4,6-dimethoxypyrimidin-2-yl)amine (7 g, 44.22 mmol, CAS: 36315-01-2) in acetonitrile (100 mL) was added a solution of N-bromosuccinimide (10.33 g, 57.48 mmol) in acetonitrile (100 mL) dropwise at room temperature. The reaction mixture was stirred at room temperature for 30 min. The resulting white suspension was diluted with ethyl acetate and washed with water. The organic layer was dried over sodium sulfate, filtered, diluted with heptane and concentrated in vacuo. The precipitate was filtered off and washed with heptane to provide the title compound as a white solid (9.26 g, 87 % yield). MS (ESI) m/z= 234.1 [M+H]⁺

### Step 2: 5-bromo-4,6-dimethoxy-N,N-bis[(4-methoxyphenyl)methyl]pyrimidin-2-amine

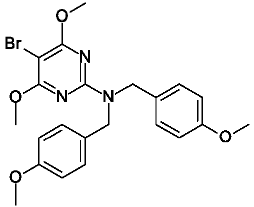

A solution of 5-bromo-4,6-dimethoxy-pyrimidin-2-amine (517 mg, 2.21 mmol) in N,N-dimethylacetamide (9 mL) was cooled to 0 °C. Sodium hydride (265.05 mg, 6.63 mmol) was added portionwise (3 x 88 mg). The stirring was continued at 0 °C for 30 min. 4-methoxybenzyl chloride (706. mg, 608.62 uL, 4.42 mmol) was added dropwise. The reaction mixture was allowed to warm to room temperature, stirred for 1 hour, carefully quenched with a saturated ammonium chloride solution, poured into water and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as a white solid (1.11 g, 100 % yield). MS (ESI) m/z= 476.2 [M+H]⁺

### Step 3: 2-[bis[(4-methoxyphenyl)methyl]amino]-4,6-dimethoxy-pyrimidin-5-ol

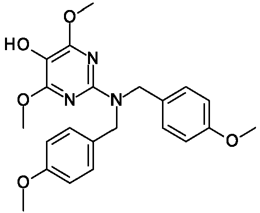

To a colorless solution of 5-bromo-4,6-dimethoxy-N,N-bis[(4-methoxyphenyl)methyl]-pyrimidin-2-amine (500 mg, 1 mmol) in tetrahydrofuran (3.5 mL) was added a 1.6 M n-butyllithium solution in hexanes (699.71 mg, 813.61 uL, 1.3 mmol) dropwise at -78 °C. The resulting yellow solution was stirred at -78 °C for 30 min. Trimethyl borate (156.08 mg, 167.47 uL, 1.5 mmol) was added dropwise and the stirring was continued at -78 °C for 1.5 hours. The reaction mixture was allowed to warm to 0 °C and acetic acid (120.27 mg, 114.62 uL, 2 mmol) was added dropwise, followed by hydrogen peroxide 35% (145.98 mg, 131.51 uL, 1.5 mmol). The stirring was continued at 0 °C for 1.5 hours. The resulting pink suspension was poured into a 0.1 N sodium thiosulfate solution and extracted twice with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as a light yellow viscous oil (194 mg, 47 % yield). MS (ESI) m/z= 412.3 [M+H]⁺

### Step 4: 5-(2,2-difluoroethoxy)-4,6-dimethoxy-N,N-bis[(4-methoxyphenyl)methyl]pyrimidin-2-amine

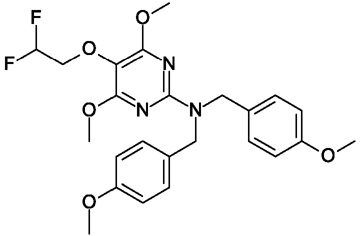

To a solution of 2-[bis[(4-methoxyphenyl)methyl]amino]-4,6-dimethoxy-pyrimidin-5-ol (100 mg, 0.214 mmol) in N,N-dimethylformamide (1.75 mL) was added potassium carbonate (88.68 g, 0.642 mmol) and 1,1-difluoro-2-iodoethane (123.16 mg, 56.5 uL, 0.642 mmol). The reaction mixture was stirred at 80 °C for 1.5 hours, cooled to room temperature, poured into water and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as an off-white solid (92 mg, 85 % yield). MS (ESI) m/z= 476.2 [M+H]⁺

### Step 5: 5-(2,2-difluoroethoxy)-4,6-dimethoxy-pyrimidin-2-amine

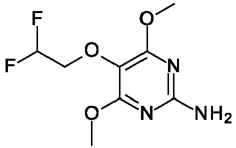

To a stirred solution of 5-(2,2-difluoroethoxy)-4,6-dimethoxy-N,N-bis[(4-methoxyphenyl)methyl]pyrimidin-2-amine (92 mg, 0.193 mmol) in dichloromethane (0.340 mL) was added trifluoroacetic acid (1.34 g, 897.97 uL, 11.61 mmol) at 0 °C. The reaction mixture was stirred at room temperature for 18 hours and at 50 °C for 4 hours. The resulting red solution was concentrated in vacuo, poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a light yellow solid (41 mg, 87 % yield). MS (ESI) m/z= 236.2 [M+H]⁺

### Intermediate B5: 5-(2-fluoroethoxy)-4,6-dimethoxy-pyrimidin-2-amine

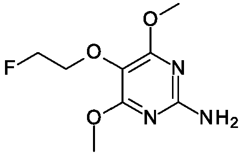

### Step 1: 5-(2-fluoroethoxy)-4,6-dimethoxy-N,N-bis[(4-methoxyphenyl)methyl]pyrimidin-2-amine

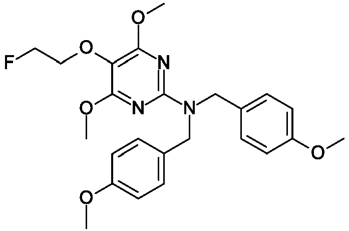

A suspension of 2-[bis[(4-methoxyphenyl)methyl]amino]-4,6-dimethoxy-pyrimidin-5-ol (2 g, 4.52 mmol, intermediate B4, step 3), potassium carbonate (1.89 g, 13.56 mmol) and 1-bromo-2-fluoroethane (1.76 g, 1.03 mL, 13.56 mmol) in N,N-dimethylformamide (45 mL) was heated to 80 °C and stirred for 2.5 hours. The reaction mixture was poured into brine and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a white solid (1.79 g, 85 % yield). MS (ESI) m/z= 458.3 [M+H]⁺

### Step 2: 5-(2-fluoroethoxy)-4,6-dimethoxy-pyrimidin-2-amine

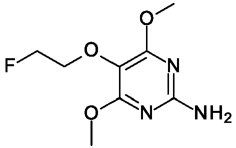

To a stirred solution of 5-(2-fluoroethoxy)-4,6-dimethoxy-N,N-bis[(4-methoxyphenyl)methyl]pyrimidin-2-amine (1.79 g, 3.83 mmol) in dichloromethane (4 mL) was added trifluoroacetic acid (26.23 g, 17.62 mL, 230.06 mmol). The reaction mixture was stirred at 50 °C for 3 hours, at room temperature for 15 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-60% to provide the title compound as an off-white solid (885 mg, 100 % yield). MS (ESI) m/z= 218.1 [M+H]⁺

### Intermediate B6: 5-(difluoromethoxy)-4,6-dimethoxy-pyrimidin-2-amine

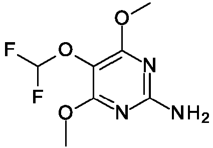

### Step 1: 5-(difluoromethoxy)-4,6-dimethoxy-N,N-bis[(4-methoxyphenyl)methyl]pyrimidin-2-amine

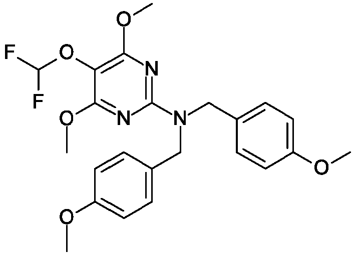

To a solution of 2-[bis[(4-methoxyphenyl)methyl]amino]-4,6-dimethoxy-pyrimidin-5-ol (130 mg, 0.316 mmol, intermediate B4, step 3) in acetonitrile (5 mL) was added a 5 M potassium hydroxide solution (1.26 mL, 6.32 mmol) dropwise at 0 °C, followed by addition of bromodifluoromethyl diethylphosphonate (168.72 mg, 112.26 uL, 0.632 mmol) in acetonitrile (1 mL) dropwise at 0 °C. The reaction mixture was stirred at 0 °C for 10 min. The resulting light yellow biphasic mixture was poured into water and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a white solid (57 mg, 39 % yield). MS (ESI) m/z= 462.3 [M+H]+

### Step 2: 5-(difluoromethoxy)-4,6-dimethoxy-pyrimidin-2-amine

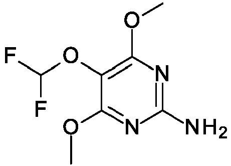

To a stirred solution of 5-(difluoromethoxy)-4,6-dimethoxy-N,N-bis[(4-methoxyphenyl)methyl]pyrimidin-2-amine (56 mg, 0.121 mmol) in dichloromethane (100 uL) was added trifluoroacetic acid (838.62 mg, 563.21 uL, 7.28 mmol). The reaction mixture was stirred at room temperature for 40 hours, at 50 °C for 6 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a white solid (24 mg, 89 % yield). MS (ESI) m/z= 222.1 [M+H]⁺

### Intermediate B7: 5-(2,3-difluoropropyl)-4,6-dimethoxy-pyrimidin-2-amine

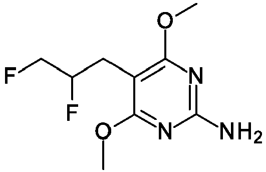

### Step 1: 5-allyl-4,6-dimethoxy-pyrimidin-2-amine

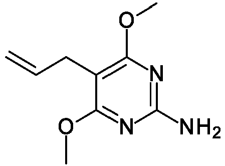

To a solution of 5-bromo-4,6-dimethoxy-pyrimidin-2-amine (5000 mg, 21.4 mmol, intermediate B4, step 1), allylboronic acid pinacol ester (4307.8 mg, 25.64 mmol) and potassium carbonate (8857.47 mg, 64.09 mmol) in 1,4-dioxane (125 mL) and water (25 mL) was added 1,1'-bis(diphenylphosphino)ferrocene-palladium(II)dichloride dichloromethane complex (1743.22 mg, 2.14 mmol) under nirogen. The mixture was stirred for 12 hours at 110 °C and extracted three times with ethyl acetate. The organic layers were washed with brine, dried over sodium sulfate, filtered and concentrated in vacuo. The residue was purified by preparative HPLC and freeze-dried to provide the title compound as an off-white solid (835 mg, 14 % yield). MS (ESI) m/z= 195.8 [M+H]⁺

### Step 2: 5-allyl-4,6-dimethoxy-N,N-bis[(4-methoxyphenyl)methyl]pyrimidin-2-amine

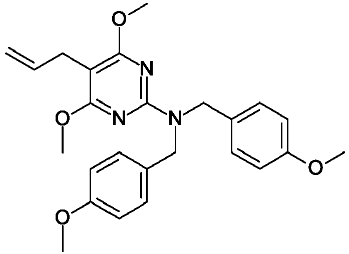

To a solution of 5-allyl-4,6-dimethoxy-pyrimidin-2-amine (1.8 g, 9.22 mmol) in N,N-dimethylformamide (20 mL) was added sodium hydride (1.48 g, 36.88 mmol) at 0 °C under nitrogen and the mixture was stirred at room temperature for 10 min. Then 4-methoxybenzylchloride (2.5 mL, 18.44 mmol) was added. The reaction mixture was stirred at room temperature overnight. The reaction mixture was poured into water and extracted three times with ethyl acetate. The organic layers were washed successively with brine and a saturated solution of ammonium chloride, dried over sodium sulfate, filtered and concentrated in vacuo. The residue was purified by flash chromatography over silica gel using a gradient ethylacetate/petroleum ether 30-50% to provide the title compound as a white solid (3.3 g, 72 % yield). MS (ESI) m/z= 436.3 [M+H]⁺

### Step 3: 3-[2-[bis[(4-methoxyphenylmethyl]amino]-4,6-dimethoxy-pyrimidin-5-yl]propane-1,2-diol

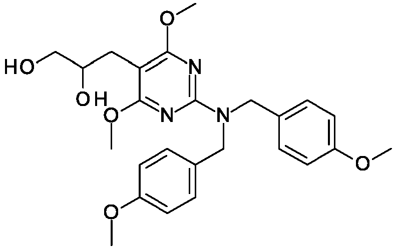

To a stirring solution of 5-allyl-4,6-dimethoxy-N,N-bis[(4-methoxyphenyl)methyl]pyrimidin-2-amine (3.3 g, 7.58 mmol) in THF (25 mL) and water (5 mL) was added N-Methylmorpholine N-oxide (1.78 g, 15.15 mmol) and then osmium tetroxide (385.27 mg, 1.52 mmol). The reaction mixture was stirred at 25 °C for 16 hours. 50 mL of an aqueous solution of Na2S2O3 (10 g) was added to the mixture. After stirring for a few minutes, the reaction mixture was extracted three times with ethyl acetate. The organic layers were washed with brine, dried over sodium sulfate, filtered and concentrated in vacuo to provide the title compound as a white solid (1.5 g, 42 % yield). MS (ESI) m/z= 470.3 [M+H]⁺

### Step 4: 5-(2,3-difluoropropyl)-4,6-dimethoxy-N,N-bis[(4-methoxyphenyl)methyl]pyrimidin-2-amine

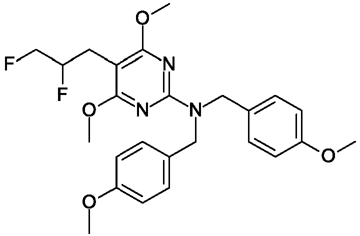

To a solution of diethylaminosulfur trifluoride (0.84 mL, 6.39 mmol) in dichloromethane (10 mL) was added 3-[2-[bis[(4-methoxyphenyl)methyl]amino]-4,6-dimethoxy-pyrimidin-5-yl]propane-1,2-diol (600.0 mg, 1.28 mmol) in dichloromethane (10 mL) at 0-10 °C under nitrogen. The reaction mixture was stirred at 0 °C for 1 hour, quenched with sat. NaHCO3 and extracted three times with dichloromethane. The organic layers were dried over sodium sulfate, filtered and concentrated in vacuo. The residue was purified by flash chromatography over silica gel using a gradient ethylacetate/petroleum ether 10-20% to provide the title compound as a white waxy solid (605 mg, 100 % yield). MS (ESI) m/z= 474.3 [M+H]⁺

### Step 5: 5-(2,3-difluoropropyl)-4,6-dimethoxy-pyrimidin-2-amine

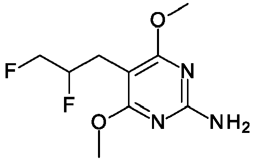

A solution of 5-(2,3-difluoropropyl)-4,6-dimethoxy-N,N-bis[(4-methoxyphenyl)methyl]pyrimidin-2-amine (900.0 mg, 1.9 mmol) in trifluoroactic acid (12.86 mL, 173.1 mmol) was stirred for 2 hours at 60 °C, poured into sat. NaHCO3 and extracted twice with ethyl acetate. The organic layers were washed with brine, dried over sodium sulfate, filtered and concentrated in vacuo. The residue was purified by flash chromatography over silica gel using a gradient ethylacetate/petroleum ether 30-50%, followed by preparative HPLC to provide the title compound as a white solid (85 mg, 19 % yield). MS (ESI) m/z= 234.2 [M+H]⁺

### Intermediate B8: 5-(3,3-difluoropropyl)-4,6-dimethoxy-pyrimidin-2-amine

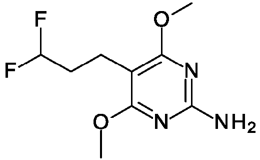

### Step 1: diethyl 2-(3,3-difluoropropyl)propanedioate

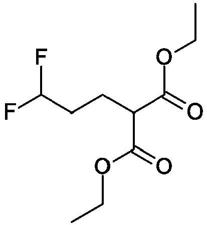

Sodium hydride (538.92 mg, 13.47 mmol) was supended in tetrahydrofuran, extra dry (30 mL) and the mixture was cooled to 0 °C. Diethyl malonate (1.92 g, 1.83 mL, 11.99 mmol) was added slowly. The mixture was stirred 10 min at 0 °C then a solution of 3,3-difluoropropyl 4-methylbenzenesulfonate (3 g, 11.99 mmol, intermediate B32, step 1) in tetrahydrofuran (5 mL) was added slowly. The reaction mixture was stirred 5 min at 0 °C, 20 hours at 60 °C, then cooled to 0 °C and quenched with a saturated ammonium chloride solution, poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as a colorless liquid (2.16 g, 50 % yield). MS (ESI) m/z= 239.2 [M+H]⁺

### Step 2: 2-amino-5-(3,3-difluoropropyl)pyrimidine-4,6-diol

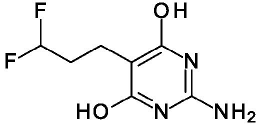

Diethyl 2-(3,3-difluoropropyl)propanedioate (1.98 g, 8.31 mmol) was combined with ethanol (16 mL) to give a colorless solution. Guanidine hydrochloride (793.98 mg, 8.31 mmol) was added followed by sodium ethoxide 21% in ethanol (8.08 g, 9.31 mL, 24.93 mmol) and the orange suspension was stirred at 75 °C for 4 hours. The reaction mixture was concentrated by half, 5 mL of water were added, followed by acetic acid (1.69 g, 1.61 mL, 28.1 mmol). The mixture was heated at 80 °C for 10 min then cooled to room temperature. The solid was filtered off and successively washed with water, ethanol and heptane to afford 460 mg of the product as a light brown solid. The filtrate was washed with ethyl acetate and concentrated by half. The precipitated was filtered off, successively washed with water, ethanol and heptane, combined with the previously obtained solid and dried on high vacuo to provide the title compound as a light brown solid (568 mg, 28 % yield). MS (ESI) m/z= 206.1 [M+H]⁺

### Step 3: 4,6-dichloro-5-(3,3-difluoropropyl)pyrimidin-2-amine

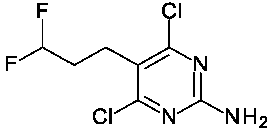

2-amino-5-(3,3-difluoropropyl)pyrimidine-4,6-diol (746 mg, 3.64 mmol) was suspended in phosphorus oxychloride (9.9 g, 6.02 mL, 64.54 mmol) and stirred at 100 °C for 4 hours. The excess of phosphorus oxychloride was removed under reduced pressure. The residue was diluted in dichloromethane, poured into an ice cooled NaHCO3 solution and stirred for 10 min at room temperature. After extraction, the organic layer was washed with brine, dried over sodium sulfate, filtered and concentrated in vacuo. The residue was purified by flash chromatography over silica gel using a gradient ethyl acetate/heptane 0-50% to provide the title compound as an off-white solid (432 mg, 44 % yield). MS (ESI) m/z= 242.1 [M+H]⁺

### Step 4: 5-(3,3-difluoropropyl)-4,6-dimethoxy-pyrimidin-2-amine

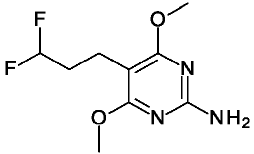

4,6-Dichloro-5-(3,3-difluoropropyl)pyrimidin-2-amine (432 mg, 1.78 mmol) was dissolved in tetrahydrofuran, extra dry (15 ml). Sodium methoxide 25% in methanol (1.93 g, 2.04 ml) was added. The reaction mixture was stirred at 60 °C for 5 hours, cooled to 0 °C, quenched with a saturated ammonium chloride solution and extracted twice with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as an off-white solid (328 mg, 76 % yield). MS (ESI) m/z= 234.2 [M+H]⁺

### Intermediate B9: 2-(2-amino-4,6-dimethoxy-pyrimidin-5-yl)cyclopropanecarbonitrile

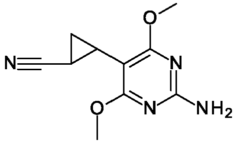

### Step 1: N,N-bis[(2,4-dimethoxyphenyl)methyl]-4,6-dimethoxy-pyrimidin-2-amine

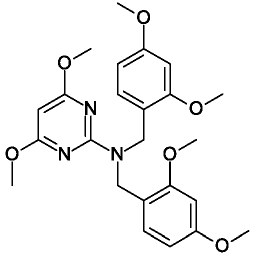

To a solution of 2-chloro-4,6-dimethoxypyrimidine (5.0 g, 28.6 mmol) and 1-(2,4-dimethoxyphenyl)-N-[(2,4-dimethoxyphenyl)methyl]methanamine (10.9 g, 34.37 mmol) in N-methylpyrrolidone (100 ml) was added cesium carbonate (18.7 g, 57.3 mmol) and the reaction mixture was stirred at 120 °C for 16 h. The mixture was diluted with water (500 ml) and extracted with ethyl acetate (100 ml × 2). The combined organic layers were washed with brine (100 ml × 2), dried over Na2SO4, filtered and concentrated under reduced pressure. The residue was purified by column chromatography (silica gel, petroleum ether/ethyl acetate = 1:0 to 5:1) to give N-bis[(2,4-dimethoxyphenyl)methyl]-4,6-dimethoxy-pyrimidin-2-amine (12.0 g, 89% yield) as a white solid. MS (ESI) m/z= 456.3 [M+H]⁺

### Step 2: N,N-bis[(2,4-dimethoxyphenyl)methyl]-5-iodo-4,6-dimethoxy-pyrimidin-2-amine

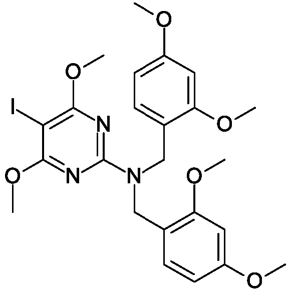

To a solution of N,N-bis[(2,4-dimethoxyphenyl)methyl]-4,6-dimethoxy-pyrimidin-2-amine (12.0 g, 26.3 mmol) in acetonitrile (150 ml) was added N-iodosuccinimide (7.11 g, 31.6 mmol) in portions at 20 °C and the mixture was stirred at 20 °C for 2 h. The reaction mixture was quenched by pouring into saturated sodium hydrogencarbonate solution (300 ml) and extracted with ethyl acetate (100 ml × 2). The combined organic layers were washed with brine (100 ml × 2), dried over sodium sulfate, filtered and concentrated under reduced pressure. The residue was purified by column chromatography (silica gel, petroleum ether / ethyl acetate = 1:0 to 3:1) to give N,N-bis[(2,4-dimethoxyphenyl)methyl]-5-iodo-4,6-dimethoxy-pyrimidin-2-amine (12.0 g, 78% yield) as a yellow solid. MS (ESI) m/z= 582.2 [M+H]⁺

### Step 3: (E)-3-[2-[bis[(2,4-dimethoxyphenyl)methyl]amino]-4,6-dimethoxy-pyrimidin-5-yl]prop-2-enenitrile

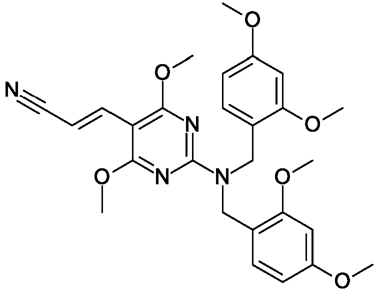

To a mixture of N,N-bis[(2,4-dimethoxyphenyl)methyl]-5-iodo-4,6-dimethoxy-pyrimidin-2-amine (9.0 g, 15.5 mmol) and acrylonitrile (6.0 ml, 90.5 mmol) in 1,4-dioxane (90 ml) was added cesium carbonate (10.8 g, 33.1 mmol) and bis(triphenylphosphine)palladium chloride (681 mg, 1.55 mmol) under nitrogen atmosphere and the reaction mixture was stirred in a sealed tube at 100 °C for 16 h. The mixture was concentrated under reduced pressure.

The residue was purified by column chromatography (silica gel, petroleum ether/ethyl acetate = 1:0 to 5:1) to give (E)-3-[2-[bis[(2,4-dimethoxyphenyl)methyl]amino]-4,6-dimethoxy-pyrimidin-5-yl]prop-2-enenitrile (7.5 g, 96% yield) as a brown solid. MS (ESI) m/z= 507.3 [M+H]⁺

### Step 4: 2-[2-[bis[(2,4-dimethoxyphenyl)methyl]amino]-4,6-dimethoxy-pyrimidin-5-yl]cyclopropanecarbonitrile

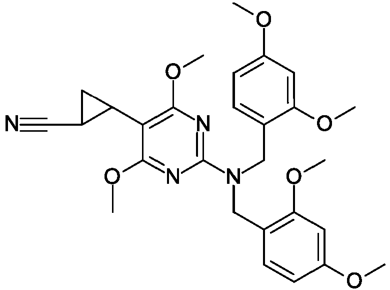

To a mixture of trimethylsulfoxonium iodide (6.52 g, 29.6 mmol) in dimethylsulfoxide (50 ml) was added sodium hydride (60% in mineral oil, 1.18 g, 29.6 mmol) in portions at 30 °C and the mixture was stirred at 30 °C for 0.5 h. The above mixture was added to a mixture of (E)-3-[2-[bis[(2,4-dimethoxyphenyl)methyl]amino]-4,6-dimethoxy-pyrimidin-5-yl]prop-2-enenitrile (5.0 g, 9.87 mmol) in dimethylsulfoxide (50 ml) at 30 °C and the mixture was stirred at 30 °C for 16 hr. The reaction mixture was diluted with water (300 ml) and extracted with ethyl acetate (100 ml × 2). The combined organic layers were washed with brine (100 ml × 2), dried over sodium sulfate, filtered and concentrated under reduced pressure. The residue was purified by column chromatography (silica gel, petroleum ether/ethyl acetate = 1:0 to 3:1) to give 2-[2-[bis[(2,4-dimethoxyphenyl)methyl]amino]-4,6-dimethoxy-pyrimidin-5-yl]cyclopropanecarbonitrile (3.0 g, 54% yield) as yellow oil. MS (ESI) m/z= 521.3 [M+H]⁺

### Step 5: 2-(2-amino-4,6-dimethoxy-pyrimidin-5-yl)cyclopropanecarbonitrile

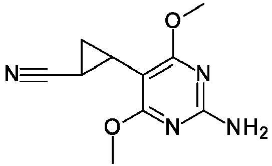

To a solution of 2-[2-[bis[(2,4-dimethoxyphenyl)methyl]amino]-4,6-dimethoxy-pyrimidin-5-yl]cyclopropanecarbonitrile (3.0 g, 5.76 mmol) in dichloromethane (10 ml) was added trifluoroacetic acid (15 ml, 197 mmol) dropwise at 10 °C and the mixture was stirred at 10 °C for 4 h. The reaction mixture was diluted with water (100 ml) and adjusted pH to 7 with saturated sodium bicarbonate solution at 0-10 °C. The mixture was extracted with ethyl acetate (50 ml × 2). The combined organic layers were washed with brine (50 ml × 2), dried over sodium sulfate, filtered and concentrated under reduced pressur. The residue was triturated with ethyl acetate (4 ml) and filtered. The filter cake was dried under reduced pressure to give the title compound (907 mg, 70 % yield) as a white solid. MS (ESI) m/z= 221.1 [M+H]⁺

### Intermediate B10: 4,6-dimethoxy-5-methyl-pyrimidin-2-amine

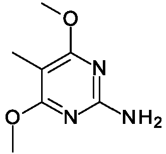

Intermediate B10 is known (CAS 341009-90-3).

### Intermediate B11: 5-bromo-4,6-dimethoxy-pyrimidin-2-amine

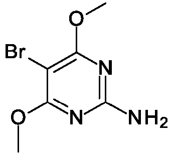

To a stirred solution of (4,6-dimethoxypyrimidin-2-yl)amine (7 g, 44.2 mmol, CAS: 36315-01-2) in acetonitrile (100 ml) was added a solution of N-bromosuccinimide (10.3 g, 57.5 mmol) in acetonitrile (100 ml) dropwise at room temperature. The reaction mixture was stirred at room temperature for 30 min. The resulting white suspension was diluted with ethyl acetate and washed with water. The organic layer was dried over sodium sulfate, filtered, diluted with heptane and concentrated in vacuo. The precipitate was filtered off and washed with heptane to provide the title compound as a white solid (9.26 g, 87 % yield). MS (ESI) m/z= 234.1 [M+H]⁺.

### Intermediate B12: 3-(2-amino-4,6-dimethoxy-pyrimidin-5-yl)propanenitrile

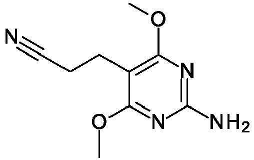

### Step 1: 3-[2-[bis[(4-methoxyphenyl)methyl]amino]-4,6-dimethoxy-pyrimidin-5-yl]propanenitrile

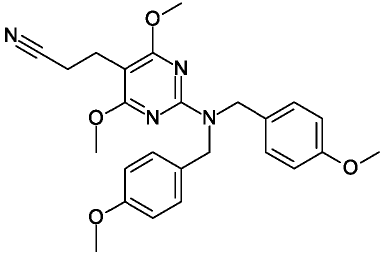

To a solution of 5-bromo-4,6-dimethoxy-N,N-bis[(4-methoxyphenyl)methyl]pyrimidin-2-amine (5.0 g, 10.54 mmol, see Intermediate B4 step 2) in methoxycyclopentane (75.0 ml) was added 3-(4,4,5,5-tetramethyl-1,3,2-dioxaborolan-2-yl)propanenitrile (2.29 g, 12.6 mmol), cesium carbonate (10.3 g, 31.62 mmol) and cataCXium^{®} A Pd G3 (768 mg, 1.05 mmol, CAS: 1651823-59-4) under nitrogen. The mixture was purged three times with nitrogen, stirred at 90 °C for 12 h under nitrogen, poured into NaHCO3 solution and extracted three times with ethyl acetate. The combined organic layers were washed with dried over sodium sulfate, filtered and concentrated in vacuo. The residue was purified by flash chromatography (silica gel, ethyl acetate / petroleum ether 0-30%) to provide the title compound as a yellow solid (2.9 g, 61 % yield). MS (ESI) m/z= 449.0 [M+H]⁺.

### Step 2: 3-(2-amino-4,6-dimethoxy-pyrimidin-5-yl)propanenitrile

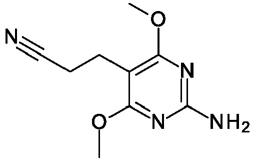

A mixture of 3-[2-[bis[(4-methoxyphenyl)methyl]amino]-4,6-dimethoxy-pyrimidin-5-yl]propanenitrile (2300 mg, 5.13 mmol) in trifluoroacetic acid (23 ml) was stirred at 25 °C for 48 h, quenched with ice/saturated NaHCO3 solution and extracted three times with ethyl acetate. The combined organic layers were dried over sodium sulfate, filtered and concentrated in vacuo. The residue was purified by flash chromatography (silica gel, ethyl acetate / petroleum ether 0-100%) to provide the title compound as a yellow solid (1000 mg, 89 % yield). MS (ESI) m/z= 209.0 [M+H]⁺.

### Intermediate B13: 2-(2-amino-4,6-dimethoxy-pyrimidin-5-yl)oxyacetonitrile

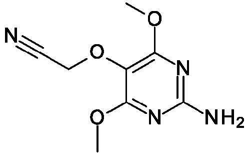

### Step 1: 2-[2-[bis[(4-methoxyphenyl)methyl]amino]-4,6-dimethoxy-pyrimidin-5-yl]oxyacetonitrile

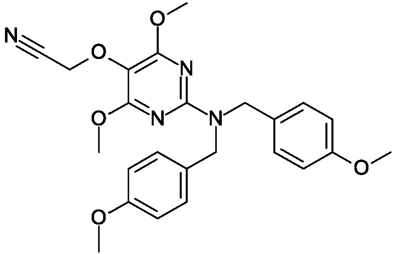

A mixture of 2-[bis[(4-methoxyphenyl)methyl]amino]-4,6-dimethoxy-pyrimidin-5-ol (1 g, 2.43 mmol, see Intermediate B4 step 3), iodoacetonitrile (1.22 g, 529 ul, 7.29 mmol) and potassium carbonate (1.01 g, 7.29 mmol) in N,N-dimethylformamide (16 ml) was stirred at 80°C overnight. The reaction mixture was poured into water and extracted twice with ethyl acetate. The organic layers were washed with brine, dried over sodium sulfate, filtered and concentrated in vacuo. The residue was purified by flash chromatography (silica gel, ethyl acetate / heptane 0-20%) to provide the title compound as a white solid (855 mg, 77 % yield). MS (ESI) m/z= 451.3 [M+H]⁺.

### Step 2: 2-(2-amino-4,6-dimethoxy-pyrimidin-5-yl)oxyacetonitrile

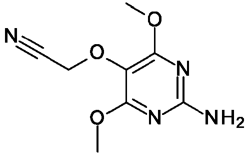

A mixture of 2-[2-[bis[(4-methoxyphenyl)methyl]amino]-4,6-dimethoxy-pyrimidin-5-yl]oxyacetonitrile (855 mg, 1.9 mmol) and trifluoroacetic acid (6.49 g, 4.39 mL, 56.94 mmol) in dichloromethane (6 ml) was stirred at 50 °C overnight. The reaction mixture was concentrated in vacuo, poured into saturated NaHCO3 solution and extracted twice with ethyl acetate. The combined organic layers were washed with brine, dried over sodium sulfate, filtered and concentrated in vacuo. The residue was purified by flash chromatography (silica gel, ethyl acetate / heptane 0-39%) to provide the title compound as an off-white solid (406 mg, 92 % yield). MS (ESI) m/z= 211.1 [M+H]⁺.

### Intermediate B14: 4,6-dimethoxy-5-(1,1,2-trifluoroethoxy)pyrimidin-2-amine

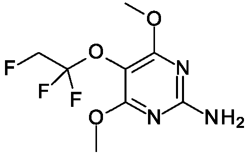

Intermediate B14 is known (CAS 2827058-64-8).

### Intermediate B15: 5-(2-fluoro-1,1-dimethyl-ethoxy)-4,6-dimethoxy-pyrimidin-2-amine

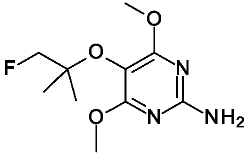

### Step 1: ethyl 2-[2-[bis[(4-methoxyphenyl)methyl]amino]-4,6-dimethoxy-pyrimidin-5-yl]oxy-2-methyl-propanoate

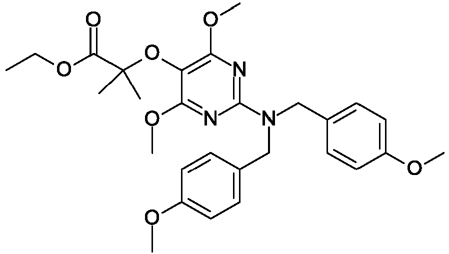

To a stirred solution of 2-[bis[(4-methoxyphenyl)methyl]amino]-4,6-dimethoxy-pyrimidin-5-ol (975 mg, 2.2 mmol, see Intermediate B4 step 3) in acetonitrile (15 ml) was added potassium carbonate (914 mg, 6.61 mmol) at room temperature. The reaction mixture was heated to reflux and stirred for 1 h. Then ethyl alpha-bromoisobutyrate (1.33 g, 1.0 ml, 6.61 mmol) was added dropwise, and the reaction mixture was continued stirring at 82°C for 16 h. The reaction mixture was diluted with water and extracted twice with ethyl acetate. Sodium hydroxide solution (1 N) was added to facilitate phase separation. The organic layers were washed with brine, dried over Na2SO4, filtered and concentrated in vacuo. The residue was purified by flash column chromatography (silica gel, 0% to 40 % ethyl acetate in heptane) to provide ethyl 2-[2-[bis[(4-methoxyphenyl)methyl]amino]-4,6-dimethoxy-pyrimidin-5-yl]oxy-2-methyl-propanoate (752 mg, 63%) as colorless oil. MS (ESI) m/z: 527.3 [M+H]⁺.

### Step 2: 2-[2-[bis[(4-methoxyphenyl)methyl]amino]-4,6-dimethoxy-pyrimidin-5-yl]oxy-2-methyl-propan-1-ol

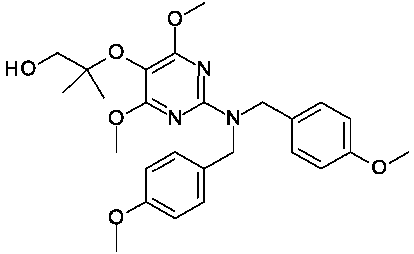

A solution of ethyl 2-[2-[bis[(4-methoxyphenyl)methyl]amino]-4,6-dimethoxy-pyrimidin-5-yl]oxy-2-methyl-propanoate (748 mg, 1.38 mmol) in tetrahydrofuran (12 ml) was cooled to 0°C. Then 1 M lithium tetrahydroaluminate in THF (1.88 g, 2.07 ml, 2.07 mmol) was added dropwise. The reaction mixture was stirred at 0°C for 4.5 h. The reaction mixture was quenched with NH4Cl solution, poured into brine, and extracted twice with ethyl acetate. The combined organic layers were dried over Na2SO4, filtered and concentrated in vacuo to provide 2-[2-[bis[(4-methoxyphenyl)methyl]amino]-4,6-dimethoxy-pyrimidin-5-yl]oxy-2-methyl-propan-1-ol (728 mg, 100%) as colorless oil. MS (ESI) m/z: 483.3 [M+H]⁺.

### Step 3: 5-(2-fluoro-1,1-dimethyl-ethoxy)-4,6-dimethoxy-N,N-bis[(4-methoxyphenyl)methyl]pyrimidin-2-amine

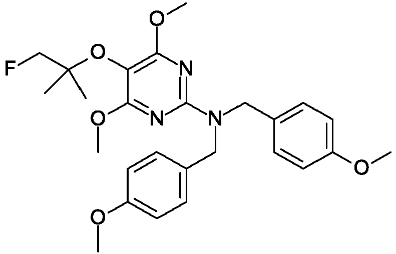

To a stirred solution of 2-[2-[bis[(4-methoxyphenyl)methyl]amino]-4,6-dimethoxy-pyrimidin-5-yl]oxy-2-methyl-propan-1-ol (661 mg, 1.34 mmol) in tetrahydrofuran (12 ml) was added perfluorobutanesulfonyl fluoride (1.62 g, 941 ul, 5.36 mmol), triethylamine trihydrofluoride (891 mg, 900 ul, 5.36 mmol) and triethylamine (1.63 g, 2.24 ml, 16.08 mmol). The reaction mixture was stirred at 60°C for 18 h. The reaction mixture was poured into ice cold saturated NaHCO3 solution and extracted twice with ethyl acetate. The combined organic layers were washed with brine, dried with Na2SO4, filtered and concentrated in vacuo. The crude mixture was suspended in dichloromethane and filtered through Celite to remove insoluble impurities. The residue was purified by flash column chromatography (silica gel, 0% to 30% ethyl acetate in heptane) to obtain 5-(2-fluoro-1,1-dimethyl-ethoxy)-4,6-dimethoxy-N,N-bis[(4-methoxyphenyl)methyl]pyrimidin-2-amine (169 mg, 21%) as white solid. MS (ESI) m/z: 486.3 [M+H]⁺.

### Step 4: 5-(2-fluoro-1,1-dimethyl-ethoxy)-4,6-dimethoxy-pyrimidin-2-amine

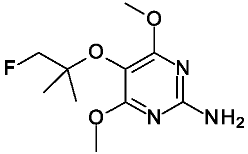

To a stirred solution of 5-(2-fluoro-1,1-dimethyl-ethoxy)-4,6-dimethoxy-N,N-bis[(4-methoxyphenyl)methyl]pyrimidin-2-amine (135 mg, 228 umol) in dichloromethane (2 ml) was added trifluoroacetic acid (3.12 g, 2.0 ml, 27.36 mmol) slowly at 0°C. The ice bath was removed and the reaction mixture was stirred for 40 h at room temperature. The reaction mixture was carefully poured into saturated NaHCO3 solution and extracted twice with ethyl acetate. The organic layers were washed with brine, dried over Na2SO4, filtered and concentrated in vacuo. The residue was purified by flash column chromatography (silicagel, 0% to 40% ethyl acetate in heptane in 15 min), followed by reversed-phase flash column chromatography (C18 15 g, 0% to 100% acetonitrile in water) to provide 5-(2-fluoro-1,1-dimethyl-ethoxy)-4,6-dimethoxy-pyrimidin-2-amine (27 mg, 48%) as white powder. MS (ESI) m/z: 246.2 [M+H]⁺.

### Intermediate B16: 4,6-dimethoxy-5-(1,1,2,2-tetradeuterio-2-fluoro-ethoxy)pyrimidin-2-amine

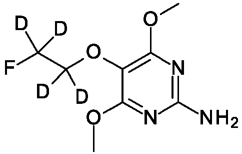

### Step 1: N,N-bis[(2,4-dimethoxyphenyl)methyl]-4,6-dimethoxy-pyrimidin-2-amine

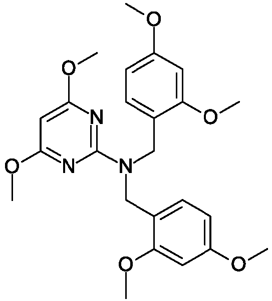

To a solution of 2-chloro-4,6-dimethoxypyrimidine (20.0 g, 115 mmol) and 1-(2,4-dimethoxyphenyl)-N-[(2,4-dimethoxyphenyl)methyl]methanamine (43.6 g, 137.5 mmol) in N-methyl-2-pyrrolidone (400 ml) was added cesium carbonate (74.7 g, 229 mmol). The mixture was stirred at 120°C for 16 h. The reaction mixture was diluted with water (500 ml) and extracted with ethyl acetate (500 ml × 2). The combined organic layers were washed with brine (200 ml × 2), dried over sodium sulfate, filtered and concentrated in vacuo. The residue was triturated in ethyl acetate. The obtained solid was filtered and dried to provide 25 g of the title compound. The filtrate was concentrated in vacuo. The residue was purified by flash chromatography over silica gel using a gradient of ethyl acetate/ether 0-100% to provide a white solid. This solid was combined with the solid obtained above by filtration to provide the title compound as a white solid (43 g, 75% yield). MS (ESI): m/z= 456.2 [M+H]+

### Step 2: 5-bromo-N,N-bis[(2,4-dimethoxyphenyl)methyl]-4,6-dimethoxy-pyrimidin-2-amine

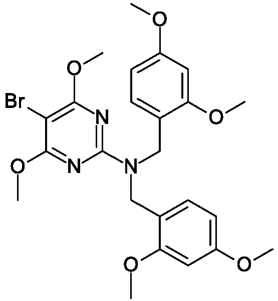

To a solution of N,N-bis[(2,4-dimethoxyphenyl)methyl]-4,6-dimethoxy-pyrimidin-2-amine (32.0 g, 70.25 mmol) in acetonitrile (320 ml) was added N-bromosuccinimide (11.25 g, 63.23 mmol). The mixture was stirred at 20 °C for 3 h. After completion of the reaction,the mixture was quenched by sodium sulfite (500 ml) aqueous solution and stirred at 20 °C for 0.5 h. The mixture was extracted with dichloromethane (500 ml × 2). The combined organic layers were washed with brine (500 ml × 2), dried over sodium sulfate, filtered and concentrated in vacuo. The gum was triturated in ethyl acetate, the formed precipitate was filtered to provide the title compound as a white solid (28g, 62 % yield). MS (ESI): m/z= 534.2 [M+H]⁺.

### Step 3: 2-[bis[(2,4-dimethoxyphenyl)methyl]amino]-4,6-dimethoxy-pyrimidin-5-ol

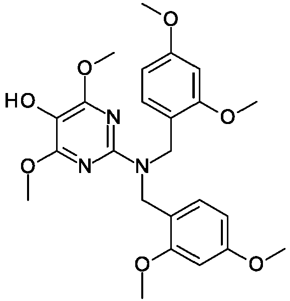

To a solution of 5-bromo-N,N-bis[(2,4-dimethoxyphenyl)methyl]-4,6-dimethoxy-pyrimidin-2-amine (33.0 g, 61.75 mmol) in THF (650 ml) was added n-BuLi (2.5 N in hexanes, 32.1 ml, 80.28 mmol) at -70°C. The mixture was stirred at -70°C for 0.5 h. Then Trimethyl borate (13.8 ml, 123.5 mmol) was added at -70°C and the mixture was stirred at -70 °C for 2 h. The mixture was warmed to 0 °C and acetic acid (7.84 ml, 135.8 mmol), hydrogen peroxide (13.87 ml, 135.8 mmol) were added at 0 °C. The mixture was stirred at 20°C for 2 h. After completion of the reaction, the mixture was quenched by sodium sulfite (500 ml) aqueous solution, stirred at 20 °C for 0.5 h and extracted with ethyl acetate (20 ml × 2). The combined organic layers were washed with brine (20 ml × 2), dried over sodium sulfate, filtered and concentrated in vacuo. The residue was purified by flash chromatography over silica gel using a gradient ethyl acetate/ether 0-50% to provide the title compound as a yellow oil (23.8 g, 76% yield). MS (ESI): m/z= 472.3 [M+H]+

### Step 4: [2-[2-[bis[(2,4-dimethoxyphenyl)methyl]amino]-4,6-dimethoxy-pyrimidin-5-yl]oxy-1,1,2,2-tetradeuterio-ethyl] 4-methylbenzenesulfonate

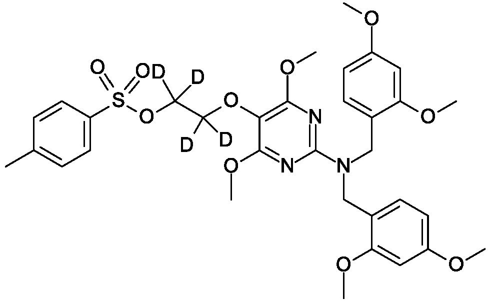

To a solution of 2-[bis[(2,4-dimethoxyphenyl)methyl]amino]-4,6-dimethoxy-pyrimidin-5-ol (2.0 g, 4.24 mmol) in dimethylformamide (20 ml) was added [1,1,2,2-tetradeuterio-2-(p-tolylsulfonyloxy)ethyl] 4-methylbenzenesulfonate (2.38 g, 6.36 mmol, CAS: 164936-35-0) and cesium carbonate (2.76 g, 8.48 mmol) and the reaction was stirred vigorously at 60°C temperature for 1 h. The reaction mixture was diluted with water (100 ml) and extracted with ethyl acetate (200 ml × 2). The combined organic layers were washed with brine, dried over Na2SO4 and concentrated under reduced pressure to give a residue. The precipitate was triturated in ethyl acetate and collected by filtration to give [2-[2-[bis[(2,4-dimethoxyphenyl)methyl]amino]-4,6-dimethoxy-pyrimidin-5-yl]oxy-1,1,2,2-tetradeuterio-ethyl] 4-methylbenzenesulfonate (10.0 g, 19% yield) as a white solid. MS (ESI): m/z= 674.4 [M+H]⁺.

### Step 5: N,N-bis[(2,4-dimethoxyphenyl)methyl]-4,6-dimethoxy-5-(1,1,2,2-tetradeuterio-2-fluoro-ethoxy)pyrimidin-2-amine

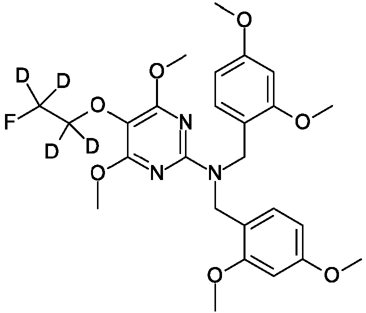

To a mixture of [2-[2-[bis[(2,4-dimethoxyphenyl)methyl]amino]-4,6-dimethoxy-pyrimidin-5-yl]oxy-1,1,2,2-tetradeuterio-ethyl] 4-methylbenzenesulfonate (8.0 g, 11.87 mmol) in acetonitrile (80 ml) was added tetrabutylammonium fluoride solution (1M in THF, 35.6 ml, 35.6 mmol) and the mixture was stirred at 85°C for 2 h. After cooling down the reaction mixture was concentrated under reduced pressure and the residue was diluted with ethyl acetate (300 ml) and washed with brine (100 ml × 3), dried over Na2SO4, filtered and concentrated under reduced pressure. The residue was purified by column chromatography (silica gel, petroleum ether / ethyl acetate = 1:0 to 5:1) to give N,N-bis[(2,4-dimethoxyphenyl)methyl]-4,6-dimethoxy-5-(1,1,2,2-tetradeuterio-2-fluoro-ethoxy)pyrimidin-2-amine (6.0 g, 21.09 mmol, 97% yield) as a white solid. MS (ESI): m/z= 522.4 [M+H]⁺.

### Step 6: 4,6-dimethoxy-5-(1,1,2,2-tetradeuterio-2-fluoro-ethoxy)pyrimidin-2-amine

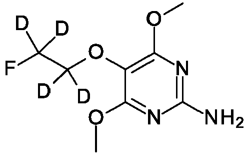

To a solution of N,N-bis[(2,4-dimethoxyphenyl)methyl]-4,6-dimethoxy-5-(1,1,2,2-tetradeuterio-2-fluoro-ethoxy)pyrimidin-2-amine (10.0 g, 19.17 mmol) in dichloromethane (50 ml) was added trifluoroacetic acid (50 ml, 673 mmol) at 0-10°C and the mixture was stirred at 25°C for 2 h . The pH of the reaction mixture was adjusted to 7 with saturated NaHCO3 solution and the mixture was extracted with ethyl acetate (200 ml × 2). The combined organic layers were washed with brine, dried over Na2SO4, filtered and concentrated to give 4,6-dimethoxy-5-(1,1,2,2-tetradeuterio-2-fluoro-ethoxy)pyrimidin-2-amine (4.14 g, 94% yield) as a white solid, MS (ESI): m/z= 222.2 [M+H]⁺.

### Intermediate B17: 5-(1,1-dideuterio-2,2-difluoro-ethyl)-4-methoxy-pyrimidin-2-amine

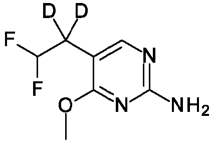

### Step 1: 1-[2-[bis[(2,4-dimethoxyphenyl)methyl]amino]-4-methoxy-pyrimidin-5-yl]-2,2-difluoroethanone

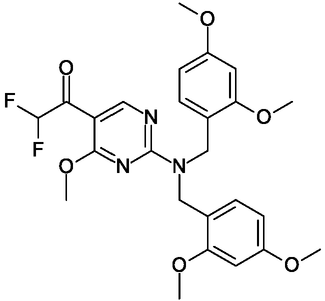

To a solution of 5-bromo-N,N-bis[(2,4-dimethoxyphenyl)methyl]-4-methoxy-pyrimidin-2-amine (2.00 g, 3.97 mmol, CAS 2827058-10-4) in tetrahydrofuran (3 ml) was added n-BuLi solution (2.5 M in hexanes, 1.91 ml, 4.77 mmol) in portions at -70°C, and the solution was stirred at - 70°C for 0.5 h. Then ethyl difluoroacetate (1.31 ml, 11.92 mmol) was added in portions at -70°C. The solution was stirred at 20°C for 2 h. The reaction mixture was poured into a saturated ammonium chloride solution (250 mL) and extracted with ethyl acetate (150 ml x 3). The organic layer was washed with brine, dried over sodium sulfate and concentrated in vacuo. The residue was purified by flash chromatography over silica gel using a gradient ethyl acetate/ether 0-30% to provide the title compound as a yellow oil (0.8 g, 33 % yield). MS (ESI): m/z= 504.1 [M+H]+

### Step 2: 5-(1,1-dideuterio-2,2-difluoro-ethyl)-N,N-bis[(2,4-dimethoxyphenyl)methyl]-4-methoxy-pyrimidin-2-amine

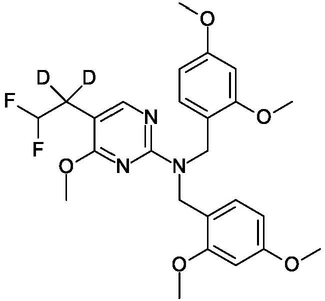

To a solution of lithium aluminium deuteride (2.0 g, 47.67 mmol) and aluminium trichloride (6.99 g, 52.43 mmol) in diethyl ether (200 mL) stirred for 15 min under a nitrogen atmosphere, was added a solution of 1-[2-[bis[(2,4-dimethoxyphenyl)methyl]amino]-4-methoxy-pyrimidin-5-yl]-2,2-difluoro-ethanone (24.0 g, 47.67 mmol) in diethyl ether (50 ml). The mixture was stirred at 20 °C for 2 h, quenched with D₂O (100 ml) followed by 6N sulfuric acid (100 ml) and then diluted with water (150 ml). The aqueous layer was extracted with ethyl acetate (3x300 ml), the combined organic layers were washed with water, and with a 10% sodium bicarbonate aqueous solution. The combined organic phases were dried over sodium sulfate, filtered, and concentrated in vacuo. The residue was purified by flash chromatography over silica gel using a gradient ethyl acetate/ether 0-30% to provide the title compound as a yellow oil (15 g, 64% yield). MS (ESI): m/z= 492.2 [M+H]⁺.

### Step 3: 5-(1,1-dideuterio-2,2-difluoro-ethyl)-4-methoxy-pyrimidin-2-amine

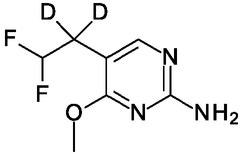

The title compound was prepared in analogy to intermediate B16 step 6 from 5-(1,1-dideuterio-2,2-difluoro-ethyl)-N,N-bis[(2,4-dimethoxyphenyl)methyl]-4-methoxy-pyrimidin-2-amine as a white solid. MS (ESI): m/z= 192.1 [M+H]⁺.

### Intermediate B18: 5-(1,1-dideuterio-2,2-difluoro-ethyl)-4,6-dimethoxy-pyrimidin-2-amine

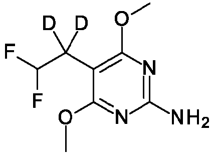

The title compound was prepared in analogy to Intermediate B17 from 5-bromo-N,N-bis[(2,4-dimethoxyphenyl)methyl]-4,6-dimethoxy-pyrimidin-2-amine (see Intermediate B16 step 2) instead of 5-bromo-N,N-bis[(2,4-dimethoxyphenyl)methyl]-4-methoxy-pyrimidin-2-amine in step 1) as white solid. MS (ESI) m/z: 222.1 [M+H]⁺.

### Intermediate B19: 5-(1,1-dideuterio-2,2-difluoro-ethoxy)-4,6-dimethoxy-pyrimidin-2-amine

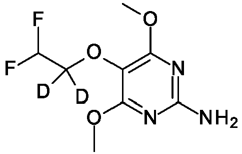

### Step 1: 1,1-dideuterio-2,2-difluoro-ethanol

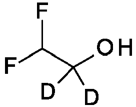

To the suspension of lithium aluminium deuteride (437 mg, 10.4 mmol) in tetrahydrofuran (20 ml) was added slowly difluoroacetic acid (1.0 g, 10.4 mmol) at 0 °C under nitrogen, then the mixture was stirred at 60°C for 2 h under nitrogen atmosphere. After cooling to 20°C, wet Na2SO4 was added to the above mixture until bubbling ceased. Then dichloromethane (30 ml) and dry Na2SO4 was added and the mixture was stirred for 5 min, then filtered and evaporated carefully. A colorless liquid was obtained (875 mg, 99% yield) which was directly used for the next step.

### Step 2: (1,1-dideuterio-2,2-difluoro-ethyl) 4-methylbenzenesulfonate

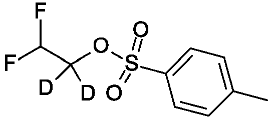

To a solution of p-toluenesulfonyl chloride (2.38 g, 12.5 mmol) in dichloromethane (10 ml) were added triethylamine (2.16 ml, 15.6 mmol), N,N-dimethylpyridin-4-amine (64 mg, 0.52 mmol, 0.05 eq) and 1,1-dideuterio-2,2-difluoro-ethanol (875 mg, 10.4 mmol) and the mixture was stirred at 20° C for 12 h. The reaction mixture was quenched by addition of saturated.NaHCO3 solution (80 ml) at 20 °C, and then extracted with ethyl acetate (50 ml x 3). The combined organic layers were washed with brine, dried over Na2SO4, filtered and concentrated under reduced pressure. The residue was purified by flash chromatography (silica gel, ethyl acetate / petroleum ether) to give (1,1-dideuterio-2,2-difluoro-ethyl) 4-methylbenzenesulfonate (1.44 g, 58% yield) as a colorless oil, MS (ESI) m/z: 238.8 [M+H]⁺.

### Step 3: 5-(1,1-dideuterio-2,2-difluoro-ethoxy)-N,N-bis[(2,4-dimethoxyphenyl)methyl]-4,6-dimethoxy-pyrimidin-2-amine

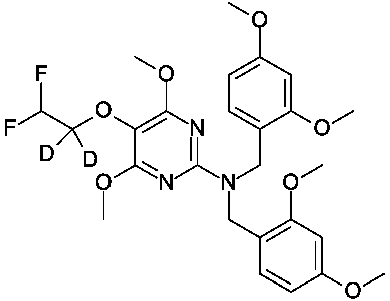

The title compound was prepared in analogy to Intermediate B16 from (1,1-dideuterio-2,2-difluoro-ethyl) 4-methylbenzenesulfonate instead of [1,1,2,2-tetradeuterio-2-(p-tolylsulfonyloxy)ethyl] 4-methylbenzenesulfonate in step 4) as a yellow solid, MS (ESI): m/z= 538.2 [M+H]⁺.

### Step 4: 5-(1,1-dideuterio-2,2-difluoro-ethoxy)-4,6-dimethoxy-pyrimidin-2-amine

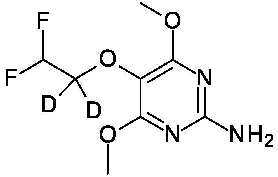

The title compound was prepared in analogy to Intermediate B16 from 5-(1,1-dideuterio-2,2-difluoro-ethoxy)-N,N-bis[(2,4-dimethoxyphenyl)methyl]-4,6-dimethoxy-pyrimidin-2-amine instead of N,N-bis[(2,4-dimethoxyphenyl)methyl]-4,6-dimethoxy-5-(1,1,2,2-tetradeuterio-2-fluoro-ethoxy)pyrimidin-2-amine in step 6) as off-white solid, MS (ESI): m/z= 238.0 [M+H]⁺.

### Examples

### Example 1: N-[5-(2,2-difluoroethyl)-4,6-dimethoxy-pyrimidin-2-yl]-7-(3-methylpyrazol-1-yl)-1H-indole-3-sulfonamide

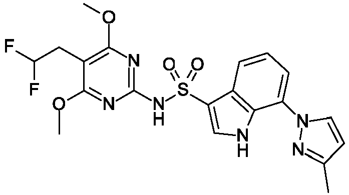

### Step 1: 7-bromo-1H-indole-3-sulfonyl chloride

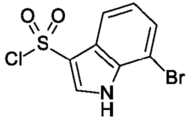

A solution of 7-bromo-1H-indole (1 g, 5.1 mmol) in acetonitrile (12 ml) was cooled to 0°C, then chlorosulfonic acid (1.78 g, 1.02 mL, 15.3 mmol) was added slowly. The ice-bath was removed and the reaction mixture was stirred at room temperature for 3h. The reaction mixture was poured into ice and water and extracted with ethyl acetate twice. The combined organic layers were washed with ice and brine, dried over sodium sulfate, filtered and concentrated in vacuo. The crude material was used without further purification.

### Step 2: 7-bromo-N-[5-(2,2-difluoroethyl)-4,6-dimethoxy-pyrimidin-2-yl]-1H-indole-3-sulfonamide

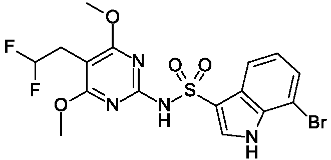

To a stirred solution of 5-(2,2-difluoroethyl)-4,6-dimethoxy-pyrimidin-2-amine (Intermediate B1, 330 mg, 1.51 mmol) and N-ethyl diisopropylamine (389 mg, 515 ul, 3.0 mmol) in dichloromethane (10 ml) was added a solution of 7-bromo-1H-indole-3-sulfonyl chloride (475 mg, 1.61 mmol) in ethyl acetate (5 ml) within 15 min . The reaction mixture was stirred at room temperature for 15 min, then it was poured into water and extracted twice with ethyl acetate. The combined organic layers were dried over sodium sulfate, filtered and concentrated in vacuo. The residue was purified by flash chromatography (silica gel, 0% to 80% ethyl acetate in heptane) to afford 7-bromo-N-[5-(2,2-difluoroethyl)-4,6-dimethoxy-pyrimidin-2-yl]-1H-indole-3-sulfonamide (390 mg, 49%) as off-white solid. MS (ESI) m/z= 479.1 [M+H]⁺

### Step 3: N-[5-(2,2-difluoroethyl)-4,6-dimethoxy-pyrimidin-2-yl]-7-(3-methylpyrazol-1-yl)-1H-indole-3-sulfonamide

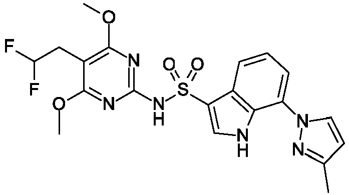

In a small glass tube, 7-bromo-N-[5-(2,2-difluoroethyl)-4,6-dimethoxy-pyrimidin-2-yl]-1H-indole-3-sulfonamide (44 mg, 0.092 mmol),1,4-dioxane (2 ml), potassium carbonate (38 mg, 0.277 mmol), tBuXPhos Pd(allyl)OTf (CAS 1798782-25-8, 5.5 mg, 0.007 mmol, 0.08 eq) and 3-methyl-pyrazole (15 mg, 15 ul, 0.184 mmol) were added under argon and the reaction mixture was stirred at 80°C overnight. After cooling the mixture was filtered over decalite and washed with ethyl acetate. The filtrate was concentrated in vacuo and the residue was purified by flash chromatography (Silicycle SiliaSep Amine, 0 % to 100 % ethyl acetate in heptane) to afford the title compound (12 mg, 24%) as light brown solid. MS (ESI) m/z= 479.1 [M+H]⁺

### Example 2: N-[5-(2,2-difluoroethyl)-4,6-dimethoxy-pyrimidin-2-yl]-7-(3,5-dimethylpyrazol-1-yl)-1H-indole-3-sulfonamide

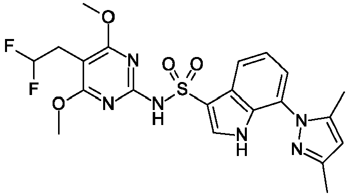

The title compound was prepared in analogy to Example 1 from 3,5-dimethylpyrazole instead of 3-methyl-pyrazole in Step 3) as a white solid. MS (ESI) m/z: 493.2 [M+H]⁺.

### Example 3: N-[5-(2,2-difluoroethyl)-4,6-dimethoxy-pyrimidin-2-yl]-7-imidazol-1-yl-1H-indole-3-sulfonamide

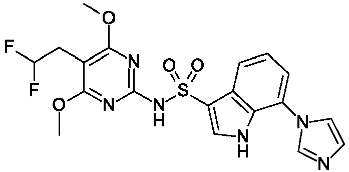

The title compound was prepared in analogy to Example 1 from imidazole instead of 3-methyl-pyrazole and a reaction time of 3 days in Step 3) as a white solid. MS (ESI) m/z: 465.2 [M+H]⁺.

### Example 4: 6-chloro-N-[5-(2,2-difluoroethyl)-4,6-dimethoxy-pyrimidin-2-yl]-7-pyrazol-1-yl-1H-indole-3-sulfonamide

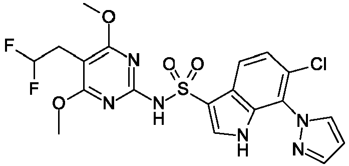

The title compound was prepared in analogy to Example 1 from 7-bromo-6-chloro-1H-indole instead of 7-bromo-1H-indole in Step 1) and pyrazole instead of 3-methyl-pyrazole in Step 3) as a white solid. MS (ESI) m/z: 499.2 [M+H]⁺.

### Example 5: N-[5-(2,2-difluoroethyl)-4,6-dimethoxy-pyrimidin-2-yl]-7-(2-methylimidazol-1-yl)-1H-indole-3-sulfonamide

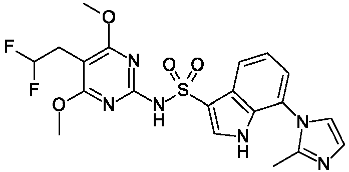

A mixture of 7-(2-methylimidazol-1-yl)-1H-indole-3-sulfonyl chloride (Intermediate A1, 88 mg, 0.30 mmol), 5-(2,2-difluoroethyl)-4,6-dimethoxy-pyrimidin-2-amine (Intermediate B1, 50 mg, 0.23 mmol) and tripotassium phosphate (145 mg, 0.685 mmol) in acetonitrile (5 ml) was stirred for 4 days at room temperature. The reaction mixture was poured into water and extracted with ethyl acetate twice. The combined organic layers were dried over sodium sulfate, filtered and concentrated in vacuo. The residue was purified by flash chromatography (silica gel, 0% to 100% ethyl acetate in heptane followed by 0 % to 20 % methanol in ethyl acetate) to afford the title compound (8 mg, 7%) as off-white solid. MS (ESI) m/z: 479.2 [M+H]⁺.

### Example 6: 6-chloro-N-[5-(2,2-difluoroethoxy)-4,6-dimethoxy-pyrimidin-2-yl]-7-imidazol-1-yl-1H-indole-3-sulfonamide

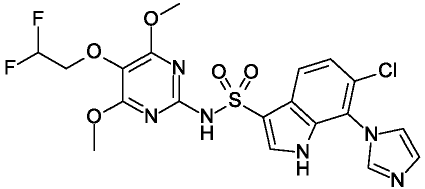

A mixture of 5-(2,2-difluoroethoxy)-4,6-dimethoxy-pyrimidin-2-amine (Intermediate B4, 40 mg, 0.17 mmol) and 6-chloro-7-(triazol-2-yl)-1H-indole-3-sulfonyl chloride (Intermediate A2, 54 mg, 0.17 mmol) in pyridine (1.47 g, 1.5 ml, 18.55 mmol) was stirred at room temperature overnight. The reaction mixture was concentrated in vacuo. The residue was dissolved in ethyl acetate and water and extracted with ethyl acetate twice. The combined organic layers were washed with brine, dried over sodium sulfate and concentrated in vacuo. The residue was purified by flash chromatography (silica gel, 0 % to 10 % methanol in dichloromethane) to afford the title compound (44 mg, 49%) as white solid. MS (ESI) m/z: 515.1 [M+H]⁺.

### Example 7: 6-chloro-N-[5-(2,2-difluoroethyl)-4,6-dimethoxy-pyrimidin-2-yl]-7-(triazol-2-yl)-1H-indole-3-sulfonamide

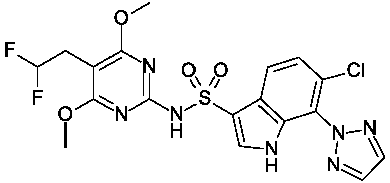

In a 25 ml two-necked flask, 5-(2,2-difluoroethyl)-4,6-dimethoxy-pyrimidin-2-amine (Intermediate B1, 50 mg, 0.228 mmol) and 6-chloro-7-(triazol-2-yl)-1H-indole-3-sulfonyl chloride (Intermediate A2, 94 mg, 0.30 mmol) were dissolved in dichloromethane (8 ml). A solution of N,N-diisopropyl ethylamine (88 mg, 120 ul, 0.68 mmol) in dichloromethane (1.5 ml) was added. The reaction mixture was further stirred at room temperature, then the mixture was poured into water and extracted with ethyl acetate twice. The combined organic layers were washed with brine, dried over sodium sulfate, filtered and concentrated in vacuo. The crude material was purified by flash chromatography (silica gel, 0 % to 80 % ethyl acetate in heptane) followed by another flash chromatography (RediSep Rf Gold C18 column,10% to 80% acetonitrile in water) to provide the title compound (26 mg, 22%) as white solid. MS (ESI) m/z: 500.1 [M+H]⁺.

The following **Examples 8-11** were prepared in analogy to Example 7 by coupling the indicated sulfonylchloride intermediates A and amine intermediates B.

| Ex. | Structure | Name | Int. A | Int. B | MS (ESI): m/z |
|---|---|---|---|---|---|
| 8 | 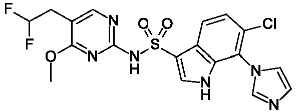 | 6-chloro-N-[5-(2,2-difluoroethyl)-4-methoxy-pyrimidin-2-yl]-7-imidazol-1-yl-1H-indole-3-sulfonamide | A3 | B3 | 469.1 [M+H]⁺ |
| 9 | 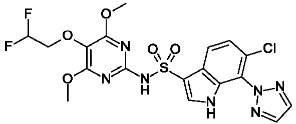 | 6-chloro-N-[5-(2,2-difluoroethoxy)-4,6-dimethoxy-pyrimidin-2-yl]-7-(triazol-2-yl)-1H-indole-3-sulfonamide | A2 | B4 | 516.1 [M+H]⁺ |
| 10 | 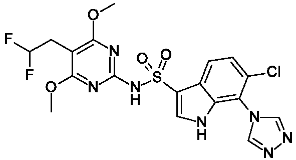 | 6-chloro-N-[5-(2,2-difluoroethyl)-4,6-dimethoxy-pyrimidin-2-yl]-7-(1,2,4-triazol-4-yl)-1H-indole-3-sulfonamide | A11 | B1 | 500.1 [M+H]⁺ |
| 11 | 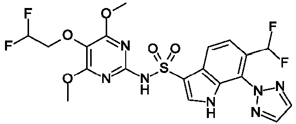 | N-[5-(2,2-difluoroethoxy)-4,6-dimethoxy-pyrimidin-2-yl]-6-(difluoromethyl)-7-(triazol-2-yl)-1H-indole-3-sulfonamide | A5 | B4 | 532.2 [M+H]⁺ |

### Example 12: 6-chloro-N-[5-(2-fluoroethoxy)-4-methoxy-pyrimidin-2-yl]-7-(triazol-2-yl)-1H-indole-3-sulfonamide

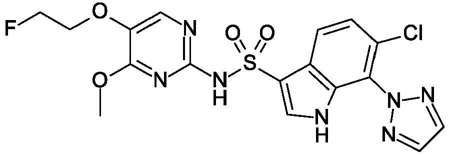

In a 10 ml two-necked flask, 5-(2-fluoroethoxy)-4-methoxy-pyrimidin-2-amine (Intermediate B2, 50 mg, 0.267 mmol) was dissolved in dichloromethane (2 ml), then N-ethyl diisopropylamine (52 mg, 70 ul, 0.4 mmol) and a solution of 6-chloro-7-(triazol-2-yl)-1H-indole-3-sulfonyl chloride (Intermediate A2, 93 mg, 0.294 mmol) in ethyl acetate (3 ml) was added. The reaction mixture was stirred 2 h at room temperature, then it was poured into water and extracted with ethyl acetate twice. The combined organic layers were washed with brine, dried over sodium sulfate, filtered and concentrated in vacuo. The residue was purified by flash chromatography (silica gel, 0 % to 100 % ethyl acetate in heptane) followed by another flash chromatography (RediSep Rf Gold C18 column, 10% to 70% acetonitrile in water) to provide the title compound (45 mg, 35% yield) as white solid. MS (ESI) m/z: 468.2 [M+H]⁺.

The following **Examples 13-41** were prepared in analogy to Example 12 by coupling the indicated sulfonylchloride intermediates A and amine intermediates B.

| Ex. | Structure | Name | Int. A | Int. B | MS (ESI): m/z |
|---|---|---|---|---|---|
| 13 | 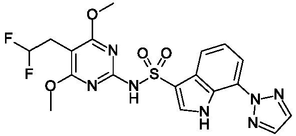 | N-[5-(2,2-difluoroethyl)-4,6-dimethoxy-pyrimidin-2-yl]-7-(triazol-2-yl)-1H-indole-3-sulfonamide | A6 | B1 | 466.3 [M+H]⁺ |
| 14 | 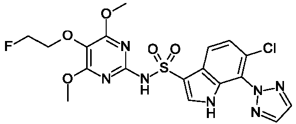 | 6-chloro-N-[5-(2-fluoroethoxy)-4,6-dimethoxy-pyrimidin-2-yl]-7-(triazol-2-yl)-1H-indole-3-sulfonamide | A2 | B5 | 498.1 [M+H]⁺ |
| 15 | 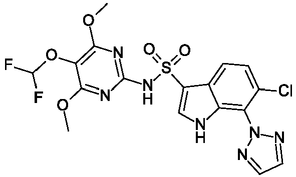 | 6-chloro-N-[5-(difluoromethoxy)-4,6-dimethoxy-pyrimidin-2-yl]-7-(triazol-2-yl)-1H-indole-3-sulfonamide | A2 | B6 | 502.1 [M+H]⁺ |
| 16 | 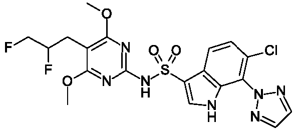 | 6-chloro-N-[5-(2,3-difluoropropyl)-4,6-dimethoxy-pyrimidin-2-yl]-7-(triazol-2-yl)-1H-indole-3-sulfonamide | A2 | B7 | 514.2 [M+H]⁺ |
| 17 | 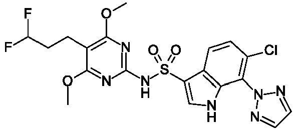 | 6-chloro-N-[5-(3,3-difluoropropyl)-4,6-dimethoxy-pyrimidin-2-yl]-7-(triazol-2-yl)-1H-indole-3-sulfonamide | A2 | B8 | 514.2 [M+H]⁺ |
| 18 | 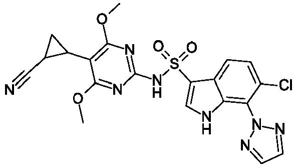 | 6-chloro-N-[5-(2-cyanocyclopropyl)-4,6-dimethoxy-pyrimidin-2-yl]-7-(triazol-2-yl)-1H-indole-3-sulfonamide | A2 | B9 | 501.1 [M+H]⁺ |
| 19 | 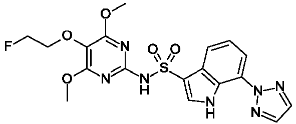 | N-[5-(2-fluoroethoxy)-4,6-dimethoxy-pyrimidin-2-yl]-7-(triazol-2-yl)-1H-indole-3-sulfonamide | A6 | B10 | 464.3 [M+H]⁺ |
| 20 | 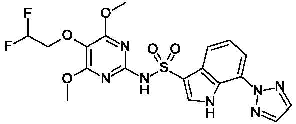 | N-[5-(2,2-difluoroethoxy)-4,6-dimethoxy-pyrimidin-2-yl]-7-(triazol-2-yl)-1H-indole-3-sulfonamide | A6 | B4 | 482.2 [M+H]⁺ |
| 21 | 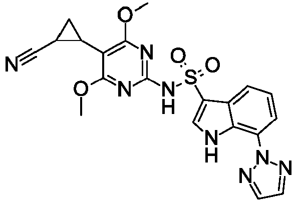 | N-[5-(2-cyanocyclopropyl)-4,6-dimethoxy-pyrimidin-2-yl]-7-(triazol-2-yl)-1H-indole-3-sulfonamide | A6 | B9 | 467.3 [M+H]⁺ |
| 22 | 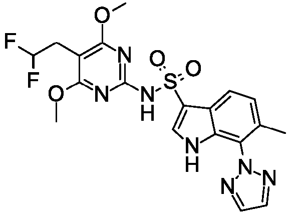 | N-[5-(2,2-difluoroethyl)-4,6-dimethoxy-pyrimidin-2-yl]-6-methyl-7-(triazol-2-yl)-1H-indole-3-sulfonamide | A7 | B1 | 480.2 [M+H]⁺ |
| 23 | 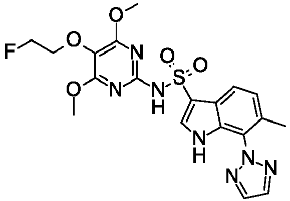 | N-[5-(2-fluoroethoxy)-4,6-dimethoxy-pyrimidin-2-yl]-6-methyl-7-(triazol-2-yl)-1H-indole-3-sulfonamide | A7 | B5 | 478.3 [M+H]⁺ |
| 24 | 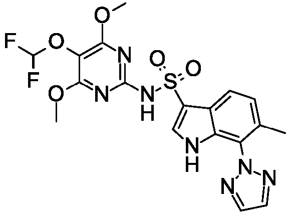 | N-[5-(difluoromethoxy)-4,6-dimethoxy-pyrimidin-2-yl]-6-methyl-7-(triazol-2-yl)-1H-indole-3-sulfonamide | A7 | B6 | 482.2 [M+H]⁺ |
| 25 | 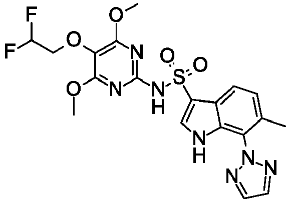 | N-[5-(2,2-difluoroethoxy)-4,6-dimethoxy-pyrimidin-2-yl]-6-methyl-7-(triazol-2-yl)-1H-indole-3-sulfonamide | A7 | B4 | 496.3 [M+H]⁺ |
| 26 | 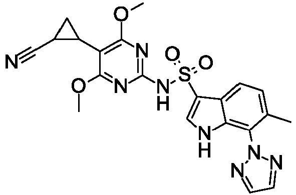 | N-[5-(2-cyanocyclopropyl)-4,6-dimethoxy-pyrimidin-2-yl]-6-methyl-7-(triazol-2-yl)-1H-indole-3-sulfonamide | A7 | B9 | 481.2 [M+H]⁺ |
| 27 | 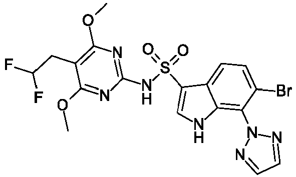 | 6-bromo-N-[5-(2,2-difluoroethyl)-4,6-dimethoxy-pyrimidin-2-yl]-7-(triazol-2-yl)-1H-indole-3-sulfonamide | A8 | B1 | 546.3 [M+H]⁺ |
| 28 | 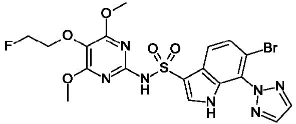 | 6-bromo-N-[5-(2-fluoroethoxy)-4,6-dimethoxy-pyrimidin-2-yl]-7-(triazol-2-yl)-1H-indole-3-sulfonamide | A8 | B5 | 544.2 [M+H]⁺ |
| 29 | 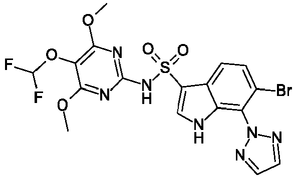 | 6-bromo-N-[5-(difluoromethoxy)-4,6-dimethoxy-pyrimidin-2-yl]-7-(triazol-2-yl)-1H-indole-3-sulfonamide | A8 | B6 | 548.1 [M+H]⁺ |
| 30 | 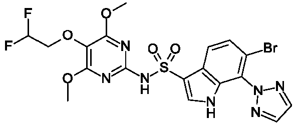 | 6-bromo-N-[5-(2,2-difluoroethoxy)-4,6-dimethoxy-pyrimidin-2-yl]-7-(triazol-2-yl)-1H-indole-3-sulfonamide | A8 | B4 | 562.1 ₊ [M+H] |
| 31 | 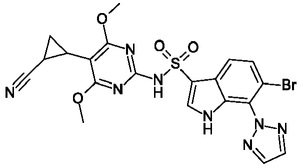 | 6-bromo-N-[5-(2-cyanocyclopropyl)-4,6-dimethoxy-pyrimidin-2-yl]-7-(triazol-2-yl)-1H-indole-3-sulfonamide | A8 | B9 | 545.2 [M+H]⁺ |
| 32 | 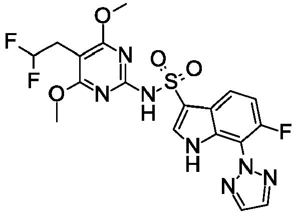 | N-[5-(2,2-difluoroethyl)-4,6-dimethoxy-pyrimidin-2-yl]-6-fluoro-7-(triazol-2-yl)-1H-indole-3-sulfonamide | A9 | B1 | 484.2 [M+H]⁺ |
| 33 | 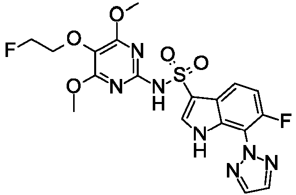 | 6-fluoro-N-[5-(2-fluoroethoxy)-4,6-dimethoxy-pyrimidin-2-yl]-7-(triazol-2-yl)-1H-indole-3-sulfonamide | A9 | B5 | 482.2 [M+H]⁺ |
| 34 | 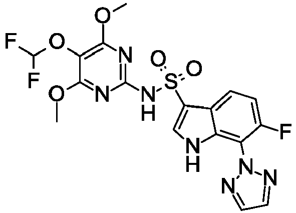 | N-[5-(difluoromethoxy)-4,6-dimethoxy-pyrimidin-2-yl]-6-fluoro-7-(triazol-2-yl)-1H-indole-3-sulfonamide | A9 | B6 | 486.2 [M+H]⁺ |
| 35 | 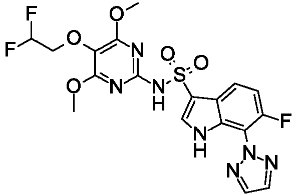 | N-[5-(2,2-difluoroethoxy)-4,6-dimethoxy-pyrimidin-2-yl]-6-fluoro-7-(triazol-2-yl)-1H-indole-3-sulfonamide | A9 | B4 | 500.2 [M+H]⁺ |
| 36 | 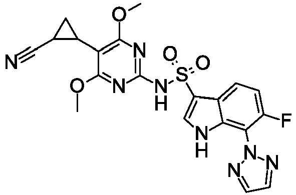 | N-[5-(2-cyanocyclopropyl)-4,6-dimethoxy-pyrimidin-2-yl]-6-fluoro-7-(triazol-2-yl)-1H-indole-3-sulfonamide | A9 | B9 | 485.3 [M+H]⁺ |
| 37 | 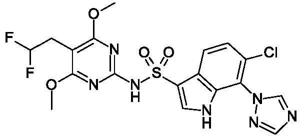 | 6-chloro-N-[5-(2,2-difluoroethyl)-4,6-dimethoxy-pyrimidin-2-yl]-7-(1,2,4-triazol-1-yl)-1H-indole-3-sulfonamide | A10 | B1 | 500.1 [M+H]⁺ |
| 38 | 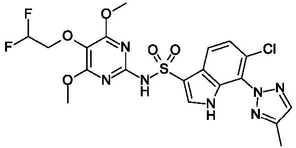 | 6-chloro-N-[5-(2,2-difluoroethoxy)-4,6-dimethoxy-pyrimidin-2-yl]-7-(4-methyltriazol-2-yl)-1H-indole-3-sulfonamide | A12 | B4 | 530.1 [M+H]⁺ |
| 39 | 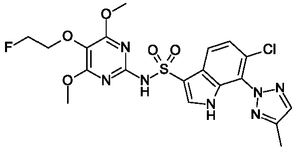 | 6-chloro-N-[5-(2-fluoroethoxy)-4,6-dimethoxy-pyrimidin-2-yl]-7-(4-methyltriazol-2-yl)-1H-indole-3-sulfonamide | A12 | B5 | 512.1 [M+H]⁺ |
| 40 | 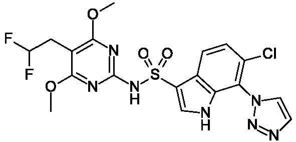 | 6-chloro-N-[5-(2,2-difluoroethyl)-4,6-dimethoxy-pyrimidin-2-yl]-7-(triazol-1-yl)-1H-indole-3-sulfonamide | A4 | B1 | 500.1 [M+H]⁺ |
| 41 | 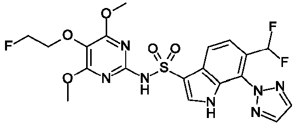 | 6-(difluoromethyl)-N-[5-(2-fluoroethoxy)-4,6-dimethoxy-pyrimidin-2-yl]-7-(triazol-2-yl)-1H-indole-3-sulfonamide | A5 | B5 | 514.3 [M+H]⁺ |
| 42 | 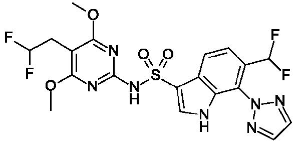 | N-[5-(2,2-difluoroethyl)-4,6-dimethoxy-pyrimidin-2-yl]-6-(difluoromethyl)-7-(triazol-2-yl)-1H-indole-3-sulfonamide | A5 | B1 | 516.2 [M+H]⁺ |

The following **Examples 43-59** were prepared in analogy to Example 7 by coupling the indicated sulfonylchloride intermediates A and amine intermediates B.

| Ex. | Structure | Name | Int. A | Int. B | MS (ESI): m/z |
|---|---|---|---|---|---|
| 43 | 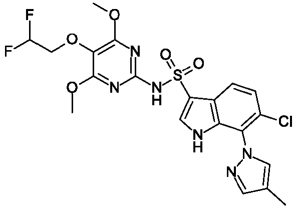 | 6-chloro-N-[5-(2,2-difluoroethoxy)-4,6-dimethoxy-pyrimidin-2-yl]-7-(4-methylpyrazol-1-yl)-1H-indole-3-sulfonamide | A20 | B4 | 529.2 [M+H]⁺ |
| 44 | 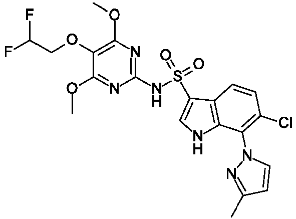 | 6-chloro-N-[5-(2,2-difluoroethoxy)-4,6-dimethoxy-pyrimidin-2-yl]-7-(3-methylpyrazol-1-yl)-1H-indole-3-sulfonamide | A18 | B4 | 529.2 [M+H]⁺ |
| 45 | 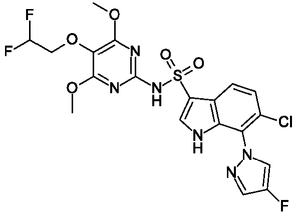 | 6-chloro-N-[5-(2,2-difluoroethoxy)-4,6-dimethoxy-pyrimidin-2-yl]-7-(4-fluoropyrazol-1-yl)-1H-indole-3-sulfonamide | A19 | B4 | 533.2 [M+H]⁺ |
| 46 | 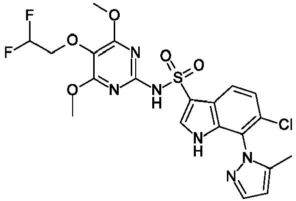 | 6-chloro-N-[5-(2,2-difluoroethoxy)-4,6-dimethoxy-pyrimidin-2-yl]-7-(5-methylpyrazol-1-yl)-1H-indole-3-sulfonamide | A17 | B4 | 529.3 [M+H]⁺ |
| 47 | 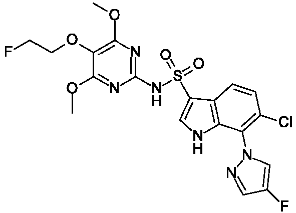 | 6-chloro-N-[5-(2-fluoroethoxy)-4,6-dimethoxy-pyrimidin-2-yl]-7-(4-fluoropyrazol-1-yl)-1H-indole-3-sulfonamide | A19 | B5 | 515.1 [M+H]⁺ |
| 48 | 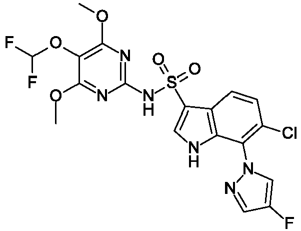 | 6-chloro-N-[5-(difluoromethoxy)-4,6-dimethoxy-pyrimidin-2-yl]-7-(4-fluoropyrazol-1-yl)-1H-indole-3-sulfonamide | A19 | B6 | 519.1 [M+H]⁺ |
| 49 | 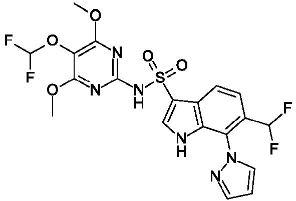 | N-[5-(difluoromethoxy)-4,6-dimethoxy-pyrimidin-2-yl]-6-(difluoromethyl)-7-pyrazol-1-yl-1H-indole-3-sulfonamide | A22 | B6 | 517.1 + [M+H] |
| 50 | 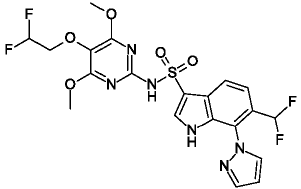 | N-[5-(2,2-difluoroethoxy)-4,6-dimethoxy-pyrimidin-2-yl]-6-(difluoromethyl)-7-pyrazol-1-yl-1H-indole-3-sulfonamide | A22 | B4 | 531.2 [M+H]⁺ |
| 51 | 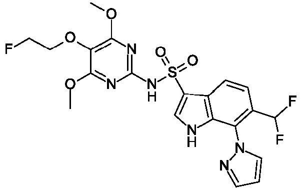 | 6-(difluoromethyl)-N-[5-(2-fluoroethoxy)-4,6-dimethoxy-pyrimidin-2-yl]-7-pyrazol-1-yl-1H-indole-3-sulfonamide | A22 | B5 | 513.2 [M+H]⁺ |
| 52 | 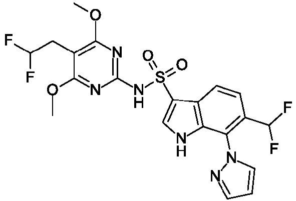 | N-[5-(2,2-difluoroethyl)-4,6-dimethoxy-pyrimidin-2-yl]-6-(difluoromethyl)-7-pyrazol-1-yl-1H-indole-3-sulfonamide | A22 | B1 | 515.2 [M+H]⁺ |
| 53 | 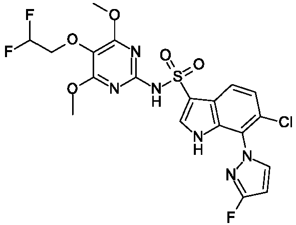 | 6-chloro-N-[5-(2,2-difluoroethoxy)-4,6-dimethoxy-pyrimidin-2-yl]-7-(3-fluoropyrazol-1-yl)-1H-indole-3-sulfonamide | A21 | B4 | 533.1 [M+H]⁺ |
| 54 | 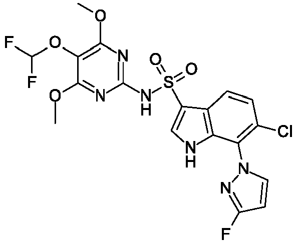 | 6-chloro-N-[5-(difluoromethoxy)-4,6-dimethoxy-pyrimidin-2-yl]-7-(3-fluoropyrazol-1-yl)-1H-indole-3-sulfonamide | A21 | B6 | 519.1 [M+H]⁺ |
| 55 | 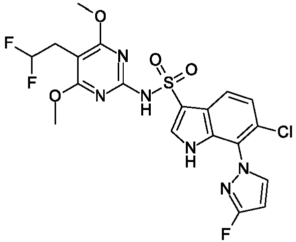 | 6-chloro-N-[5-(2,2-difluoroethyl)-4,6-dimethoxy-pyrimidin-2-yl]-7-(3-fluoropyrazol-1-yl)-1H-indole-3-sulfonamide | A21 | B1 | 517.1 [M+H]⁺ |
| 56 | 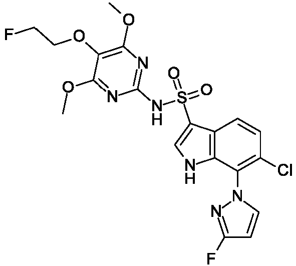 | 6-chloro-N-[5-(2-fluoroethoxy)-4,6-dimethoxy-pyrimidin-2-yl]-7-(3-fluoropyrazol-1-yl)-1H-indole-3-sulfonamide | A21 | B5 | 515.2 [M+H]⁺ |
| 57 | 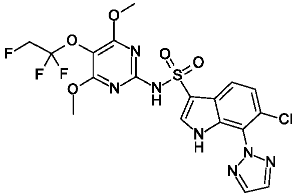 | 6-chloro-N-[4,6-dimethoxy-5-(1,1,2-trifluoroethoxy)pyrimidin-2-yl]-7-(triazol-2-yl)-1H-indole-3-sulfonamide | A2 | B14 | 534.1 [M+H]⁺ |
| 58 | 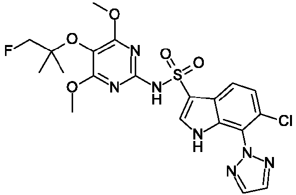 | 6-chloro-N-[5-(2-fluoro-1,1-dimethyl-ethoxy)-4,6-dimethoxy-pyrimidin-2-yl]-7-(triazol-2-yl)-1H-indole-3-sulfonamide | A2 | B15 | 526.1 [M+H]⁺ |
| 59 | 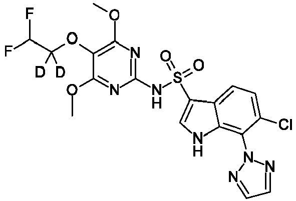 | 6-chloro-N-[5-(1,1-dideuterio-2,2-difluoro-ethoxy)-4,6-dimethoxy-pyrimidin-2-yl]-7-(triazol-2-yl)-1H-indole-3-sulfonamide | A2 | B19 | 518.0 [M+H]⁺ |

The following **Examples 60-86** were prepared in analogy to Example 12 by coupling the indicated sulfonylchloride intermediates A and amine intermediates B.

| Ex. | Structure | Name | Int. A | Int. B | MS (ESI): m/z |
|---|---|---|---|---|---|
| 60 | 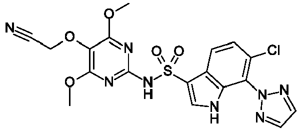 | 6-chloro-N-[5-(cyanomethoxy)-4,6-dimethoxy-pyrimidin-2-yl]-7-(triazol-2-yl)-1H-indole-3-sulfonamide | A2 | B13 | 491.2 [M+H]⁺ |
| 61 | 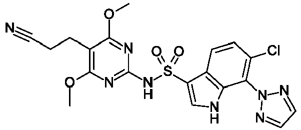 | 6-chloro-N-[5-(2-cyanoethyl)-4,6-dimethoxy-pyrimidin-2-yl]-7-(triazol-2-yl)-1H-indole-3-sulfonamide | A2 | B12 | 489.1 [M+H]⁺ |
| 62 | 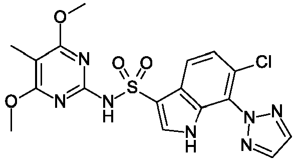 | 6-chloro-N-(4,6-dimethoxy-5-methyl-pyrimidin-2-yl)-7-(triazol-2-yl)-1H-indole-3-sulfonamide | A2 | B10 | 450.2 [M+H]⁺ |
| 63 | 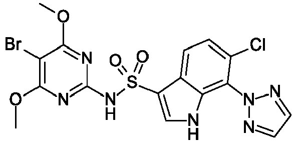 | N-(5-bromo-4,6-dimethoxy-pyrimidin-2-yl)-6-chloro-7-(triazol-2-yl)-1H-indole-3-sulfonamide | A2 | B11 | 516.1 [M+H]⁺ |
| 64 | 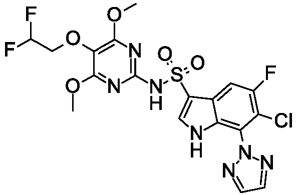 | 6-chloro-N-[5-(2,2-difluoroethoxy)-4,6-dimethoxy-pyrimidin-2-yl]-5-fluoro-7-(triazol-2-yl)-1H-indole-3-sulfonamide | A16 | B4 | 534.2 [M+H]⁺ |
| 65 | 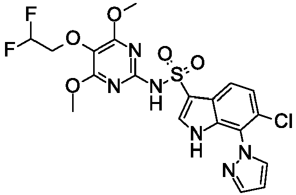 | 6-chloro-N-[5-(2,2-difluoroethoxy)-4,6-dimethoxy-pyrimidin-2-yl]-7-pyrazol-1-yl-1H-indole-3-sulfonamide | A13 | B4 | 515.2 [M+H]⁺ |
| 66 | 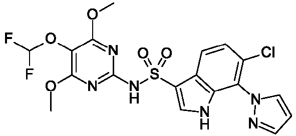 | 6-chloro-N-[5-(difluoromethoxy)-4,6-dimethoxy-pyrimidin-2-yl]-7-pyrazol-1-yl-1H-indole-3-sulfonamide | A13 | B6 | 501.1 [M+H]⁺ |
| 67 | 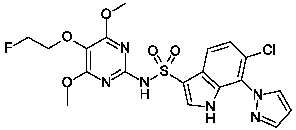 | 6-chloro-N-[5-(2-fluoroethoxy)-4,6-dimethoxy-pyrimidin-2-yl]-7-pyrazol-1-yl-1H-indole-3-sulfonamide | A13 | B5 | 497.2 [M+H]⁺ |
| 68 | 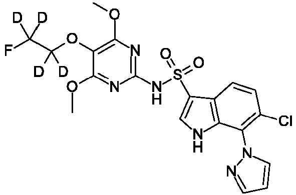 | 6-chloro-N-[4,6-dimethoxy-5-(1,1,2,2-tetradeuterio-2-fluoro-ethoxy)pyrimidin-2-yl]-7-pyrazol-1-yl-1H-indole-3-sulfonamide | A13 | B16 | 501.2 [M+H]⁺ |
| 69 | 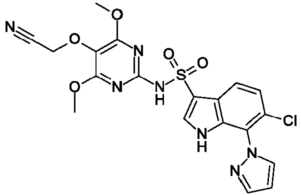 | 6-chloro-N-[5-(cyanomethoxy)-4,6-dimethoxy-pyrimidin-2-yl]-7-pyrazol-1-yl-1H-indole-3-sulfonamide | A13 | B13 | 490.3 [M+H]⁺ |
| 70 | 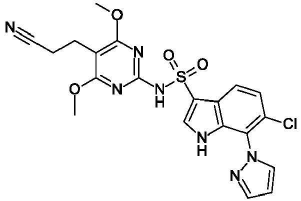 | 6-chloro-N-[5-(2-cyanoethyl)-4,6-dimethoxy-pyrimidin-2-yl]-7-pyrazol-1-yl-1H-indole-3-sulfonamide | A13 | B12 | 488.3 [M+H]⁺ |
| 71 | 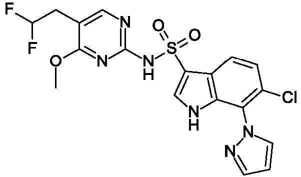 | 6-chloro-N-[5-(2,2-difluoroethyl)-4-methoxy-pyrimidin-2-yl]-7-pyrazol-1-yl-1H-indole-3-sulfonamide | A13 | B3 | 469.2 [M+H]⁺ |
| 72 | 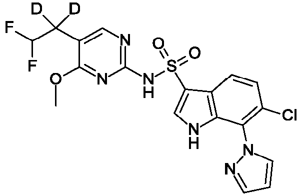 | 6-chloro-N-[5-(1,1-dideuterio-2,2-difluoroethyl)-4-methoxy-pyrimidin-2-yl]-7-pyrazol-1-yl-1H-indole-3-sulfonamide | A13 | B17 | 471.2 [M+H]⁺ |
| 73 | 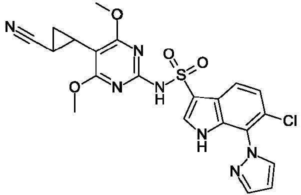 | 6-chloro-N-[5-(2-cyanocyclopropyl)-4,6-dimethoxy-pyrimidin-2-yl]-7-pyrazol-1-yl-1H-indole-3-sulfonamide | A13 | B9 | 500.3 [M+H]⁺ |
| 74 | 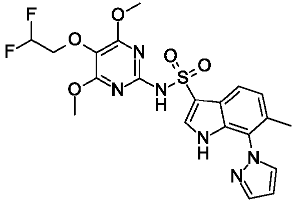 | N-[5-(2,2-difluoroethoxy)-4,6-dimethoxy-pyrimidin-2-yl]-6-methyl-7-pyrazol-1-yl-1H-indole-3-sulfonamide | A15 | B4 | 495.3 [M+H]⁺ |
| 75 | 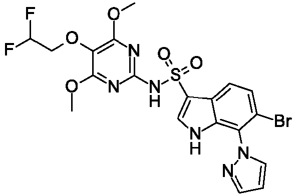 | 6-bromo-N-[5-(2,2-difluoroethoxy)-4,6-dimethoxy-pyrimidin-2-yl]-7-pyrazol-1-yl-1H-indole-3-sulfonamide | A14 | B4 | 559.2 [M+H]⁺ |
| 76 | 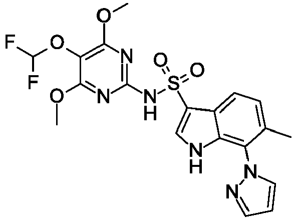 | N-[5-(difluoromethoxy)-4,6-dimethoxy-pyrimidin-2-yl]-6-methyl-7-pyrazol-1-yl-1H-indole-3-sulfonamide | A15 | B6 | 481.3 [M+H]⁺ |
| 77 | 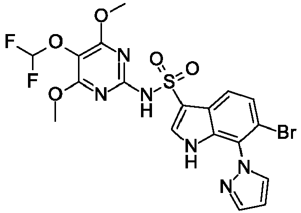 | 6-bromo-N-[5-(difluoromethoxy)-4,6-dimethoxy-pyrimidin-2-yl]-7-pyrazol-1-yl-1H-indole-3-sulfonamide | A14 | B6 | 545.2 [M+H]⁺ |
| 78 | 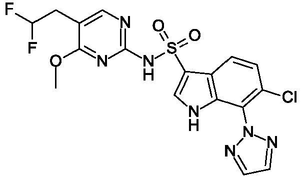 | 6-chloro-N-[5-(2,2-difluoroethyl)-4-methoxy-pyrimidin-2-yl]-7-(triazol-2-yl)-1H-indole-3-sulfonamide | A2 | B3 | 468.1 [M-H] |
| 79 | 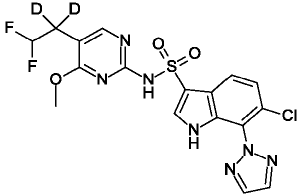 | 6-chloro-N-[5-(1,1-dideuterio-2,2-difluoroethyl)-4-methoxy-pyrimidin-2-yl]-7-(triazol-2-yl)-1H-indole-3-sulfonamide | A2 | B17 | 470.1 [M-H] |
| 80 | 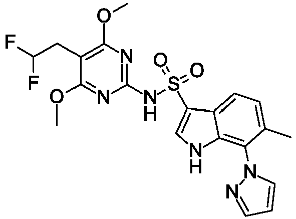 | N-[5-(2,2-difluoroethyl)-4,6-dimethoxy-pyrimidin-2-yl]-6-methyl-7-pyrazol-1-yl-1H-indole-3-sulfonamide | A15 | B1 | 479.3 [M+H]⁺ |
| 81 | 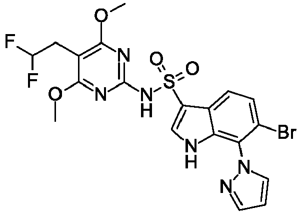 | 6-bromo-N-[5-(2,2-difluoroethyl)-4,6-dimethoxy-pyrimidin-2-yl]-7-pyrazol-1-yl-1H-indole-3-sulfonamide | A14 | B1 | 543.1 [M+H]⁺ |
| 82 | 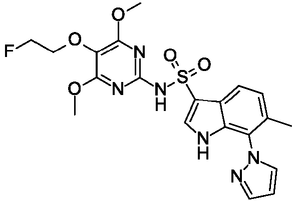 | N-[5-(2-fluoroethoxy)-4,6-dimethoxy-pyrimidin-2-yl]-6-methyl-7-pyrazol-1-yl-1H-indole-3-sulfonamide | A15 | B5 | 477.3 [M+H]⁺ |
| 83 | 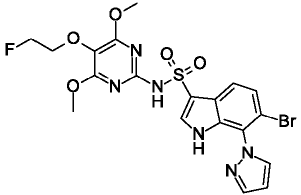 | 6-bromo-N-[5-(2-fluoroethoxy)-4,6-dimethoxy-pyrimidin-2-yl]-7-pyrazol-1-yl-1H-indole-3-sulfonamide | A14 | B5 | 541.1 [M+H]⁺ |
| 84 | 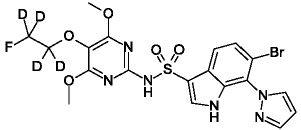 | 6-bromo-N-[4,6-dimethoxy-5-(1,1,2,2-tetradeuterio-2-fluoro-ethoxy)pyrimidin-2-yl]-7-pyrazol-1-yl-1H-indole-3-sulfonamide | A14 | B16 | 547.1 [M+H]⁺ |
| 85 | 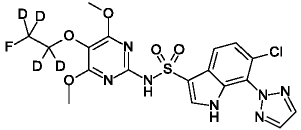 | 6-chloro-N-[4,6-dimethoxy-5-(1,1,2,2-tetradeuterio-2-fluoro-ethoxy)pyrimidin-2-yl]-7-(triazol-2-yl)-1H-indole-3-sulfonamide | A2 | B16 | 502.2 [M+H]⁺ |
| 86 | 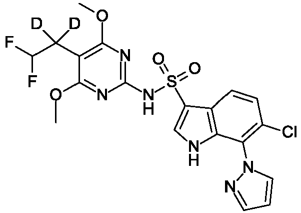 | 6-chloro-N-[5-(1,1-dideuterio-2,2-difluoroethyl)-4,6-dimethoxy-pyrimidin-2-yl]-7-pyrazol-1-yl-1H-indole-3-sulfonamide | A13 | B18 | 501.2 [M+H]⁺ |

### Example A

A compound of formula I can be used in a manner known per se as the active ingredient for the production of tablets of the following composition:

### Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example B

A compound of formula I can be used in a manner known per se as the active ingredient for the production of capsules of the following composition:

| | |
|---|---|
| | Per capsule |
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

## Claims

1. Compounds of formula I 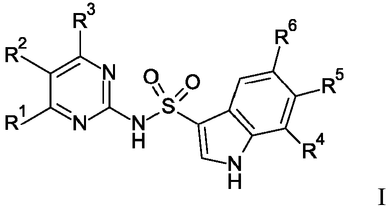 wherein
R¹ is alkoxy or haloalkoxy;
R² is halo, alkyl, alkoxy, haloalkyl, haloalkoxy, cyanoalkyl, cyanoalkoxy, or cyclopropyl optionally substituted with up to two substituents independently selected from cyano and halo;
R³ is H, alkoxy, or haloalkoxy;
R⁵ is H, halo, alkyl, or haloalkyl;
R⁶ is H or halo;
R⁴ is an N-linked heteroaryl or substituted N-linked heteroaryl, represented by 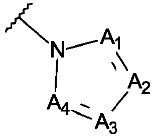
A₁ is CR^{y1} or N;
A₂ is CR^{y2} or N;
A₃ is CR^{y3} or N;
A₄ is CR^{y4} or N;
wherein R^{y1}, R^{y2}, R^{y3} and R^{y4} are independently selected from H, alkyl, halo and haloalkyl;
and pharmaceutically acceptable salts.

2. A compound according to claim 1, wherein R² is halo, alkyl, haloalkyl, haloalkoxy, cyanoalkyl, cyanoalkoxy, or cyclopropyl substituted with cyano.

3. A compound according to any of claims 1 to 2, wherein R³ is H or alkoxy.

4. A compound according to any of claims 1 to 3, wherein wherein at least one, but no more than two, of A₁, A₂, A₃ and A₄ are N, and R^{y1}, R^{y2}, R^{y3} and R^{y4} are independently selected from H or alkyl.

5. A compound according to claim 1, wherein
R¹ is alkoxy;
R² is halo, alkyl, haloalkyl, haloalkoxy, cyanoalkyl, cyanoalkoxy, or cyclopropyl substituted with cyano;
R³ is H or alkoxy;
R⁵ is H, halo, alkyl, or haloalkyl;
R⁶ is H or halo;
R⁴ is an N-linked heteroaryl or substituted N-linked heteroaryl, represented by 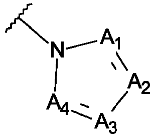
A₁ is CR^{y1} or N;
A₂ is CR^{y2} or N;
A₃ is CR^{y3} or N;
A₄ is CR^{y4} or N;
wherein R^{y1}, R^{y2}, R^{y3} and R^{y4} are independently selected from H, alkyl, and halo;
and pharmaceutically acceptable salts.

6. A compound according to claim 1, wherein
R¹ is alkoxy;
R² is haloalkyl, haloalkoxy, or cyclopropyl optionally substituted with cyano;
R³ is H or alkoxy;
R⁵ is H, halo, alkyl, or haloalkyl;
R⁶ is H;
R⁴ is selected from 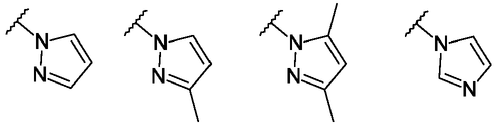 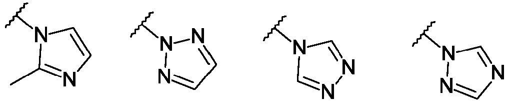 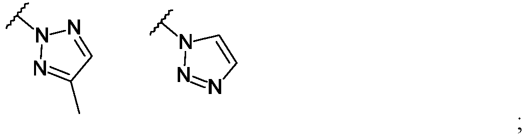
and pharmaceutically acceptable salts.

7. A compound according to any of claims 1 to 6, selected from
N-[5-(2,2-difluoroethyl)-4,6-dimethoxy-pyrimidin-2-yl]-7-(3-methylpyrazol-1-yl)-1H-indole-3-sulfonamide;
N-[5-(2,2-difluoroethyl)-4,6-dimethoxy-pyrimidin-2-yl]-7-(3,5-dimethylpyrazol-1-yl)-1H-indole-3-sulfonamide;
N-[5-(2,2-difluoroethyl)-4,6-dimethoxy-pyrimidin-2-yl]-7-imidazol-1-yl-1H-indole-3-sulfonamide;
6-chloro-N-[5-(2,2-difluoroethyl)-4,6-dimethoxy-pyrimidin-2-yl]-7-pyrazol-1-yl-1H-indole-3-sulfonamide;
N-[5-(2,2-difluoroethyl)-4,6-dimethoxy-pyrimidin-2-yl]-7-(2-methylimidazol-1-yl)-1H-indole-3-sulfonamide;
6-chloro-N-[5-(2,2-difluoroethoxy)-4,6-dimethoxy-pyrimidin-2-yl]-7-imidazol-1-yl-1H-indole-3-sulfonamide;
6-chloro-N-[5-(2,2-difluoroethyl)-4,6-dimethoxy-pyrimidin-2-yl]-7-(triazol-2-yl)-1H-indole-3-sulfonamide;
6-chloro-N-[5-(2,2-difluoroethyl)-4-methoxy-pyrimidin-2-yl]-7-imidazol-1-yl-1H-indole-3-sulfonamide;
6-chloro-N-[5-(2,2-difluoroethoxy)-4,6-dimethoxy-pyrimidin-2-yl]-7-(triazol-2-yl)-1H-indole-3-sulfonamide;
6-chloro-N-[5-(2,2-difluoroethyl)-4,6-dimethoxy-pyrimidin-2-yl]-7-(1,2,4-triazol-4-yl)-1H-indole-3-sulfonamide;
N-[5-(2,2-difluoroethoxy)-4,6-dimethoxy-pyrimidin-2-yl]-6-(difluoromethyl)-7-(triazol-2-yl)-1H-indole-3-sulfonamide;
6-chloro-N-[5-(2-fluoroethoxy)-4-methoxy-pyrimidin-2-yl]-7-(triazol-2-yl)-1H-indole-3-sulfonamide;
N-[5-(2,2-difluoroethyl)-4,6-dimethoxy-pyrimidin-2-yl]-7-(triazol-2-yl)-1H-indole-3-sulfonamide;
6-chloro-N-[5-(2-fluoroethoxy)-4,6-dimethoxy-pyrimidin-2-yl]-7-(triazol-2-yl)-1H-indole-3-sulfonamide;
6-chloro-N-[5-(difluoromethoxy)-4,6-dimethoxy-pyrimidin-2-yl]-7-(triazol-2-yl)-1H-indole-3-sulfonamide;
6-chloro-N-[5-(2,3-difluoropropyl)-4,6-dimethoxy-pyrimidin-2-yl]-7-(triazol-2-yl)-1H-indole-3-sulfonamide;
6-chloro-N-[5-(3,3-difluoropropyl)-4,6-dimethoxy-pyrimidin-2-yl]-7-(triazol-2-yl)-1H-indole-3-sulfonamide;
6-chloro-N-[5-(2-cyanocyclopropyl)-4,6-dimethoxy-pyrimidin-2-yl]-7-(triazol-2-yl)-1H-indole-3-sulfonamide;
N-[5-(2-fluoroethoxy)-4,6-dimethoxy-pyrimidin-2-yl]-7-(triazol-2-yl)-1H-indole-3-sulfonamide;
N-[5-(2,2-difluoroethoxy)-4,6-dimethoxy-pyrimidin-2-yl]-7-(triazol-2-yl)-1H-indole-3-sulfonamide;
N-[5-(2-cyanocyclopropyl)-4,6-dimethoxy-pyrimidin-2-yl]-7-(triazol-2-yl)-1H-indole-3-sulfonamide;
N-[5-(2,2-difluoroethyl)-4,6-dimethoxy-pyrimidin-2-yl]-6-methyl-7-(triazol-2-yl)-1H-indole-3-sulfonamide;
N-[5-(2-fluoroethoxy)-4,6-dimethoxy-pyrimidin-2-yl]-6-methyl-7-(triazol-2-yl)-1H-indole-3-sulfonamide;
N-[5-(difluoromethoxy)-4,6-dimethoxy-pyrimidin-2-yl]-6-methyl-7-(triazol-2-yl)-1H-indole-3-sulfonamide;
N-[5-(2,2-difluoroethoxy)-4,6-dimethoxy-pyrimidin-2-yl]-6-methyl-7-(triazol-2-yl)-1H-indole-3-sulfonamide;
N-[5-(2-cyanocyclopropyl)-4,6-dimethoxy-pyrimidin-2-yl]-6-methyl-7-(triazol-2-yl)-1H-indole-3-sulfonamide;
6-bromo-N-[5-(2,2-difluoroethyl)-4,6-dimethoxy-pyrimidin-2-yl]-7-(triazol-2-yl)-1H-indole-3-sulfonamide;
6-bromo-N-[5-(2-fluoroethoxy)-4,6-dimethoxy-pyrimidin-2-yl]-7-(triazol-2-yl)-1H-indole-3-sulfonamide;
6-bromo-N-[5-(difluoromethoxy)-4,6-dimethoxy-pyrimidin-2-yl]-7-(triazol-2-yl)-1H-indole-3-sulfonamide;
6-bromo-N-[5-(2,2-difluoroethoxy)-4,6-dimethoxy-pyrimidin-2-yl]-7-(triazol-2-yl)-1H-indole-3-sulfonamide;
6-bromo-N-[5-(2-cyanocyclopropyl)-4,6-dimethoxy-pyrimidin-2-yl]-7-(triazol-2-yl)-1H-indole-3-sulfonamide;
N-[5-(2,2-difluoroethyl)-4,6-dimethoxy-pyrimidin-2-yl]-6-fluoro-7-(triazol-2-yl)-1H-indole-3-sulfonamide;
6-fluoro-N-[5-(2-fluoroethoxy)-4,6-dimethoxy-pyrimidin-2-yl]-7-(triazol-2-yl)-1H-indole-3-sulfonamide;
N-[5-(difluoromethoxy)-4,6-dimethoxy-pyrimidin-2-yl]-6-fluoro-7-(triazol-2-yl)-1H-indole-3-sulfonamide;
N-[5-(2,2-difluoroethoxy)-4,6-dimethoxy-pyrimidin-2-yl]-6-fluoro-7-(triazol-2-yl)-1H-indole-3-sulfonamide;
N-[5-(2-cyanocyclopropyl)-4,6-dimethoxy-pyrimidin-2-yl]-6-fluoro-7-(triazol-2-yl)-1H-indole-3-sulfonamide;
6-chloro-N-[5-(2,2-difluoroethyl)-4,6-dimethoxy-pyrimidin-2-yl]-7-(1,2,4-triazol-1-yl)-1H-indole-3-sulfonamide;
6-chloro-N-[5-(2,2-difluoroethoxy)-4,6-dimethoxy-pyrimidin-2-yl]-7-(4-methyltriazol-2-yl)-1H-indole-3-sulfonamide;
6-chloro-N-[5-(2-fluoroethoxy)-4,6-dimethoxy-pyrimidin-2-yl]-7-(4-methyltriazol-2-yl)-1H-indole-3-sulfonamide;
6-chloro-N-[5-(2,2-difluoroethyl)-4,6-dimethoxy-pyrimidin-2-yl]-7-(triazol-1-yl)-1H-indole-3-sulfonamide;
6-(difluoromethyl)-N-[5-(2-fluoroethoxy)-4,6-dimethoxy-pyrimidin-2-yl]-7-(triazol-2-yl)-1H-indole-3-sulfonamide;
N-[5-(2,2-difluoroethyl)-4,6-dimethoxy-pyrimidin-2-yl]-6-(difluoromethyl)-7-(triazol-2-yl)-1H-indole-3-sulfonamide; and
pharmaceutically acceptable salts thereof.

8. A compound according to any one of claims 1 to 6, selected from
6-chloro-N-[5-(2,2-difluoroethoxy)-4,6-dimethoxy-pyrimidin-2-yl]-7-(4-methylpyrazol-1-yl)-1H-indole-3-sulfonamide;
6-chloro-N-[5-(2,2-difluoroethoxy)-4,6-dimethoxy-pyrimidin-2-yl]-7-(3-methylpyrazol-1-yl)-1H-indole-3-sulfonamide;
6-chloro-N-[5-(2,2-difluoroethoxy)-4,6-dimethoxy-pyrimidin-2-yl]-7-(4-fluoropyrazol-1-yl)-1H-indole-3-sulfonamide;
6-chloro-N-[5-(2,2-difluoroethoxy)-4,6-dimethoxy-pyrimidin-2-yl]-7-(5-methylpyrazol-1-yl)-1H-indole-3-sulfonamide;
6-chloro-N-[5-(2-fluoroethoxy)-4,6-dimethoxy-pyrimidin-2-yl]-7-(4-fluoropyrazol-1-yl)-1H-indole-3-sulfonamide;
6-chloro-N-[5-(difluoromethoxy)-4,6-dimethoxy-pyrimidin-2-yl]-7-(4-fluoropyrazol-1-yl)-1H-indole-3-sulfonamide;
N-[5-(difluoromethoxy)-4,6-dimethoxy-pyrimidin-2-yl]-6-(difluoromethyl)-7-pyrazol-1-yl-1H-indole-3-sulfonamide;
N-[5-(2,2-difluoroethoxy)-4,6-dimethoxy-pyrimidin-2-yl]-6-(difluoromethyl)-7-pyrazol-1-yl-1H-indole-3-sulfonamide;
6-(difluoromethyl)-N-[5-(2-fluoroethoxy)-4,6-dimethoxy-pyrimidin-2-yl]-7-pyrazol-1-yl-1H-indole-3-sulfonamide;
N-[5-(2,2-difluoroethyl)-4,6-dimethoxy-pyrimidin-2-yl]-6-(difluoromethyl)-7-pyrazol-1-yl-1H-indole-3-sulfonamide;
6-chloro-N-[5-(2,2-difluoroethoxy)-4,6-dimethoxy-pyrimidin-2-yl]-7-(3-fluoropyrazol-1-yl)-1H-indole-3-sulfonamide;
6-chloro-N-[5-(difluoromethoxy)-4,6-dimethoxy-pyrimidin-2-yl]-7-(3-fluoropyrazol-1-yl)-1H-indole-3-sulfonamide;
6-chloro-N-[5-(2,2-difluoroethyl)-4,6-dimethoxy-pyrimidin-2-yl]-7-(3-fluoropyrazol-1-yl)-1H-indole-3-sulfonamide;
6-chloro-N-[5-(2-fluoroethoxy)-4,6-dimethoxy-pyrimidin-2-yl]-7-(3-fluoropyrazol-1-yl)-1H-indole-3-sulfonamide;
6-chloro-N-[4,6-dimethoxy-5-(1,1,2-trifluoroethoxy)pyrimidin-2-yl]-7-(triazol-2-yl)-1H-indole-3-sulfonamide;
6-chloro-N-[5-(2-fluoro-1,1-dimethyl-ethoxy)-4,6-dimethoxy-pyrimidin-2-yl]-7-(triazol-2-yl)-1H-indole-3-sulfonamide;
6-chloro-N-[5-(1,1-dideuterio-2,2-difluoro-ethoxy)-4,6-dimethoxy-pyrimidin-2-yl]-7-(triazol-2-yl)-1H-indole-3-sulfonamide;
6-chloro-N-[5-(cyanomethoxy)-4,6-dimethoxy-pyrimidin-2-yl]-7-(triazol-2-yl)-1H-indole-3-sulfonamide;
6-chloro-N-[5-(2-cyanoethyl)-4,6-dimethoxy-pyrimidin-2-yl]-7-(triazol-2-yl)-1H-indole-3-sulfonamide;
6-chloro-N-(4,6-dimethoxy-5-methyl-pyrimidin-2-yl)-7-(triazol-2-yl)-1H-indole-3-sulfonamide;
N-(5-bromo-4,6-dimethoxy-pyrimidin-2-yl)-6-chloro-7-(triazol-2-yl)-1H-indole-3-sulfonamide;
6-chloro-N-[5-(2,2-difluoroethoxy)-4,6-dimethoxy-pyrimidin-2-yl]-5-fluoro-7-(triazol-2-yl)-1H-indole-3-sulfonamide;
6-chloro-N-[5-(2,2-difluoroethoxy)-4,6-dimethoxy-pyrimidin-2-yl]-7-pyrazol-1-yl-1H-indole-3-sulfonamide;
6-chloro-N-[5-(difluoromethoxy)-4,6-dimethoxy-pyrimidin-2-yl]-7-pyrazol-1-yl-1H-indole-3-sulfonamide;
6-chloro-N-[5-(2-fluoroethoxy)-4,6-dimethoxy-pyrimidin-2-yl]-7-pyrazol-1-yl-1H-indole-3-sulfonamide;
6-chloro-N-[4,6-dimethoxy-5-(1,1,2,2-tetradeuterio-2-fluoro-ethoxy)pyrimidin-2-yl]-7-pyrazol-1-yl-1H-indole-3-sulfonamide;
6-chloro-N-[5-(cyanomethoxy)-4,6-dimethoxy-pyrimidin-2-yl]-7-pyrazol-1-yl-1H-indole-3-sulfonamide;
6-chloro-N-[5-(2-cyanoethyl)-4,6-dimethoxy-pyrimidin-2-yl]-7-pyrazol-1-yl-1H-indole-3-sulfonamide;
6-chloro-N-[5-(2,2-difluoroethyl)-4-methoxy-pyrimidin-2-yl]-7-pyrazol-1-yl-1H-indole-3-sulfonamide;
6-chloro-N-[5-(1,1-dideuterio-2,2-difluoro-ethyl)-4-methoxy-pyrimidin-2-yl]-7-pyrazol-1-yl-1H-indole-3-sulfonamide;
6-chloro-N-[5-(2-cyanocyclopropyl)-4,6-dimethoxy-pyrimidin-2-yl]-7-pyrazol-1-yl-1H-indole-3-sulfonamide;
N-[5-(2,2-difluoroethoxy)-4,6-dimethoxy-pyrimidin-2-yl]-6-methyl-7-pyrazol-1-yl-1H-indole-3-sulfonamide;
6-bromo-N-[5-(2,2-difluoroethoxy)-4,6-dimethoxy-pyrimidin-2-yl]-7-pyrazol-1-yl-1H-indole-3-sulfonamide;
N-[5-(difluoromethoxy)-4,6-dimethoxy-pyrimidin-2-yl]-6-methyl-7-pyrazol-1-yl-1H-indole-3-sulfonamide;
6-bromo-N-[5-(difluoromethoxy)-4,6-dimethoxy-pyrimidin-2-yl]-7-pyrazol-1-yl-1H-indole-3-sulfonamide;
6-chloro-N-[5-(2,2-difluoroethyl)-4-methoxy-pyrimidin-2-yl]-7-(triazol-2-yl)-1H-indole-3-sulfonamide;
6-chloro-N-[5-(1,1-dideuterio-2,2-difluoro-ethyl)-4-methoxy-pyrimidin-2-yl]-7-(triazol-2-yl)-1H-indole-3-sulfonamide;
N-[5-(2,2-difluoroethyl)-4,6-dimethoxy-pyrimidin-2-yl]-6-methyl-7-pyrazol-1-yl-1H-indole-3-sulfonamide;
6-bromo-N-[5-(2,2-difluoroethyl)-4,6-dimethoxy-pyrimidin-2-yl]-7-pyrazol-1-yl-1H-indole-3-sulfonamide;
N-[5-(2-fluoroethoxy)-4,6-dimethoxy-pyrimidin-2-yl]-6-methyl-7-pyrazol-1-yl-1H-indole-3-sulfonamide;
6-bromo-N-[5-(2-fluoroethoxy)-4,6-dimethoxy-pyrimidin-2-yl]-7-pyrazol-1-yl-1H-indole-3-sulfonamide;
6-bromo-N-[4,6-dimethoxy-5-(1,1,2,2-tetradeuterio-2-fluoro-ethoxy)pyrimidin-2-yl]-7-pyrazol-1-yl-1H-indole-3-sulfonamide;
6-chloro-N-[4,6-dimethoxy-5-(1,1,2,2-tetradeuterio-2-fluoro-ethoxy)pyrimidin-2-yl]-7-(triazol-2-yl)-1H-indole-3-sulfonamide;
6-chloro-N-[5-(1,1-dideuterio-2,2-difluoro-ethyl)-4,6-dimethoxy-pyrimidin-2-yl]-7-pyrazol-1-yl-1H-indole-3-sulfonamide;
and pharmaceutically acceptable salts thereof.

9. A compound according to any one of claims 1 to 6, selected from
6-chloro-N-[5-(2,2-difluoroethoxy)-4,6-dimethoxy-pyrimidin-2-yl]-7-(triazol-2-yl)-1H-indole-3-sulfonamide;
6-chloro-N-[5-(difluoromethoxy)-4,6-dimethoxy-pyrimidin-2-yl]-7-(triazol-2-yl)-1H-indole-3-sulfonamide;
and pharmaceutically acceptable salts thereof.

10. A process to prepare a compound according to any one of claims 1 to 9 comprising the reacting a compound of formula III with a compound of formula II in the presence of a base selected from N-ethyldiisopropylamine, pyridine, potassium phosphate or sodium hydride, to provide a compound of formula I, 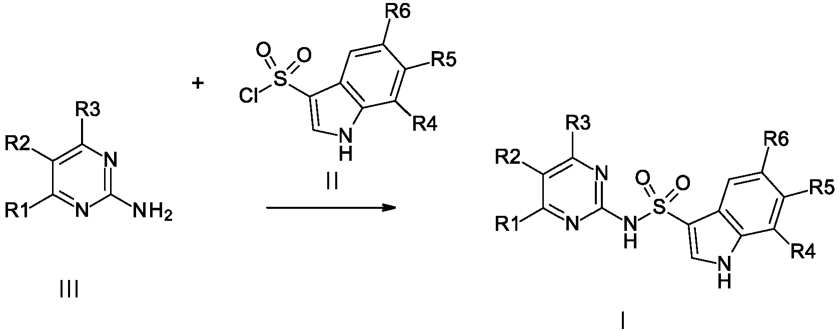 wherein R¹, R², R³, R³, R⁴, R⁵ and R⁶ are as described above.

11. A compound according to any one of claims 1 to 9 for use as therapeutically active substance.

12. A compound according to any one of claims 1 to 9 for use in the treatment of a disease modulated by GPR17.

13. A pharmaceutical composition comprising a compound according to any one of claims 1 to 9 and a therapeutically inert carrier.

14. A compound according to any one of claims 1 to 9 for use in the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and inflammation in the CNS for instance following encephalitis, primary angiitis, meningitis and obesity.

15. A compound according to any one of claims 1 to 9 for use in the treatment or prophylaxis of multiple sclerosis.

## Patentansprüche

1. Verbindungen der Formel I 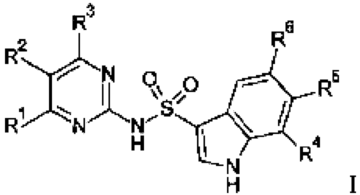 wobei
R¹ Alkoxy oder Halogenalkoxy ist;
R² Halogen, Alkyl, Alkoxy, Halogenalkyl, Halogenalkoxy, Cyanoalkyl, Cyanoalkoxy oder Cyclopropyl, gegebenenfalls substituiert mit bis zu zwei Substituenten, unabhängig ausgewählt aus Cyano und Halogen, ist;
R³ H, Alkoxy oder Halogenalkoxy ist;
R⁵ H, Halogen, Alkyl oder Halogenalkyl ist;
R⁶ H oder Halogen ist;
R⁴ ein N-verknüpftes Heteroaryl oder substituiertes N-verknüpftes Heteroaryl ist, dargestellt durch 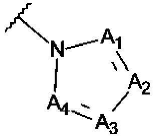
A₁ CR^{y1} oder N ist;
A₂ CR^{y2} oder N ist;
A₃ CR^{y3} oder N ist;
A₄ CR^{y4} oder N ist;
wobei R^{y1}, R^{y2}, R^{y3} und R^{y4} unabhängig voneinander ausgewählt sind aus H, Alkyl, Halogen und Halogenalkyl;
und pharmazeutisch verträgliche Salze.

2. Verbindung nach Anspruch 1, wobei R² Halogen, Alkyl, Halogenalkyl, Halogenalkoxy, Cyanoalkyl, Cyanoalkoxy oder Cyclopropyl, substituiert mit Cyano, ist.

3. Verbindung nach einem der Ansprüche 1 bis 2, wobei R³ H oder Alkoxy ist.

4. Verbindung nach einem der Ansprüche 1 bis 3, wobei mindestens eines, aber nicht mehr als zwei von A₁, A₂, A₃ und A₄ N sind und R^{y1}, R^{y2}, R^{y3} und R^{y4} unabhängig voneinander ausgewählt sind aus H oder Alkyl.

5. Verbindung nach Anspruch 1, wobei
R¹ Alkoxy ist;
R² Halogen, Alkyl, Halogenalkyl, Halogenalkoxy, Cyanoalkyl, Cyanoalkoxy oder Cyclopropyl, substituiert mit Cyano, ist;
R³ H oder Alkoxy ist;
R⁵ H, Halogen, Alkyl oder Halogenalkyl ist;
R⁶ H oder Halogen ist;
R⁴ ein N-verknüpftes Heteroaryl oder substituiertes N-verknüpftes Heteroaryl ist,
dargestellt durch 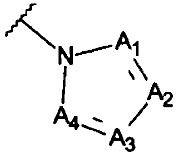
A₁ CR^{y1} oder N ist;
A₂ CR^{y2} oder N ist;
A₃ CR^{y3} oder N ist;
A₄ CR^{y4} oder N ist;
wobei R^{y1}, R^{y2}, R^{y3} und R^{y4} unabhängig voneinander ausgewählt sind aus H, Alkyl und Halogen;
und pharmazeutisch verträgliche Salze.

6. Verbindung nach Anspruch 1, wobei
R¹ Alkoxy ist;
R² Halogenalkyl, Halogenalkoxy oder Cyclopropyl, gegebenenfalls substituiert mit Cyano, ist;
R³ H oder Alkoxy ist;
R⁵ H, Halogen, Alkyl oder Halogenalkyl ist; R⁶ H ist;
R⁴ ausgewählt ist aus 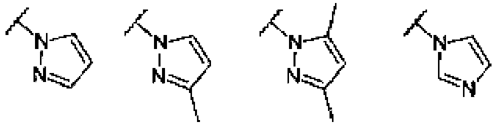 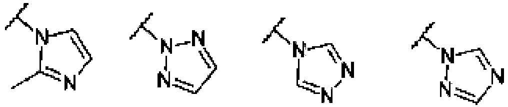 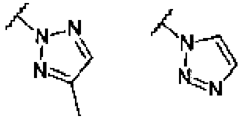
und pharmazeutisch verträgliche Salze.

7. Verbindung nach einem der Ansprüche 1 bis 6, ausgewählt aus
N-[5-(2,2-Difluorethyl)-4,6-dimethoxypyrimidin-2-yl]-7-(3-methylpyrazol-1-yl)-1H-indol-3-sulfonamid;
N-[5-(2,2-Difluorethyl)-4,6-dimethoxypyrimidin-2-yl]-7-(3,5-dimethylpyrazol-1-yl)-1H-indol-3-sulfonamid;
N-[5-(2,2-Difluorethyl)-4,6-dimethoxypyrimidin-2-yl]-7-imidazol-1-yl-1H-indol-3-sulfonamid;
6-Chlor-N-[5-(2,2-difluorethyl)-4,6-dimethoxypyrimidin-2-yl]-7-pyrazol-1-yl-1H-indol-3-sulfonamid;
N-[5-(2,2-Difluorethyl)-4,6-dimethoxypyrimidin-2-yl]-7-(2-methylimidazol-1-yl)-1H-indol-3-sulfonamid;
6-Chlor-N-[5-(2,2-difluorethoxy)-4,6-dimethoxypyrimidin-2-yl]-7-imidazol-1-yl-1H-indol-3-sulfonamid;
6-Chlor-N-[5-(2,2-difluorethyl)-4,6-dimethoxypyrimidin-2-yl]-7-(triazol-2-yl)-1H-indol-3-sulfonamid;
6-Chlor-N-[5-(2,2-difluorethyl)-4-methoxypyrimidin-2-yl]-7-imidazol-1-yl-1H-indol-3-sulfonamid;
6-Chlor-N-[5-(2,2-difluorethoxy)-4,6-dimethoxypyrimidin-2-yl]-7-(triazol-2-yl)-1H-indol-3-sulfonamid;
6-Chlor-N-[5-(2,2-difluorethyl)-4,6-dimethoxypyrimidin-2-yl]-7-(1,2,4-triazol-4-yl)-1H-indol-3-sulfonamid;
N-[5-(2,2-Difluorethoxy)-4,6-dimethoxypyrimidin-2-yl]-6-(difluormethyl)-7-(triazol-2-yl)-1H-indol-3-sulfonamid;
6-Chlor-N-[5-(2-fluorethoxy)-4-methoxypyrimidin-2-yl]-7-(triazol-2-yl)-1H-indol-3-sulfonamid;
N-[5-(2,2-Difluorethyl)-4,6-dimethoxypyrimidin-2-yl]-7-(triazol-2-yl)-1H-indol-3-sulfonamid;
6-Chlor-N-[5-(2-fluorethoxy)-4,6-dimethoxypyrimidin-2-yl]-7-(triazol-2-yl)-1H-indol-3-sulfonamid;
6-Chlor-N-[5-(difluormethoxy)-4,6-dimethoxypyrimidin-2-yl]-7-(triazol-2-yl)-1H-indol-3-sulfonamid;
6-Chlor-N-[5-(2,3-difluorpropyl)-4,6-dimethoxypyrimidin-2-yl]-7-(triazol-2-yl)-1H-indol-3-sulfonamid;
6-Chlor-N-[5-(3,3-difluorpropyl)-4,6-dimethoxypyrimidin-2-yl]-7-(triazol-2-yl)-1H-indol-3-sulfonamid;
6-Chlor-N-[5-(2-cyanocyclopropyl)-4,6-dimethoxypyrimidin-2-yl]-7-(triazol-2-yl)-1H-indol-3-sulfonamid;
N-[5-(2-Fluorethoxy)-4,6-dimethoxypyrimidin-2-yl]-7-(triazol-2-yl)-1H-indol-3-sulfonamid;
N-[5-(2,2-Difluorethoxy)-4,6-dimethoxypyrimidin-2-yl]-7-(triazol-2-yl)-1H-indol-3-sulfonamid;
N-[5-(2-Cyanocyclopropyl)-4,6-dimethoxypyrimidin-2-yl]-7-(triazol-2-yl)-1H-indol-3-sulfonamid;
N-[5-(2,2-Difluorethyl)-4,6-dimethoxypyrimidin-2-yl]-6-methyl-7-(triazol-2-yl)-1H-indol-3-sulfonamid;
N-[5-(2-Fluorethoxy)-4,6-dimethoxypyrimidin-2-yl]-6-methyl-7-(triazol-2-yl)-1H-indol-3-sulfonamid;
N-[5-(Difluormethoxy)-4,6-dimethoxypyrimidin-2-yl]-6-methyl-7-(triazol-2-yl)-1H-indol-3-sulfonamid;
N-[5-(2,2-Difluorethoxy)-4,6-dimethoxypyrimidin-2-yl]-6-methyl-7-(triazol-2-yl)-1H-indol-3-sulfonamid;
N-[5-(2-Cyanocyclopropyl)-4,6-dimethoxypyrimidin-2-yl]-6-methyl-7-(triazol-2-yl)-1H-indol-3-sulfonamid;
6-Brom-N-[5-(2,2-difluorethyl)-4,6-dimethoxypyrimidin-2-yl]-7-(triazol-2-yl)-1H-indol-3-sulfonamid;
6-Brom-N-[5-(2-fluorethoxy)-4,6-dimethoxypyrimidin-2-yl]-7-(triazol-2-yl)-1H-indol-3-sulfonamid;
6-Brom-N-[5-(difluormethoxy)-4,6-dimethoxypyrimidin-2-yl]-7-(triazol-2-yl)-1H-indol-3-sulfonamid;
6-Brom-N-[5-(2,2-difluorethoxy)-4,6-dimethoxypyrimidin-2-yl]-7-(triazol-2-yl)-1H-indol-3-sulfonamid;
6-Brom-N-[5-(2-cyanocyclopropyl)-4,6-dimethoxypyrimidin-2-yl]-7-(triazol-2-yl)-1H-indol-3-sulfonamid;
N-[5-(2,2-Difluorethyl)-4,6-dimethoxypyrimidin-2-yl]-6-fluor-7-(triazol-2-yl)-1H-indol-3-sulfonamid;
6-Fluor-N-[5-(2-fluorethoxy)-4,6-dimethoxypyrimidin-2-yl]-7-(triazol-2-yl)-1H-indol-3-sulfonamid;
N-[5-(Difluormethoxy)-4,6-dimethoxypyrimidin-2-yl]-6-fluor-7-(triazol-2-yl)-1H-indol-3-sulfonamid;
N-[5-(2,2-Difluorethoxy)-4,6-dimethoxypyrimidin-2-yl]-6-fluor-7-(triazol-2-yl)-1H-indol-3-sulfonamid;
N-[5-(2-Cyanocyclopropyl)-4,6-dimethoxypyrimidin-2-yl]-6-fluor-7-(triazol-2-yl)-1H-indol-3-sulfonamid;
6-Chlor-N-[5-(2,2-difluorethyl)-4,6-dimethoxypyrimidin-2-yl]-7-(1,2,4-triazol-1-yl)-1H-indol-3-sulfonamid;
6-Chlor-N-[5-(2,2-difluorethoxy)-4,6-dimethoxypyrimidin-2-yl]-7-(4-methyltriazol-2-yl)-1H-indol-3-sulfonamid;
6-Chlor-N-[5-(2-fluorethoxy)-4,6-dimethoxypyrimidin-2-yl]-7-(4-methyltriazol-2-yl)-1H-indol-3-sulfonamid;
6-Chlor-N-[5-(2,2-difluorethyl)-4,6-dimethoxypyrimidin-2-yl]-7-(triazol-1-yl)-1H-indol-3-sulfonamid;
6-(Difluormethyl)-N-[5-(2-fluorethoxy)-4,6-dimethoxypyrimidin-2-yl]-7-(triazol-2-yl)-1H-indol-3-sulfonamid;
N-[5-(2,2-Difluorethyl)-4,6-dimethoxypyrimidin-2-yl]-6-(difluormethyl)-7-(triazol-2-yl)-1H-indol-3-sulfonamid; und
pharmazeutisch verträgliche Salze davon.

8. Verbindung nach einem der Ansprüche 1 bis 6, ausgewählt aus
6-Chlor-N-[5-(2,2-difluorethoxy)-4,6-dimethoxypyrimidin-2-yl]-7-(4-methylpyrazol-1-yl)-1H-indol-3-sulfonamid;
6-Chlor-N-[5-(2,2-difluorethoxy)-4,6-dimethoxypyrimidin-2-yl]-7-(3-methylpyrazol-1-yl)-1H-indol-3-sulfonamid;
6-Chlor-N-[5-(2,2-difluorethoxy)-4,6-dimethoxypyrimidin-2-yl]-7-(4-fluorpyrazol-1-yl)-1H-indol-3-sulfonamid;
6-Chlor-N-[5-(2,2-difluorethoxy)-4,6-dimethoxypyrimidin-2-yl]-7-(5-methylpyrazol-1-yl)-1H-indol-3-sulfonamid;
6-Chlor-N-[5-(2-fluorethoxy)-4,6-dimethoxypyrimidin-2-yl]-7-(4-fluorpyrazol-1-yl)-1H-indol-3-sulfonamid;
6-Chlor-N-[5-(difluormethoxy)-4,6-dimethoxypyrimidin-2-yl]-7-(4-fluorpyrazol-1-yl)-1H-indol-3-sulfonamid;
N-[5-(Difluormethoxy)-4,6-dimethoxypyrimidin-2-yl]-6-(difluormethyl)-7-pyrazol-1-yl-1H-indol-3-sulfonamid;
N-[5-(2,2-Difluorethoxy)-4,6-dimethoxypyrimidin-2-yl]-6-(difluormethyl)-7-pyrazol-1-yl-1H-indol-3-sulfonamid;
6-(Difluormethyl)-N-[5-(2-fluorethoxy)-4,6-dimethoxypyrimidin-2-yl]-7-pyrazol-1-yl-1H-indol-3-sulfonamid;
N-[5-(2,2-Difluorethyl)-4,6-dimethoxypyrimidin-2-yl]-6-(difluormethyl)-7-pyrazol-1-yl-1H-indol-3-sulfonamid;
6-Chlor-N-[5-(2,2-difluorethoxy)-4,6-dimethoxypyrimidin-2-yl]-7-(3-fluorpyrazol-1-yl)-1H-indol-3-sulfonamid;
6-Chlor-N-[5-(difluormethoxy)-4,6-dimethoxypyrimidin-2-yl]-7-(3-fluorpyrazol-1-yl)-1H-indol-3-sulfonamid;
6-Chlor-N-[5-(2,2-difluorethyl)-4,6-dimethoxypyrimidin-2-yl]-7-(3-fluorpyrazol-1-yl)-1H-indol-3-sulfonamid;
6-Chlor-N-[5-(2-fluorethoxy)-4,6-dimethoxypyrimidin-2-yl]-7-(3-fluorpyrazol-1-yl)-1H-indol-3-sulfonamid;
6-Chlor-N-[4,6-dimethoxy-5-(1,1,2-trifluorethoxy)pyrimidin-2-yl]-7-(triazol-2-yl)-1H-indol-3-sulfonamid;
6-Chlor-N-[5-(2-fluor-1,1-dimethylethoxy)-4,6-dimethoxypyrimidin-2-yl]-7-(triazol-2-yl)-1H-indol-3-sulfonamid;
6-Chlor-N-[5-(1,l-dideuterio-2,2-difluorethoxy)-4,6-dimethoxypyrimidin-2-yl]-7-(triazol-2-yl)-1H-indol-3-sulfonamid;
6-Chlor-N-[5-(cyanomethoxy)-4,6-dimethoxypyrimidin-2-yl]-7-(triazol-2-yl)-1H-indol-3-sulfonamid;
6-Chlor-N-[5-(2-cyanoethyl)-4,6-dimethoxypyrimidin-2-yl]-7-(triazol-2-yl)-1H-indol-3-sulfonamid;
6-Chlor-N-(4,6-dimethoxy-5-methylpyrimidin-2-yl)-7-(triazol-2-yl)-1H-indol-3-sulfonamid;
N-(5-Brom-4,6-dimethoxypyrimidin-2-yl)-6-chlor-7-(triazol-2-yl)-1H-indol-3-sulfonamid;
6-Chlor-N-[5-(2,2-difluorethoxy)-4,6-dimethoxypyrimidin-2-yl]-5-fluor-7-(triazol-2-yl)-1H-indol-3-sulfonamid;
6-Chlor-N-[5-(2,2-difluorethoxy)-4,6-dimethoxypyrimidin-2-yl]-7-pyrazol-1-yl-1H-indol-3-sulfonamid;
6-Chlor-N-[5-(difluormethoxy)-4,6-dimethoxypyrimidin-2-yl]-7-pyrazol-1-yl-1H-indol-3-sulfonamid;
6-Chlor-N-[5-(2-fluorethoxy)-4,6-dimethoxypyrimidin-2-yl]-7-pyrazol-1-yl-1H-indol-3-sulfonamid;
6-Chlor-N-[4,6-dimethoxy-5-(1,1,2,2-tetradeuterio-2-fluorethoxy)pyrimidin-2-yl]-7-pyrazol-1-yl-1H-indol-3-sulfonamid;
6-Chlor-N-[5-(cyanomethoxy)-4,6-dimethoxypyrimidin-2-yl]-7-pyrazol-1-yl-1H-indol-3-sulfonamid;
6-Chlor-N-[5-(2-cyanoethyl)-4,6-dimethoxypyrimidin-2-yl]-7-pyrazol-1-yl-1H-indol-3-sulfonamid;
6-Chlor-N-[5-(2,2-difluorethyl)-4-methoxypyrimidin-2-yl]-7-pyrazol-1-yl-1H-indol-3-sulfonamid;
6-Chlor-N-[5-(1,l-dideuterio-2,2-difluorethyl)-4-methoxypyrimidin-2-yl]-7-pyrazol-1-yl-1H-indol-3-sulfonamid;
6-Chlor-N-[5-(2-cyanocyclopropyl)-4,6-dimethoxypyrimidin-2-yl]-7-pyrazol-1-yl-1H-indol-3-sulfonamid;
N-[5-(2,2-Difluorethoxy)-4,6-dimethoxypyrimidin-2-yl]-6-methyl-7-pyrazol-1-yl-1H-indol-3-sulfonamid;
6-Brom-N-[5-(2,2-difluorethoxy)-4,6-dimethoxypyrimidin-2-yl]-7-pyrazol-1-yl-1H-indol-3-sulfonamid;
N-[5-(Difluormethoxy)-4,6-dimethoxypyrimidin-2-yl]-6-methyl-7-pyrazol-1-yl-1H-indol-3-sulfonamid;
6-Brom-N-[5-(difluormethoxy)-4,6-dimethoxypyrimidin-2-yl]-7-pyrazol-1-yl-1H-indol-3-sulfonamid;
6-Chlor-N-[5-(2,2-difluorethyl)-4-methoxypyrimidin-2-yl]-7-(triazol-2-yl)-1H-indol-3-sulfonamid;
6-Chlor-N-[5-(1,l-dideuterio-2,2-difluorethyl)-4-methoxypyrimidin-2-yl]-7-(triazol-2-yl)-1H-indol-3-sulfonamid;
N-[5-(2,2-Difluorethyl)-4,6-dimethoxypyrimidin-2-yl]-6-methyl-7-pyrazol-1-yl-1H-indol-3-sulfonamid;
6-Brom-N-[5-(2,2-difluorethyl)-4,6-dimethoxypyrimidin-2-yl]-7-pyrazol-1-yl-1H-indol-3-sulfonamid;
N-[5-(2-Fluorethoxy)-4,6-dimethoxypyrimidin-2-yl]-6-methyl-7-pyrazol-1-yl-1H-indol-3-sulfonamid;
6-Brom-N-[5-(2-fluorethoxy)-4,6-dimethoxypyrimidin-2-yl]-7-pyrazol-1-yl-1H-indol-3-sulfonamid;
6-Brom-N-[4,6-dimethoxy-5-(1,1,2,2-tetradeuterio-2-fluorethoxy)pyrimidin-2-yl]-7-pyrazol-1-yl-1H-indol-3-sulfonamid;
6-Chlor-N-[4,6-dimethoxy-5-(1,1,2,2-tetradeuterio-2-fluorethoxy)pyrimidin-2-yl]-7-(triazol-2-yl)-1H-indol-3-sulfonamid;
6-Chlor-N-[5-(1,l-dideuterio-2,2-difluorethyl)-4,6-dimethoxypyrimidin-2-yl]-7-pyrazol-1-yl-1H-indol-3-sulfonamid;
und pharmazeutisch verträgliche Salze davon.

9. Verbindung nach einem der Ansprüche 1 bis 6, ausgewählt aus
6-Chlor-N-[5-(2,2-difluorethoxy)-4,6-dimethoxypyrimidin-2-yl]-7-(triazol-2-yl)-1H-indol-3-sulfonamid;
6-Chlor-N-[5-(difluormethoxy)-4,6-dimethoxypyrimidin-2-yl]-7-(triazol-2-yl)-1H-indol-3-sulfonamid;
und pharmazeutisch verträgliche Salze davon.

10. Verfahren zur Herstellung einer Verbindung nach einem der Ansprüche 1 bis 9, umfassend das Umsetzen einer Verbindung der Formel III mit einer Verbindung der Formel II in Gegenwart einer Base, ausgewählt aus N-Ethyldiisopropylamin, Pyridin, Kaliumphosphat oder Natriumhydrid, um eine Verbindung der Formel I bereitzustellen, 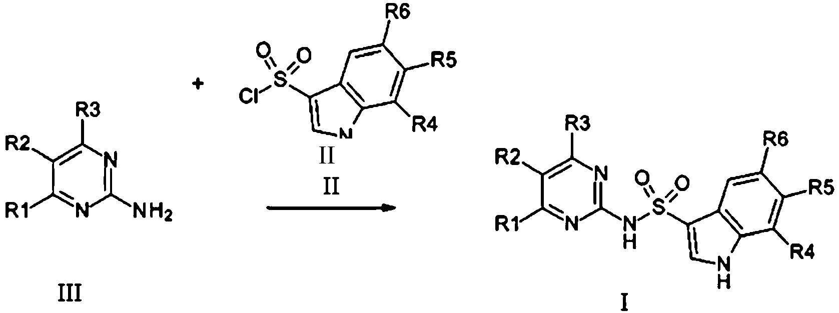 wobei R¹, R², R³, R³, R⁴, R⁵ und R⁶ wie oben beschrieben sind.

11. Verbindung nach einem der Ansprüche 1 bis 9 zur Verwendung als therapeutisch wirksame Substanz.

12. Verbindung nach einem der Ansprüche 1 bis 9 zur Verwendung bei der Behandlung einer Krankheit, die durch GPR17 moduliert wird.

13. Pharmazeutische Zusammensetzung, umfassend eine Verbindung nach einem der Ansprüche 1 bis 9 und einen therapeutisch inerten Träger.

14. Verbindung nach einem der Ansprüche 1 bis 9 zur Verwendung bei der Behandlung oder Prophylaxe von Erkrankungen, die aus direkter Schädigung von Myelinscheiden resultieren (einschließlich, aber nicht beschränkt auf zentrale pontine und extrapontine Myelinolyse, Kohlenmonoxidvergiftung, Mangelernährung und virusinduzierte Demyelinisierung), demyelinisierenden Störungen (einschließlich, aber nicht beschränkt auf Multiple Sklerose, akute und mehrphasige disseminierte Enzephalomyelitis, Neuromyelitis-optica-Spektrum-Störungen und Leukodystrophien), mit Myelinverlust assoziierte Störungen des ZNS (einschließlich, aber nicht beschränkt auf Alzheimer-Krankheit, Schizophrenie, Parkinson-Krankheit, Huntington-Krankheit, amyotrophe Lateralsklerose und Ischämie aufgrund von Schlaganfall) und Entzündungen im ZNS, zum Beispiel nach Enzephalitis, primärer Angiitis, Meningitis und Adipositas.

15. Verbindung nach einem der Ansprüche 1 bis 9 zur Verwendung bei der Behandlung oder Prophylaxe von Multipler Sklerose.

## Revendications

1. Composés de formule I 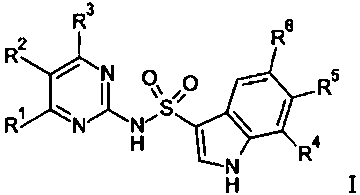 dans lesquels
R¹ représente un alcoxy ou un halogénoalcoxy ;
R² représente un halogène, un alkyle, un alcoxy, un halogénoalkyle, un halogénoalcoxy, un cyanoalkyle, un cyanoalcoxy ou un cyclopropyle éventuellement substitué par jusqu'à deux substituants indépendamment choisis parmi un cyano et un halogène ;
R³ représente H, un alcoxy ou un halogénoalcoxy ;
R⁵ représente H, un halogène, un alkyle ou un halogénoalkyle ;
R⁶ représente H ou un halogène ;
R⁴ représente un hétéroaryle lié par N ou un hétéroaryle lié par N substitué, représenté par 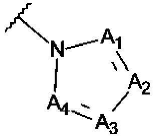
A₁ représente CR^{y1} ou N ;
A₂ représente CR^{y2} ou N ;
A₃ représente CR^{y3} ou N ;
A₄ représente CR^{y4} ou N ;
dans lesquels R^{y1}, R^{y2}, R^{y3} et R^{y4} sont indépendamment choisis parmi H, un alkyle, un halogène et un halogénoalkyle ;
et des sels pharmaceutiquement acceptables.

2. Composé selon la revendication 1, dans lequel R² représente un halogène, un alkyle, un halogénoalkyle, un halogénoalcoxy, un cyanoalkyle, un cyanoalcoxy ou un cyclopropyle substitué par un cyano.

3. Composé selon l'une quelconque des revendications 1 à 2, dans lequel R³ représente H ou un alcoxy.

4. Composé selon l'une quelconque des revendications 1 à 3, dans lequel au moins un, mais pas plus de deux, de A₁, A₂, A₃ et A₄ représentent N, et R^{y1}, R^{y2}, R^{y3} et R^{y4} sont indépendamment choisis parmi H ou un alkyle.

5. Composé selon la revendication 1, dans lequel
R¹ représente un alcoxy ;
R² représente un halogène, un alkyle, un halogénoalkyle, un halogénoalcoxy, un cyanoalkyle, un cyanoalcoxy, ou un cyclopropyle substitué par un cyano ;
R³ représente H ou un alcoxy ;
R⁵ représente H, un halogène, un alkyle ou un halogénoalkyle ;
R⁶ représente H ou un halogène ;
R⁴ représente un hétéroaryle lié par N ou un hétéroaryle lié par N substitué, représenté par 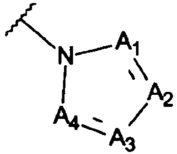
A₁ représente CR^{y1} ou N ;
A₂ représente CR^{y2} ou N ;
A₃ représente CR^{y3} ou N ;
A₄ représente CR^{y4} ou N ;
dans lequel R^{y1}, R^{y2}, R^{y3} et R^{y4} sont indépendamment choisis parmi H, un alkyle et un halogène ;
et des sels pharmaceutiquement acceptables.

6. Composé selon la revendication 1, dans lequel
R¹ représente un alcoxy ;
R² représente un halogénoalkyle, un halogénoalcoxy ou un cyclopropyle éventuellement substitué par un cyano ;
R³ représente H ou un alcoxy ;
R⁵ représente H, un halogène, un alkyle ou un halogénoalkyle ;
R⁶ représente H ;
R⁴ est choisi parmi 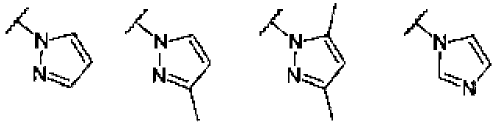 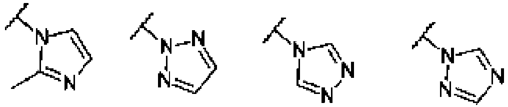 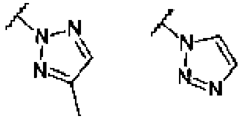
et des sels pharmaceutiquement acceptables.

7. Composé selon l'une quelconque des revendications 1 à 6, choisi parmi
le N-[5-(2,2-difluoroéthyl)-4,6-diméthoxy-pyrimidin-2-yl]-7-(3-méthylpyrazol-1-yl)-1H-indole-3-sulfonamide ;
le N-[5-(2,2-difluoroéthyl)-4,6-diméthoxy-pyrimidin-2-yl]-7-(3,5-diméthylpyrazol-1-yl)-1H-indole-3-sulfonamide ;
le N-[5-(2,2-difluoroéthyl)-4,6-diméthoxy-pyrimidin-2-yl]-7-imidazol-1-yl-1H-indole-3-sulfonamide ;
le 6-chloro-N-[5-(2,2-difluoroéthyl)-4,6-diméthoxy-pyrimidin-2-yl]-7-pyrazol-1-yl-1H-indole-3-sulfonamide ;
le N-[5-(2,2-difluoroéthyl)-4,6-diméthoxy-pyrimidin-2-yl]-7-(2-méthylimidazol-1-yl)-1H-indole-3-sulfonamide ;
le 6-chloro-N-[5-(2,2-difluoroéthoxy)-4,6-diméthoxy-pyrimidin-2-yl]-7-imidazol-1-yl-1H-indole-3-sulfonamide ;
le 6-chloro-N-[5-(2,2-difluoroéthyl)-4,6-diméthoxy-pyrimidin-2-yl]-7-(triazol-2-yl)-1H-indole-3-sulfonamide ;
le 6-chloro-N-[5-(2,2-difluoroéthyl)-4-méthoxy-pyrimidin-2-yl]-7-imidazol-1-yl-1H-indole-3-sulfonamide ;
le 6-chloro-N-[5-(2,2-difluoroéthoxy)-4,6-diméthoxy-pyrimidin-2-yl]-7-(triazol-2-yl)-1H-indole-3-sulfonamide ;
le 6-chloro-N-[5-(2,2-difluoroéthyl)-4,6-diméthoxy-pyrimidin-2-yl]-7-(1,2,4-triazol-4-yl)-1H-indole-3-sulfonamide ;
le N-[5-(2,2-difluoroéthoxy)-4,6-diméthoxy-pyrimidin-2-yl]-6-(difluorométhyl)-7-(triazol-2-yl)-1H-indole-3-sulfonamide ;
le 6-chloro-N-[5-(2-fluoroéthoxy)-4-méthoxy-pyrimidin-2-yl]-7-(triazol-2-yl)-1H-indole-3-sulfonamide ;
le N-[5-(2,2-difluoroéthyl)-4,6-diméthoxy-pyrimidin-2-yl]-7-(triazol-2-yl)-1H-indole-3-sulfonamide ;
le 6-chloro-N-[5-(2-fluoroéthoxy)-4,6-diméthoxy-pyrimidin-2-yl]-7-(triazol-2-yl)-1H-indole-3-sulfonamide ;
le 6-chloro-N-[5-(difluorométhoxy)-4,6-diméthoxy-pyrimidin-2-yl]-7-(triazol-2-yl)-1H-indole-3-sulfonamide ;
le 6-chloro-N-[5-(2,3-difluoropropyl)-4,6-diméthoxy-pyrimidin-2-yl]-7-(triazol-2-yl)-1H-indole-3-sulfonamide ;
le 6-chloro-N-[5-(3,3-difluoropropyl)-4,6-diméthoxy-pyrimidin-2-yl]-7-(triazol-2-yl)-1H-indole-3-sulfonamide ;
le 6-chloro-N-[5-(2-cyanocyclopropyl)-4,6-diméthoxy-pyrimidin-2-yl]-7-(triazol-2-yl)-1H-indole-3-sulfonamide ;
le N-[5-(2-fluoroéthoxy)-4,6-diméthoxy-pyrimidin-2-yl]-7-(triazol-2-yl)-1H-indole-3-sulfonamide ;
le N-[5-(2,2-difluoroéthoxy)-4,6-diméthoxy-pyrimidin-2-yl]-7-(triazol-2-yl)-1H-indole-3-sulfonamide ;
le N-[5-(2-cyanocyclopropyl)-4,6-diméthoxy-pyrimidin-2-yl]-7-(triazol-2-yl)-1H-indole-3-sulfonamide ;
le N-[5-(2,2-difluoroéthyl)-4,6-diméthoxy-pyrimidin-2-yl]-6-méthyl-7-(triazol-2-yl)-1H-indole-3-sulfonamide ;
le N-[5-(2-fluoroéthoxy)-4,6-diméthoxy-pyrimidin-2-yl]-6-méthyl-7-(triazol-2-yl)-1H-indole-3-sulfonamide ;
le N-[5-(difluorométhoxy)-4,6-diméthoxy-pyrimidin-2-yl]-6-méthyl-7-(triazol-2-yl)-1H-indole-3-sulfonamide ;
le N-[5-(2,2-difluoroéthoxy)-4,6-diméthoxy-pyrimidin-2-yl]-6-méthyl-7-(triazol-2-yl)-1H-indole-3-sulfonamide ;
le N-[5-(2-cyanocyclopropyl)-4,6-diméthoxy-pyrimidin-2-yl]-6-méthyl-7-(triazol-2-yl)-1H-indole-3-sulfonamide ;
le 6-bromo-N-[5-(2,2-difluoroéthyl)-4,6-diméthoxy-pyrimidin-2-yl]-7-(triazol-2-yl)-1H-indole-3-sulfonamide ;
le 6-bromo-N-[5-(2-fluoroéthoxy)-4,6-diméthoxy-pyrimidin-2-yl]-7-(triazol-2-yl)-1H-indole-3-sulfonamide ;
le 6-bromo-N-[5-(difluorométhoxy)-4,6-diméthoxy-pyrimidin-2-yl]-7-(triazol-2-yl)-1H-indole-3-sulfonamide ;
le 6-bromo-N-[5-(2,2-difluoroéthoxy)-4,6-diméthoxy-pyrimidin-2-yl]-7-(triazol-2-yl)-1H-indole-3-sulfonamide ;
le 6-bromo-N-[5-(2-cyanocyclopropyl)-4,6-diméthoxy-pyrimidin-2-yl]-7-(triazol-2-yl)-1H-indole-3-sulfonamide ;
le N-[5-(2,2-difluoroéthyl)-4,6-diméthoxy-pyrimidin-2-yl]-6-fluoro-7-(triazol-2-yl)-1H-indole-3-sulfonamide ;
le 6-fluoro-N-[5-(2-fluoroéthoxy)-4,6-diméthoxy-pyrimidin-2-yl]-7-(triazol-2-yl)-1H-indole-3-sulfonamide ;
le N-[5-(difluorométhoxy)-4,6-diméthoxy-pyrimidin-2-yl]-6-fluoro-7-(triazol-2-yl)-1H-indole-3-sulfonamide ;
le N-[5-(2,2-difluoroéthoxy)-4,6-diméthoxy-pyrimidin-2-yl]-6-fluoro-7-(triazol-2-yl)-1H-indole-3-sulfonamide ;
le N-[5-(2-cyanocyclopropyl)-4,6-diméthoxy-pyrimidin-2-yl]-6-fluoro-7-(triazol-2-yl)-1H-indole-3-sulfonamide ;
le 6-chloro-N-[5-(2,2-difluoroéthyl)-4,6-diméthoxy-pyrimidin-2-yl]-7-(1,2,4-triazol-1-yl)-1H-indole-3-sulfonamide ;
le 6-chloro-N-[5-(2,2-difluoroéthoxy)-4,6-diméthoxy-pyrimidin-2-yl]-7-(4-méthyltriazol-2-yl)-1H-indole-3-sulfonamide ;
le 6-chloro-N-[5-(2-fluoroéthoxy)-4,6-diméthoxy-pyrimidin-2-yl]-7-(4-méthyltriazol-2-yl)-1H-indole-3-sulfonamide ;
le 6-chloro-N-[5-(2,2-difluoroéthyl)-4,6-diméthoxy-pyrimidin-2-yl]-7-(triazol-1-yl)-1H-indole-3-sulfonamide ;
le 6-(difluorométhyl)-N-[5-(2-fluoroéthoxy)-4,6-diméthoxy-pyrimidin-2-yl]-7-(triazol-2-yl)-1H-indole-3-sulfonamide ;
le N-[5-(2,2-difluoroéthyl)-4,6-diméthoxy-pyrimidin-2-yl]-6-(difluorométhyl)-7-(triazol-2-yl)-1H-indole-3-sulfonamide ; et
des sels pharmaceutiquement acceptables de ceux-ci.

8. Composé selon l'une quelconque des revendications 1 à 6, choisi parmi
le 6-chloro-N-[5-(2,2-difluoroéthoxy)-4,6-diméthoxy-pyrimidin-2-yl]-7-(4-méthylpyrazol-1-yl)-1H-indole-3-sulfonamide ;
le 6-chloro-N-[5-(2,2-difluoroéthoxy)-4,6-diméthoxy-pyrimidin-2-yl]-7-(3-méthylpyrazol-1-yl)-1H-indole-3-sulfonamide ;
le 6-chloro-N-[5-(2,2-difluoroéthoxy)-4,6-diméthoxy-pyrimidin-2-yl]-7-(4-fluoropyrazol-1-yl)-1H-indole-3-sulfonamide ;
le 6-chloro-N-[5-(2,2-difluoroéthoxy)-4,6-diméthoxy-pyrimidin-2-yl]-7-(5-méthylpyrazol-1-yl)-1H-indole-3-sulfonamide ;
le 6-chloro-N-[5-(2-fluoroéthoxy)-4,6-diméthoxy-pyrimidin-2-yl]-7-(4-fluoropyrazol-1-yl)-1H-indole-3-sulfonamide ;
le 6-chloro-N-[5-(difluorométhoxy)-4,6-diméthoxy-pyrimidin-2-yl]-7-(4-fluoropyrazol-1-yl)-1H-indole-3-sulfonamide ;
le N-[5-(difluorométhoxy)-4,6-diméthoxy-pyrimidin-2-yl]-6-(difluorométhyl)-7-pyrazol-1-yl-1H-indole-3-sulfonamide ;
le N-[5-(2,2-difluoroéthoxy)-4,6-diméthoxy-pyrimidin-2-yl]-6-(difluorométhyl)-7-pyrazol-1-yl-1H-indole-3-sulfonamide ;
le 6-(difluorométhyl)-N-[5-(2-fluoroéthoxy)-4,6-diméthoxy-pyrimidin-2-yl]-7-pyrazol-1-yl-1H-indole-3-sulfonamide ;
le N-[5-(2,2-difluoroéthyl)-4,6-diméthoxy-pyrimidin-2-yl]-6-(difluorométhyl)-7-pyrazol-1-yl-1H-indole-3-sulfonamide ;
le 6-chloro-N-[5-(2,2-difluoroéthoxy)-4,6-diméthoxy-pyrimidin-2-yl]-7-(3-fluoropyrazol-1-yl)-1H-indole-3-sulfonamide ;
le 6-chloro-N-[5-(difluorométhoxy)-4,6-diméthoxy-pyrimidin-2-yl]-7-(3-fluoropyrazol-1-yl)-1H-indole-3-sulfonamide ;
le 6-chloro-N-[5-(2,2-difluoroéthyl)-4,6-diméthoxy-pyrimidin-2-yl]-7-(3-fluoropyrazol-1-yl)-1H-indole-3-sulfonamide ;
le 6-chloro-N-[5-(2-fluoroéthoxy)-4,6-diméthoxy-pyrimidin-2-yl]-7-(3-fluoropyrazol-1-yl)-1H-indole-3-sulfonamide ;
le 6-chloro-N-[4,6-diméthoxy-5-(1,1,2-trifluoroéthoxy)pyrimidin-2-yl]-7-(triazol-2-yl)-1H-indole-3-sulfonamide ;
le 6-chloro-N-[5-(2-fluoro-1,1-diméthyl-éthoxy)-4,6-diméthoxy-pyrimidin-2-yl]-7-(triazol-2-yl)-1H-indole-3-sulfonamide ;
le 6-chloro-N-[5-(1,1-dideutério-2,2-difluoro-éthoxy)-4,6-diméthoxy-pyrimidin-2-yl]-7-(triazol-2-yl)-1H-indole-3-sulfonamide ;
le 6-chloro-N-[5-(cyanométhoxy)-4,6-diméthoxy-pyrimidin-2-yl]-7-(triazol-2-yl)-1H-indole-3-sulfonamide ;
le 6-chloro-N-[5-(2-cyanoéthyl)-4,6-diméthoxy-pyrimidin-2-yl]-7-(triazol-2-yl)-1H-indole-3-sulfonamide ;
le 6-chloro-N-(4,6-diméthoxy-5-méthyl-pyrimidin-2-yl)-7-(triazol-2-yl)-1H-indole-3-sulfonamide ;
le N-(5-bromo-4,6-diméthoxy-pyrimidin-2-yl)-6-chloro-7-(triazol-2-yl)-1H-indole-3-sulfonamide ;
le 6-chloro-N-[5-(2,2-difluoroéthoxy)-4,6-diméthoxy-pyrimidin-2-yl]-5-fluoro-7-(triazol-2-yl)-1H-indole-3-sulfonamide ;
le 6-chloro-N-[5-(2,2-difluoroéthoxy)-4,6-diméthoxy-pyrimidin-2-yl]-7-pyrazol-1-yl-1H-indole-3-sulfonamide ;
le 6-chloro-N-[5-(difluorométhoxy)-4,6-diméthoxy-pyrimidin-2-yl]-7-pyrazol-1-yl-1H-indole-3-sulfonamide ;
le 6-chloro-N-[5-(2-fluoroéthoxy)-4,6-diméthoxy-pyrimidin-2-yl]-7-pyrazol-1-yl-1H-indole-3-sulfonamide ;
le 6-chloro-N-[4,6-diméthoxy-5-(1,1,2,2-tétradeutério-2-fluoro-éthoxy)pyrimidin-2-yl]-7-pyrazol-1-yl-1H-indole-3-sulfonamide ;
le 6-chloro-N-[5-(cyanométhoxy)-4,6-diméthoxy-pyrimidin-2-yl]-7-pyrazol-1-yl-1H-indole-3-sulfonamide ;
le 6-chloro-N-[5-(2-cyanoéthyl)-4,6-diméthoxy-pyrimidin-2-yl]-7-pyrazol-1-yl-1H-indole-3-sulfonamide ;
le 6-chloro-N-[5-(2,2-difluoroéthyl)-4-méthoxy-pyrimidin-2-yl]-7-pyrazol-1-yl-1H-indole-3-sulfonamide ;
le 6-chloro-N-[5-(1,1-dideutério-2,2-difluoro-éthyl)-4-méthoxy-pyrimidin-2-yl]-7-pyrazol-1-yl-1H-indole-3-sulfonamide ;
le 6-chloro-N-[5-(2-cyanocyclopropyl)-4,6-diméthoxy-pyrimidin-2-yl]-7-pyrazol-1-yl-1H-indole-3-sulfonamide ;
le N-[5-(2,2-difluoroéthoxy)-4,6-diméthoxy-pyrimidin-2-yl]-6-méthyl-7-pyrazol-1-yl-1H-indole-3-sulfonamide ;
le 6-bromo-N-[5-(2,2-difluoroéthoxy)-4,6-diméthoxy-pyrimidin-2-yl]-7-pyrazol-1-yl-1H-indole-3-sulfonamide ;
le N-[5-(difluorométhoxy)-4,6-diméthoxy-pyrimidin-2-yl]-6-méthyl-7-pyrazol-1-yl-1H-indole-3-sulfonamide ;
le 6-bromo-N-[5-(difluorométhoxy)-4,6-diméthoxy-pyrimidin-2-yl]-7-pyrazol-1-yl-1H-indole-3-sulfonamide ;
le 6-chloro-N-[5-(2,2-difluoroéthyl)-4-méthoxy-pyrimidin-2-yl]-7-(triazol-2-yl)-1H-indole-3-sulfonamide ;
le 6-chloro-N-[5-(1,1-dideutério-2,2-difluoro-éthyl)-4-méthoxy-pyrimidin-2-yl]-7-(triazol-2-yl)-1H-indole-3-sulfonamide ;
le N-[5-(2,2-difluoroéthyl)-4,6-diméthoxy-pyrimidin-2-yl]-6-méthyl-7-pyrazol-1-yl-1H-indole-3-sulfonamide ;
le 6-bromo-N-[5-(2,2-difluoroéthyl)-4,6-diméthoxy-pyrimidin-2-yl]-7-pyrazol-1-yl-1H-indole-3-sulfonamide ;
le N-[5-(2-fluoroéthoxy)-4,6-diméthoxy-pyrimidin-2-yl]-6-méthyl-7-pyrazol-1-yl-1H-indole-3-sulfonamide ;
le 6-bromo-N-[5-(2-fluoroéthoxy)-4,6-diméthoxy-pyrimidin-2-yl]-7-pyrazol-1-yl-1H-indole-3-sulfonamide ;
le 6-bromo-N-[4,6-diméthoxy-5-(1,1,2,2-tétradeutério-2-fluoro-éthoxy)pyrimidin-2-yl]-7-pyrazol-1-yl-1H-indole-3-sulfonamide ;
le 6-chloro-N-[4,6-diméthoxy-5-(1,1,2,2-tétradeutério-2-fluoro-éthoxy)pyrimidin-2-yl]-7-(triazol-2-yl)-1H-indole-3-sulfonamide ;
le 6-chloro-N-[5-(1,1-dideutério-2,2-difluoro-éthyl)-4,6-diméthoxy-pyrimidin-2-yl]-7-pyrazol-1-yl-1H-indole-3-sulfonamide ;
et des sels pharmaceutiquement acceptables de ceux-ci.

9. Composé selon l'une quelconque des revendications 1 à 6, choisi parmi
le 6-chloro-N-[5-(2,2-difluoroéthoxy)-4,6-diméthoxy-pyrimidin-2-yl]-7-(triazol-2-yl)-1H-indole-3-sulfonamide ;
le 6-chloro-N-[5-(difluorométhoxy)-4,6-diméthoxy-pyrimidin-2-yl]-7-(triazol-2-yl)-1H-indole-3-sulfonamide ;
et des sels pharmaceutiquement acceptables de ceux-ci.

10. Procédé de préparation d'un composé selon l'une quelconque des revendications 1 à 9 comprenant la réaction d'un composé de formule III avec un composé de formule II en présence d'une base choisie parmi la N-éthyldiisopropylamine, la pyridine, le phosphate de potassium ou l'hydrure de sodium, pour fournir un composé de formule I, 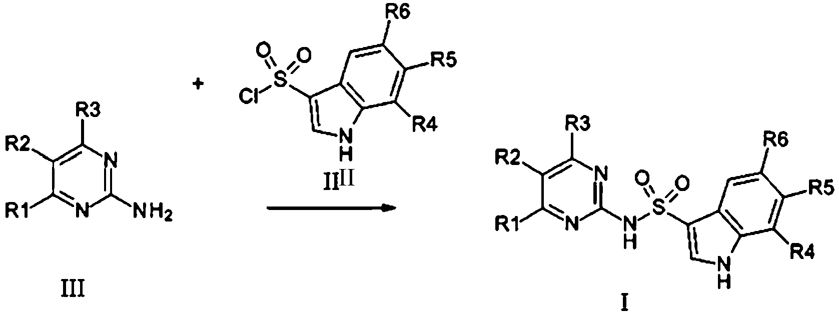 dans lequel R¹, R², R³, R³, R⁴, R⁵ et R⁶ sont tels que décrits ci-dessus.

11. Composé selon l'une quelconque des revendications 1 à 9 pour une utilisation comme substance thérapeutiquement active.

12. Composé selon l'une quelconque des revendications 1 à 9 pour une utilisation dans le traitement d'une maladie modulée par GPR17.

13. Composition pharmaceutique comprenant un composé selon l'une quelconque des revendications 1 à 9 et un véhicule thérapeutiquement inerte.

14. Composé selon l'une quelconque des revendications 1 à 9 pour une utilisation dans le traitement ou la prophylaxie d'affections résultant d'une lésion directe de gaines de myéline (notamment mais sans s'y limiter une myélinolyse centropontine et extrapontine, un empoisonnement au monoxyde de carbone, une carence nutritionnelle et une démyélinisation induite par un virus), de troubles de démyélinisation (notamment mais sans s'y limiter une sclérose en plaques, une encéphalomyélite disséminée aigüe et multiphasique, des troubles du spectre de la neuromyélite optique et des leucodystrophies), de troubles du SNC associés à une perte de myéline (notamment mais sans s'y limiter la maladie d'Alzheimer, une schizophrénie, la maladie de Parkinson, la maladie d'Huntington, une sclérose latérale amyotrophique et une ischémie due à un accident vasculaire cérébral) et d'une inflammation dans le SNC, par exemple suite à une encéphalite, une angéite primaire, une méningite et une obésité.

15. Composé selon l'une quelconque des revendications 1 à 9 pour une utilisation dans le traitement ou la prophylaxie d'une sclérose en plaques.
